# EUROPEAN PATENT SPECIFICATION

(11) **EP 2 399 131 B1**
(45) Date of publication and mention of the grant of the patent: **27.08.2014**
(21) Application number: 10705514.7
(22) Date of filing: 19.02.2010
(51) Int. Cl.: G01N 33/68

(54) **METHOD FOR THE DIAGNOSIS OF AGE-ASSOCIATED VASCULAR DISORDERS**
VERFAHREN ZUR DIAGNOSE VON ALTERSBEDINGTEN GEFÄSSERKRANKUNG
METHODE DE DIAGNOSE DES TROUBLES VASCULAIRE LIES A L'AGE

(30) Priority: 20.02.2009 US 154329 P
(43) Date of publication of application: 28.12.2011
(73) Proprietor: The U.S.A. as represented by the Secretary, Department of Health and Human Services, Bethesda, MD 20892-7660 (US); The Johns Hopkins University, Baltimore, MD 21218 (US)
(72) Inventor: WANG, Mingyi, Baltimore, MD 21224 (US); FU, Zongming, Laurel, MD 20723 (US); LAKATTA, Edward, G., Bel Air, MD 21014 (US); VAN EYK, Jennifer, Baltimore, MD 21239 (US)
(74) Representative: Bentham, Andrew
(86) International application number: PCT/US2010/024816
(87) International publication number: WO 2010/096713

(56) References cited:
- EP-A1- 1 978 034
- EP-A1- 2 019 318
- WO-A1-96/41194
- WO-A1-2008/089994
- US-A1- 2003 148 334
- US-A1- 2008 166 734
- US-B1- 7 108 982
- PENG SIWEI ET AL: "Medin-amyloid: a recently characterized age-associated arterial amyloid form affects mainly arteries in the upper part of the body." AMYLOID : THE INTERNATIONAL JOURNAL OF EXPERIMENTAL AND CLINICAL INVESTIGATION : THE OFFICIAL JOURNAL OF THE INTERNATIONAL SOCIETY OF AMYLOIDOSIS JUN 2005 LNKD- PUBMED:16011985, vol. 12, no. 2, June 2005 (2005-06), pages 96-102, XP002578164 ISSN: 1350-6129
- AIT-OUFELLA H ET AL: "MFGE8-dependent phagocytosis of apoptotic cells protects against atherosclerosis development and prevents necrotic core formation" VASCULAR PHARMACOLOGY, ELSEVIER, AMSTERDAM, NL LNKD- DOI:10.1016/J.VPH.2006.08.031, vol. 45, no. 3, 1 September 2006 (2006-09-01), pages 184-185, XP024970068 ISSN: 1537-1891 [retrieved on 2006-09-01]
- BEAUCHAMP N J ET AL: "Gene expression profiling of resting and activated vascular smooth muscle cells by serial analysis of gene expression and clustering analysis" GENOMICS, ACADEMIC PRESS, SAN DIEGO, US LNKD- DOI:10.1016/S0888-7543(03)00127-7, vol. 82, no. 3, 1 September 2003 (2003-09-01), pages 288-299, XP004442997 ISSN: 0888-7543
- WANG MINGYI ET AL: "Proinflammatory profile within the grossly normal aged human aortic wall." HYPERTENSION JUL 2007 LNKD- PUBMED:17452499, vol. 50, no. 1, July 2007 (2007-07), pages 219-227, XP002578324 ISSN: 1524-4563
- FU Z ET AL: "Milk fat globule protein epidermal growth factor-8: A pivotal relay element within the angiotensin II and monocyte chemoattractant protein-1 signaling cascade mediating vascular smooth muscle cells invasion" CIRCULATION RESEARCH 20090619 LIPPINCOTT WILLIAMS AND WILKINS USA LNKD- DOI:10.1161/CIRCRESAHA.108.187088, vol. 104, no. 12, 19 June 2009 (2009-06-19), pages 1337-1346, XP002578325
- CHUNG A W ET AL: "Increased matrix metalloproteinase 2 activity in the human internal mammary artery is associated with ageing, hypertension, diabetes and kidney dysfunction" JOURNAL OF VASCULAR RESEARCH, vol. 45, no. 4, 2008, pages 357-362, XP002595506 ISSN: 1018-1172
- DATABASE BIOSIS [Online] BIOSCIENCES INFORMATION SERVICE, PHILADELPHIA, PA, US; 2007, KUNZ JOCHEN: "Matrix metalloproteinases and atherogenesis in dependence of age" XP002595507 Database accession no. PREV200700127169 & GERONTOLOGY, vol. 53, no. 2, 2007, pages 63-73, ISSN: 0304-324X
- KAI HISASHI ET AL: "Peripheral blood levels of matrix metalloproteases-2 and -9 are elevated in patients with acute coronary syndromes" JOURNAL OF THE AMERICAN COLLEGE OF CARDIOLOGY, vol. 32, no. 2, August 1998 (1998-08), pages 368-372, XP002595508 ISSN: 0735-1097
- KITTLESON M M ET AL: "Molecular Signature Analysis: Using the Myocardial Transcriptome as a Biomarker in Cardiovascular Disease" TRENDS IN CARDIOVASCULAR MEDICINE, ELSEVIER SCIENCE, NEW YORK, NY, US, vol. 15, no. 4, 1 May 2005 (2005-05-01), pages 130-138, XP025394957 ISSN: 1050-1738 [retrieved on 2005-05-01]

### SEQUENCE LISTING

<110> THE UNITED STATES OF AMERICA, as represented by the
   Secretary, Department of Health and Human Services
   Johns Hopkins University
   Wang, Mingyi
   Fu, Zongming
   Lakatta, Edward G.
   Van Eyk, Jennifer G.
<120> METHOD FOR THE DIAGNOSIS OF AGE-ASSOCIATED VASCULAR DISORDERS
<130> 4239-81526-02
<140> PCTUS1024816
   <141> 2010-02-19
<150> US 61/154,329
   <151> 2009-02-20
<160> 14
<170> PatentIn version 3.5
<210> 1
   <211> 9
   <212> PRT
   <213> Rattus norvegicus
<400> 1
   Val Leu Pro Leu Ser Trp His Asn Arg
   1               5
<210> 2
   <211> 9
   <212> PRT
   <213> Rattus norvegicus
<400> 2
   Cys Leu Val Thr Glu Asp Thr Gln Arg
   1               5
<210> 3
   <211> 20
   <212> DNA
   <213> Artificial Sequence
<220>
   <223> Synthetic oligonuceotide primer.
<400> 3
   ctggacaatc agggcaagat 20
<210> 4
   <211> 21
   <212> DNA
   <213> Artificial Sequence
<220>
   <223> Synthetic oligonucleotide primer.
<400> 4
   gtccactgca caaccatcat c 21
<210> 5
   <211> 387
   <212> PRT
   <213> Homo sapiens
<220>
   <221> CARBOHYD
   <222> (238)..(238)
   <223> potential N-linked glycosylation site
<220>
   <221> CARBOHYD
   <222> (325)..(325)
   <223> potential N-linked glycosylation site
<220>
   <221> CARBOHYD
   <222> (329)..(329)
   <223> potential N-linked glycosylation site
<220>
   <221> CARBOHYD
   <222> (350)..(350)
   <223> potential N-linked glycosylation site
<400> 5
   Met Pro Arg Pro Arg Leu Leu Ala Ala Leu Cys Gly Ala Leu Leu Cys
   1               5                   10                  15
   Ala Pro Ser Leu Leu Val Ala Leu Asp Ile Cys Ser Lys Asn Pro Cys
               20                  25                  30
   His Asn Gly Gly Leu Cys Glu Glu Ile Ser Gln Glu Val Arg Gly Asp
           35                  40                  45
   Val Phe Pro Ser Tyr Thr Cys Thr Cys Leu Lys Gly Tyr Ala Gly Asn
       50                  55                  60
   His Cys Glu Thr Lys Cys Val Glu Pro Leu Gly Met Glu Asn Gly Asn
   65 70 75 80
   Ile Ala Asn Ser Gln Ile Ala Ala Ser Ser Val Arg Val Thr Phe Leu
   85 90 95
   Gly Leu Gln His Trp Val Pro Glu Leu Ala Arg Leu Asn Arg Ala Gly
   100 105 110
   Met Val Asn Ala Trp Thr Pro Ser Ser Asn Asp Asp Asn Pro Trp Ile
   115 120 125
   Gln Val Asn Leu Leu Arg Arg Met Trp Val Thr Gly Val Val Thr Gln
   130 135 140
   Gly Ala Ser Arg Leu Ala Ser His Glu Tyr Leu Lys Ala Phe Lys Val
   145 150 155 160
   Ala Tyr Ser Leu Asn Gly His Glu Phe Asp Phe Ile His Asp Val Asn
   165 170 175
   Lys Lys His Lys Glu Phe Val Gly Asn Trp Asn Lys Asn Ala Val His
   180 185 190
   Val Asn Leu Phe Glu Thr Pro Val Glu Ala Gln Tyr Val Arg Leu Tyr
   195 200 205
   Pro Thr Ser Cys His Thr Ala Cys Thr Leu Arg Phe Glu Leu Leu Gly
   210 215 220
   Cys Glu Leu Asn Gly Cys Ala Asn Pro Leu Gly Leu Lys Asn Asn Ser
   225 230 235 240
   Ile Pro Asp Lys Gln Ile Thr Ala Ser Ser Ser Tyr Lys Thr Trp Gly
   245 250 255
   Leu His Leu Phe Ser Trp Asn Pro Ser Tyr Ala Arg Leu Asp Lys Gln
   260 265 270
   Gly Asn Phe Asn Ala Trp Val Ala Gly Ser Tyr Gly Asn Asp Gln Trp
   275 280 285
   Leu Gln Val Asp Leu Gly Ser Ser Lys Glu Val Thr Gly Ile Ile Thr
   290 295 300
   Gln Gly Ala Arg Asn Phe Gly Ser Val Gln Phe Val Ala Ser Tyr Lys
   305 310 315 320
   Val Ala Tyr Ser Asn Asp Ser Ala Asn Trp Thr Glu Tyr Gln Asp Pro
   325 330 335
   Arg Thr Gly Ser Ser Lys Ile Phe Pro Gly Asn Trp Asp Asn His Ser
   340 345 350
   His Lys Lys Asn Leu Phe Glu Thr Pro Ile Leu Ala Arg Tyr Val Arg
   355 360 365
   Ile Leu Pro Val Ala Trp His Asn Arg Ile Ala Leu Arg Leu Glu Leu
   370 375 380
   Leu Gly Cys
   385
<210> 6
   <211> 1164
   <212> DNA
   <213> Homo sapiens
<400> 6
   atgccgcgcc cccgcctgct ggccgcgctg tgcggcgcgc tgctctgcgc ccccagcctc 60
   ctcgtcgccc tggatatctg ttccaaaaac cctgccacaa acggtggttt atgcgaggag 120
   atttcccaag aagtgcgagg agatgtcttc ccctcgtaca cctgcacgtg ccttaagggc 180
   tacgcgggca accactgtga gacgaaatgt gtcgagccac tgggcctgga gaatgggaac 240
   attgccaact cacagatcgc cgcctcgtct gtgcgtgtga ccttcttggg tttgcagcat 300
   tgggtcccgg agctggcccg cctgaaccgc gcaggcatgg tcaatgcctg gacacccagc 360
   agcaatgacg ataacccctg gatccaggtg aacctgctgc ggaggatgtg ggtaacaggt 420
   gtggtgacgc agggtgccag ccgcttggcc agtcatgagt acctgaaggc cttcaaggtg 480
   gcctacagcc ttaatggaca cgaattcgat ttcatccatg atgttaataa aaaacacaag 540
   gagtttgtgg gtaactggaa caaaaacgcg gtgcatgtca acctgtttga gacccctgtg 600
   gaggctcagt acgtgagatt gtaccccacg agctgccaca cggcctgcac tctgcgcttt 660
   gagctactgg gctgtgagct gaacggatgc gccaatcccc tgggcctgaa gaataacagc 720
   atccctgaca agcagatcac ggcctccagc agctacaaga cctggggctt gcatctcttc      780
   agctggaacc cctcctatgc acggctggac aagcagggca acttcaacgc ctgggttgcg      840
   gggagctacg gtaacgatca gtggctgcag gtggacctgg gctcctcgaa ggaggtgaca      900
   ggcatcatca cccagggggc ccgtaacttt ggctctgtcc agtttgtggc atcctacaag      960
   gttgcctaca gtaatgacag tgcgaactgg actgagtacc aggaccccag gactggcagc     1020
   agtaagatct tccctggcaa ctgggacaac cactcccaca agaagaactt gtttgagacg     1080
   cccatcctgg ctcgctatgt gcgcatcctg cctgtagcct ggcacaaccg catcgccctg     1140
   cgcctggagc tgctgggctg ttag                                             1164
<210> 7
   <211> 480
   <212> PRT
   <213> Rattus norvegicus
<400> 7
   Met Gln Phe Ser Arg Val Leu Ala Ala Leu Cys Gly Val Leu Leu Cys
   1               5                   10                  15
   Ala Ser Gly Leu Phe Ala Ala Ser Gly Asp Phe Cys Asp Ser Ser Leu
               20                  25                  30
   Cys Leu Asn Gly Gly Thr Cys Leu Met Gly Gln Asp Asn Asp Ile Tyr
           35                  40                  45
   Cys Leu Cys Pro Glu Gly Phe Thr Gly Leu Val Cys Asn Glu Thr Glu
       50                  55                  60
   Lys Gly Pro Cys Ser Pro Asn Pro Cys Phe His Asp Ala Lys Cys Leu
   65                  70                  75                  80
   Val Thr Glu Asp Thr Gln Arg Gly Asp Ile Phe Thr Glu Tyr Ile Cys
                   85                  90                  95
   Gln Cys Pro Val Gly Tyr Ser Gly Ile His Cys Glu Leu Glu Thr Thr
               100                 105                 110
   Ser Tyr Leu Asp Gly Glu Tyr Leu Ser Ser Pro Ala Val Pro Thr Thr
           115                 120                 125
   Ala Val Pro Thr Thr Ala Ile Pro Thr Thr Ala Val Pro Thr Thr Ala
   130 135 140
   Val Pro Thr Thr Ala Val Pro Thr Pro Ala Pro Asn Pro Asp Leu Ser
   145 150 155 160
   Asn His Leu Ala Ser Arg Cys Ser Thr Lys Leu Gly Leu Glu Gly Gly
   165 170 175
   Ala Ile Ala Asp Ser Gln Ile Ser Ala Ser Ser Val Tyr Met Gly Phe
   180 185 190
   Met Gly Leu Gln Arg Trp Gly Pro Glu Leu Ala Arg Leu Tyr Arg Thr
   195 200 205
   Gly Ile Val Asn Ala Trp Thr Ala Ser Ser Tyr Asp Ser Lys Pro Trp
   210 215 220
   Ile Gln Val Asp Phe Leu Arg Lys Met Arg Val Ser Gly Val Met Thr
   225 230 235 240
   Gln Gly Ala Ser Arg Ala Gly Arg Ala Glu Tyr Leu Lys Thr Phe Lys
   245 250 255
   Val Ala Tyr Ser Leu Asp Gly Arg Arg Phe Glu Phe Ile Gln Asp Glu
   260 265 270
   Ser Gly Thr Gly Asp Lys Glu Phe Met Gly Asn Gln Asp Asn Asn Ser
   275 280 285
   Leu Lys Ile Asn Met Phe Asn Pro Thr Leu Glu Ala Gln Tyr Ile Arg
   290 295 300
   Leu Tyr Pro Val Ser Cys His Arg Gly Cys Thr Leu Arg Phe Glu Leu
   305 310 315 320
   Leu Gly Cys Glu Leu His Gly Cys Ser Glu Pro Leu Gly Leu Lys Asn
   325 330 335
   Asn Thr Ile Pro Asp Ser Gln Ile Thr Ala Ser Ser Ser Tyr Lys Thr
   340 345 350
   Trp Asn Leu Arg Ala Phe Gly Trp Tyr Pro His Leu Gly Arg Leu Asp
   355 360 365
   Asn Gln Gly Lys Ile Asn Ala Trp Thr Ala Gln Ser Asn Ser Ala Lys
       370                 375                 380
   Glu Trp Leu Gln Val Asp Leu Gly Thr Gln Lys Lys Val Thr Gly Ile
   385                 390                 395                 400
   Ile Thr Gln Gly Ala Arg Asp Phe Gly His Ile Gln Tyr Val Ala Ser
                   405                 410                 415
   Tyr Lys Val Ala His Ser Asp Asp Gly Val Gln Trp Thr Val Tyr Glu
               420                 425                 430
   Glu Gln Gly Thr Ser Lys Val Phe Gln Gly Asn Leu Asp Asn Asn Ser
           435                 440                 445
   His Lys Lys Asn Ile Phe Glu Lys Pro Phe Met Ala Arg Tyr Val Arg
       450                 455                 460
   Val Leu Pro Leu Ser Trp His Asn Arg Ile Thr Leu Arg Leu Glu Leu
   465                 470                 475                 480
<210> 8
   <211> 1452
   <212> DNA
   <213> Rattus norvegicus
<400> 8
   atgcagttct cccgtgtgct ggccgcgctg tgcggtgtgc tgctctgcgc ctccggcctc 60
   ttcgctgcgt ccggtgactt ctgtgactcc agcctgtgcc tgaatggtgg gacctgcttg 120
   atgggccaag acaatgacat ctactgcctc tgccctgaag gcttcacagg ccttgtgtgc 180
   aacgagactg agaaaggacc gtgttcccca aacccttgct tccacgatgc caaatgcctg 240
   gtgactgagg acacacagcg aggggacatc ttcactgagt acatctgcca gtgccctgtg 300
   ggctactcgg gcatccactg tgaactcgag accacctcct acctggatgg agagtacctg 360
   tccagcccag ccgtccctac cacagccgtc cccaccacag ccatccccac cacagccgtc 420
   cccaccacag ccgtccccac cacagccgtc cccaccccgg cccccaaccc cgatctttcc 480
   aaccacctag cctcccgctg ttccacaaag ctgggcttgg aagggggcgc cattgccgat 540
   tcacagattt ctgcctcgtc tgtgtatatg ggcttcatgg gcttgcagcg ctggggcccg 600
   gagctggctc gcctgtatcg cacagggatt gtcaatgcat ggacagccag cagctatgat 660
   agcaagccct ggatccaggt ggactttctg cggaagatgc gggtatcagg tgtgatgaca      720
   cagggtgcca gccgtgccgg gagggcggaa tacctgaaga ccttcaaggt ggcttacagc      780
   ctcgatggac gcaggttcga gttcatccag gatgaaagcg gaaccggaga caaggagttt      840
   atgggtaacc aggacaacaa cagcctgaag attaacatgt tcaaccccac tctggaggca      900
   cagtacataa ggctgtaccc tgtctcgtgc caccgcggct gcaccctccg cttcgagctc      960
   ctgggctgcg agttgcatgg atgctctgag cccctgggcc tgaagaataa cacgattcct     1020
   gacagccaaa taacagcctc cagcagctac aagacgtgga acctgcgtgc ctttggctgg     1080
   tacccccact tggggcggct ggacaatcag ggcaagatca atgcctggac agctcagagc     1140
   aacagtgcca aggaatggct gcaggttgac ctgggcactc agaaaaaagt gacaggaatt     1200
   atcacccagg gggcccgtga ctttggccac atccagtatg tggcatccta taaggtagcc     1260
   cacagtgatg atggtgtgca gtggaccgta tatgaggaac aaggaaccag caaggtcttc     1320
   cagggcaact tggacaacaa ctcccacaag aagaacatct ttgagaaacc tttcatggct     1380
   cgctatgtgc gtgtccttcc actgtcctgg cataaccgta tcaccctgcg cctggagctg     1440
   ctgggctgtt ag                                                          1452
<210> 9
   <211> 8
   <212> PRT
   <213> Artificial Sequence
<220>
   <223> peptide
<400> 9
   Leu Ala Ser His Glu Tyr Leu Lys
   1               5
<210> 10
   <211> 8
   <212> PRT
   <213> Artificial Sequence
<220>
   <223> peptide
<400> 10
   Glu Phe Val Gly Asn Trp Asn Lys
   1               5
<210> 11
   <211> 7
   <212> PRT
   <213> Artificial Sequence
<220>
   <223> peptide
<400> 11
   Asn Asn Ser Ile Pro Asp Lys
   1 5
<210> 12
   <211> 6
   <212> PRT
   <213> Artificial Sequence
<220>
   <223> peptide
<400> 12
   Leu Glu Leu Leu Gly Cys
   1 5
<210> 13
   <211> 9
   <212> PRT
   <213> Artificial Sequence
<220>
   <223> peptide
<400> 13
   Asn Phe Gly Ser Val Gln Phe Val Ala
   1 5
<210> 14
   <211> 11
   <212> PRT
   <213> Artificial Sequence
<220>
   <223> peptide
<400> 14
   Glu Val Thr Gly Ile Ile Thr Gln Gly Ala Arg
   1 5 10

## Description

### PRIORITY CLAIM

This application claims the benefit of U.S. Provisional Application No. 61/154,329, filed February 20, 2009.

### FIELD

This application relates to the field of vascular disease such as atherosclerosis, more specifically to methods for diagnosis of age-associated vascular disorders.

### BACKGROUND

The age-inflamed arterial wall is a predisposed territory for the pathogenesis of proliferative vascular diseases, for example atherosclerosis, in the elderly. Age-associated molecular, structural, and functional consequences contribute to enrichment of proinflammatory proteins, intimal medial thickening, and arterial stiffness. These changes set up a stage for the pathogenesis of vascular disease that leads to morbidity and mortality in many societies all over the world. Although the underlying cellular and molecular mechanisms of age-associated vascular disease are largely undefined, this arterial aging process is believed to be determined at least partially by changes in the population, location, and behavior of vascular smooth muscle cells (VSMCs), the most predominant cell type residing within the vascular wall.

Proinflammatory processes and associated elevated invasion capacity of VSMCs are increased within the diffused thickening of the arterial wall that evolves with advancing age. In humans, this age-associated arterial remodeling is an independent risk factor for the epidemic of quintessential human cardiovascular diseases, including hypertension, atherosclerosis, and stroke. Age-associated arterial inflammation, including intimal thickening and other aspects of arterial remodeling, is evolutionarily conserved in various mammalian species, including rodents, nonhuman primates, and humans.

The thickened arterial intima is formed due to VSMC invasion and proliferation, and matrix secretion within the sub-endothelial space. A growing body of evidence indicates that VSMCs within the arterial media begin to express and activate proteases, for example matrix metalloprotease and calpain-1. Protease 5 activation enables cytoskeletal remodeling: degradation of (1) basement membranes that surround VSMC, (2) adjacent matrix, and (3) elastic lamina; and invasion into the sub-endothelial space driven, at least in part by angiotensin II (Ang II). VSMC invasion is also facilitated by an intimal-medial concentration gradient of monocyte chemoattractant protein-1 (MCP-1) and platelet-derived growth factor (PDGF), both of which are potent inflammatory chemoattractants for VSMC.

Age-associated vascular disorders are a major health risk throughout the industrialized world. Atherosclerosis, the most prevalent of such vascular disorders, is the principal cause of heart attack, stroke, and gangrene of the extremities. It is also a principal cause of death in the United States. 'Thus, there remains a need for methods to diagnose and/or treat age-associated vascular disorders, such as atherosclerosis.

Peng et al (Amyloid: The International Journal of Experimental and Clinical Investigation, vol. 12, June 2005, pages 96-102) describes medin-amyloid.

### SUMMARY

Disclosed herein are methods for diagnosing or prognosing age-associated vascular disorders in a subject. The methods include obtaining a biological sample from the subject and determining whether there is altered expression of a gene product of Milk Fat Globule Protein Epidermal Growth Factor-8 (MFG-E8) or at least one or more of the genes listed in Table 1, relative to a reference level of expression. An alteration in the expression of the one or more gene products indicates that the subject has or is at risk of an age-associated vascular disease. In some examples of the disclosed methods, a control level is the level of expression of gene product: in a biological sample obtained from the same subject at an earlier time point; in a biological sample obtained from another subject that does not have the age-associated vascular disorder; in a biological sample obtained from a younger individual; or a statistical reference value indicative of expression in a subject that does not have the age-associated vascular disorder. In some examples, the level of a MFG-E8 gene product, or the gene product of at least one other gene listed in Table 1, is determined relative to a reference level. An increase in the level of expression of the gene or gene product of MFG-E8, or at least one other gene or gene product listed in Table 1, relative to the control indicates that the subject has or is at risk of an age-associated vascular disease.

In some embodiments of the disclosed method, detection of altered expression of a gene product includes detecting the MFG-E8 protein or at least one other gene product listed in Table 1. In specific examples, detecting protein includes producing a protein digest from the biological sample, optionally fractionating the protein digest, for example by chromatographing the protein digest, and detecting a fragment of the protein listed in Table 1 in the protein digest. In some examples of the disclosed methods, the protein or a fragment thereof is detected with mass spectrometry, for example tandem mass spectrometry. Optionally the protein or peptide fragment thereof is quantitated. In some examples, the protein or peptide fragment thereof is quantitated by comparing an amount of the fragment peptide to a peptide standard of known amount, which can optionally be isotopically labelled peptide, for example with one or more heavy stable isotopes selected from ¹⁸O, ¹⁷O, ³⁴S, ¹⁵N, ¹³C, ²H, or a combination of two or more thereof. In some examples, a post translational modification of a protein gene product, or a fragment thereof, can be evaluated, for example to determine the level of post translationally modified protein or protein fragment. In some examples a post translational modification of a protein gene product can be used to purify the protein, for example using antibodies specific for the post translational modification.

In some embodiments of the disclosed methods, the product of the gene listed in Table 1 is a nucleic acid, such as an mRNA, and detecting expression includes detecting nucleic acids. By way of example, reverse-transcription-Polymerase chain reaction (RT-PCR), such as quantitative RT-PCR, or mass spectrometry, is used in some embodiments to detect an mRNA expressed from a gene listed in Table 1.

The invention provides a method for diagnosing or prognosing an age-associated vascular disorder in a subject, the method comprising detecting an altered expression level of a gene product of Milk Fat Globule Protein Epidermal Growth Factor-8 (MFG-E8), and of a gene product of at least three other genes increasing in expression as a function of age and selected from HTRA1, ITM2B, CSRP2, PLF4, IBP7, RBP1, ARPC4, CNN1, CNN3, TGFB3, CLUS, MMP2, PGS1, FINC, SPRL1, VTNC, KNT1, POSTN, APOE, TRI47, ADCY5, GTR4, and FRIL1 in the biological sample, relative to a reference level, in a biological sample obtained from the subject, thereby diagnosing or prognosing an age-associated vascular disorder in the subject.

The foregoing and other features of the disclosure will become more apparent from the following detailed description, which proceeds with reference to the accompanying figures.

### BRIEF DESCRIPTION OF THE DRAWINGS

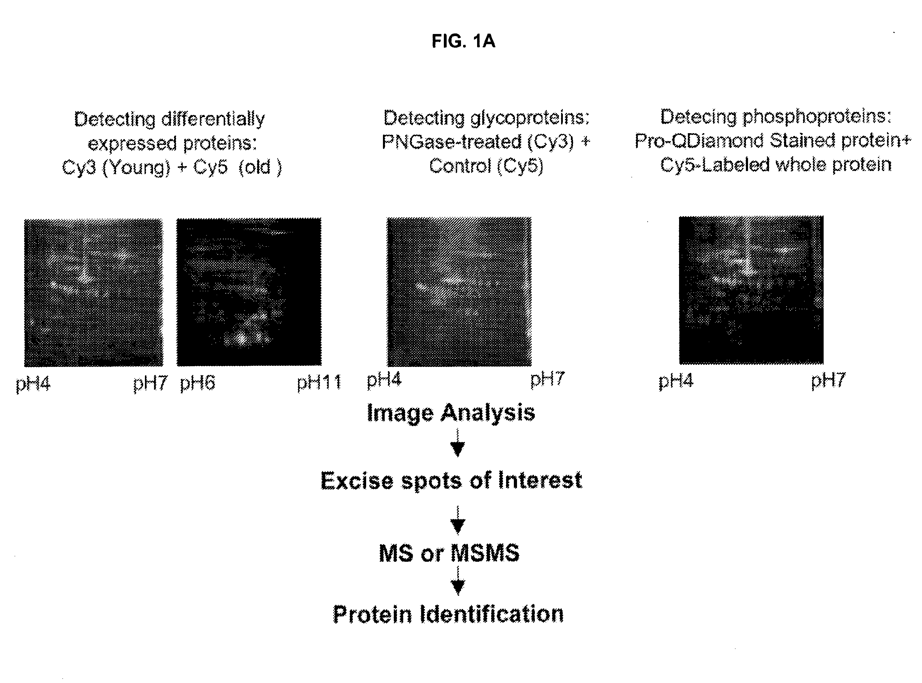

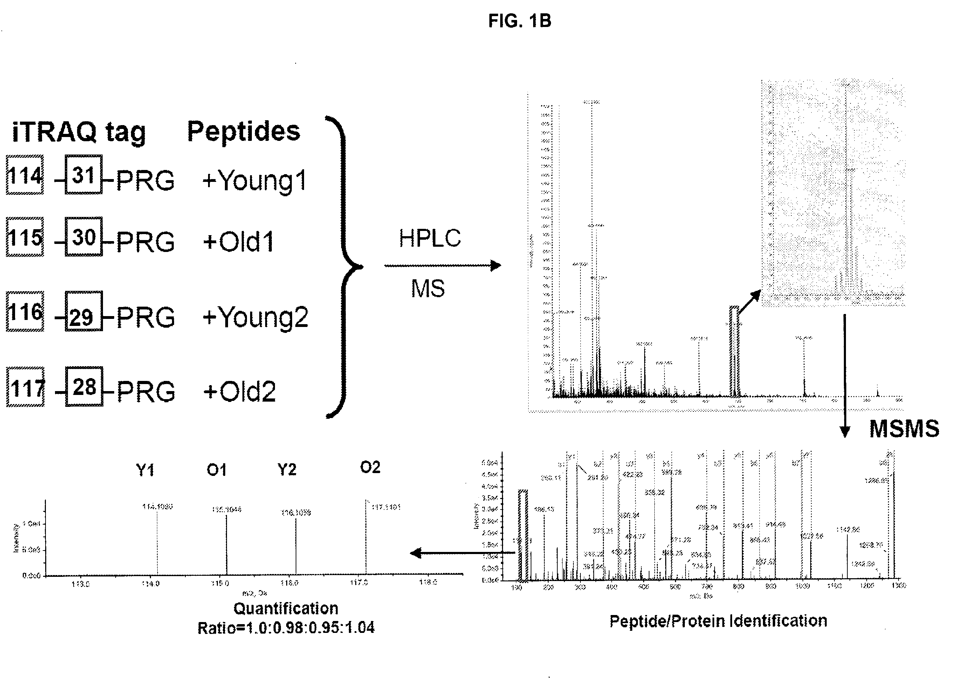

**FIG. 1A** and **1B** are schematics of an exemplary discovery platform utilized to quantify and characterize the rat arterial proteome. FIG. 1A is a set of digital images of exemplary 2-Dimensional gel electrophoresis-based (2DE) multiplex methods (pH 4-7 and 6-11) used to detect differentially-expressed proteins (DIGE), phosphoproteins based on ProQ® diamond staining and glycoproteins based on deglycosylation using PNGase F (specific for N-linked glycoproteins) in aortic tissue protein lysates obtained from young (8 month) and old (30 month) rats (n=8). FIG. 1B shows an example of a mass spectroscopic (MS) based isobaric tag for relative and absolute quantitation (iTRAQ™) methods used to simultaneously quantify differences between four samples. Tryptic peptides obtained from each test sample were independently labeled with either iTRAQ™ tag 114, 115, 116 or 117, combined, and separated by ion exchange chromatography prior to analysis by liquid chromatography (LC) MS/MS. Four biological and two technical replicates were performed.

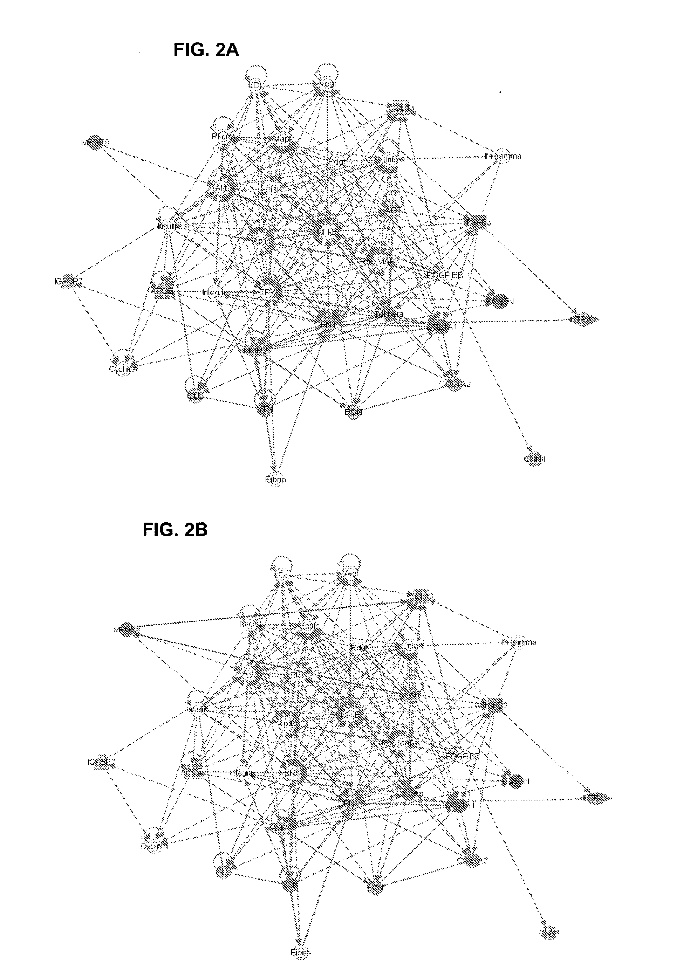

**FIG. 2A** and **2B** is a schematic representation of a dynamically computed basic biological network (FIG. 2A) and an updated network using the findings disclosed herein (FIG. 2B) using the Ingenuity Pathway Analysis program for proteomic expression data from young and old rats.

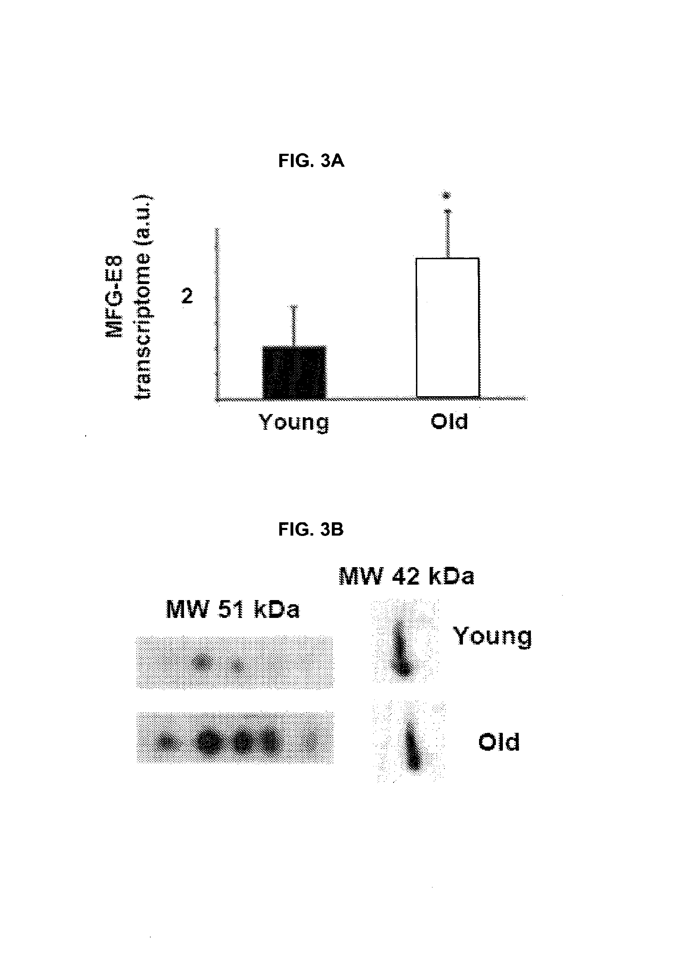

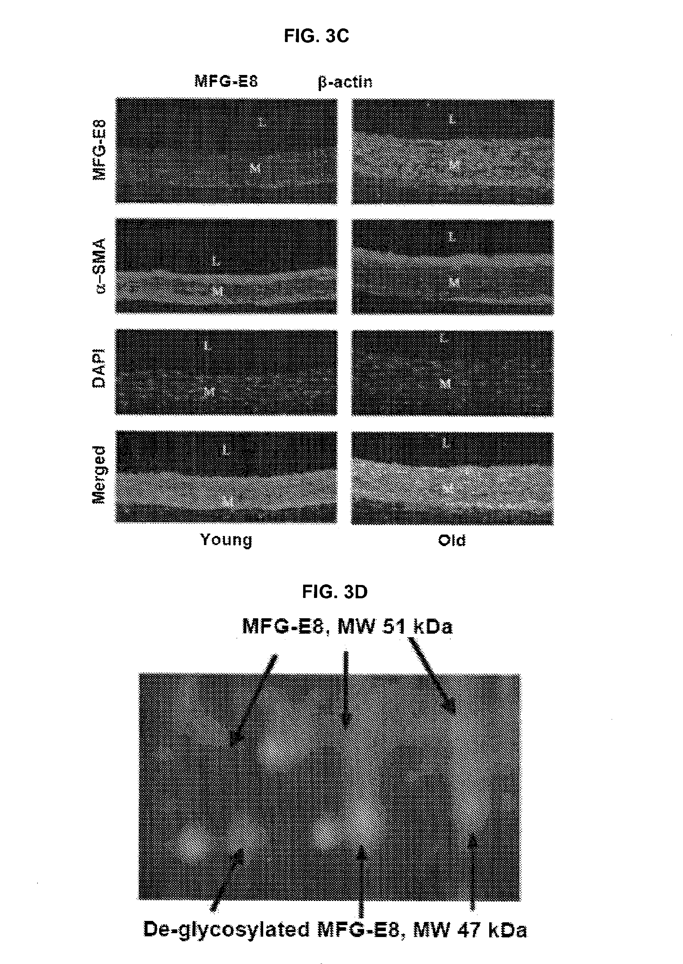

**FIG. 3A-3D** illustrate the validation and the location of MFG-E8 expression within the rat aortic wall. FIG. 3A is a bar graph showing the average MFG-E8 mRNA (transcriptome) levels (n=4) in old and young rat aorta. FIG. 3B is a digital image of a 2DE Western blot of MFG-E8 and β-actin. β-actin is an internal loading control. FIG. 3C is a digital image of immunofluorescence staining for MFG-E8 and α-SMA. Nuclei were counterstained with a DAPI dye. Staining in the merged images represents staining overlap (original magnification X200). FIG. 3D is a digital image of an enlarged region of 2DE DIGE showing PNGase F-treated MFG-E8 compared to an untreated endogenous sample from old rat aorta. PNGase F treatment results in the removal of N-linked carbohydrates shifting the position of MFG-E8 to a lower molecular weight.

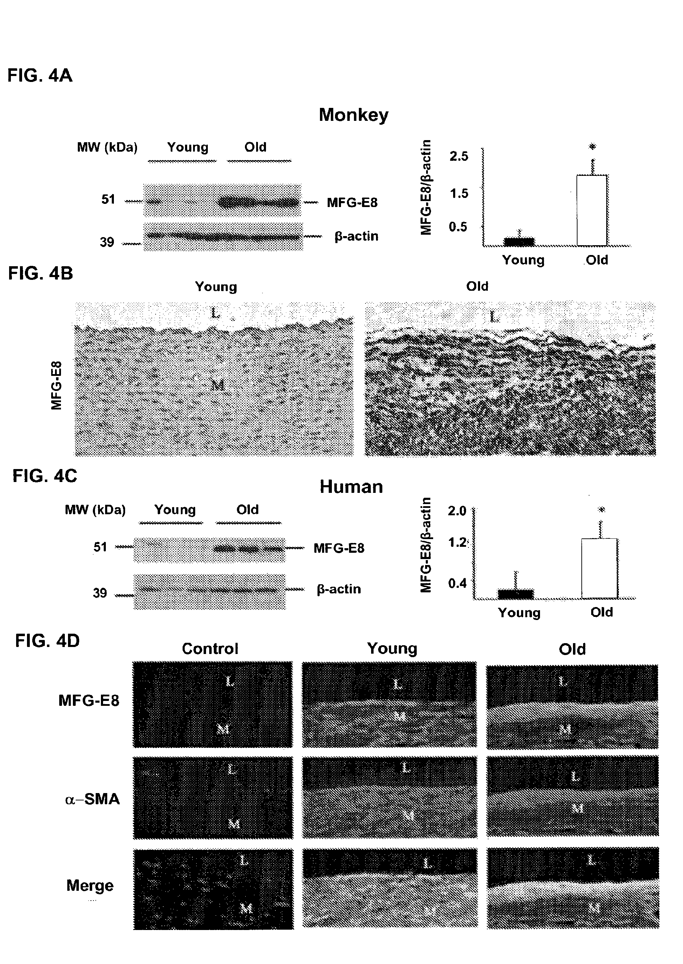

**FIG 4A-4D** illustrate that the age-associated arterial MGF-E8 increase is conserved in mammalian species, including humans. FIG. 4A is a digital image of representative Western blots of young and old monkey aortic MFG-E8 (left panel) and average data (graph representing levels of MFG-E8 normalized to the amount of β-actin in the young and old samples; right panel, n=4). *p <0.05, old versus young. FIG. 4B is a digital image showing immunohistostaining for MFG-E8 within the young and old monkey thoracic aortic wall (original magnification X100). L=lumen; M=media. FIG. 4C is a digital image of representative Western blots of young and old human aortic MFG-E8 (left panel) and a graph representing average levels of MFG-E8 normalized to the amount of (3-actin in the young and old samples (right panel, n = 5). *p <0.05, old versus young. FIG. 4D is a digital image of double immunofluorescence staining for MFG-E8 and α-SMA and a merged image within the young and old human thoracic aortic wall (original magnification X100). Staining in the merged images represents staining overlap. L=lumen; M=media.

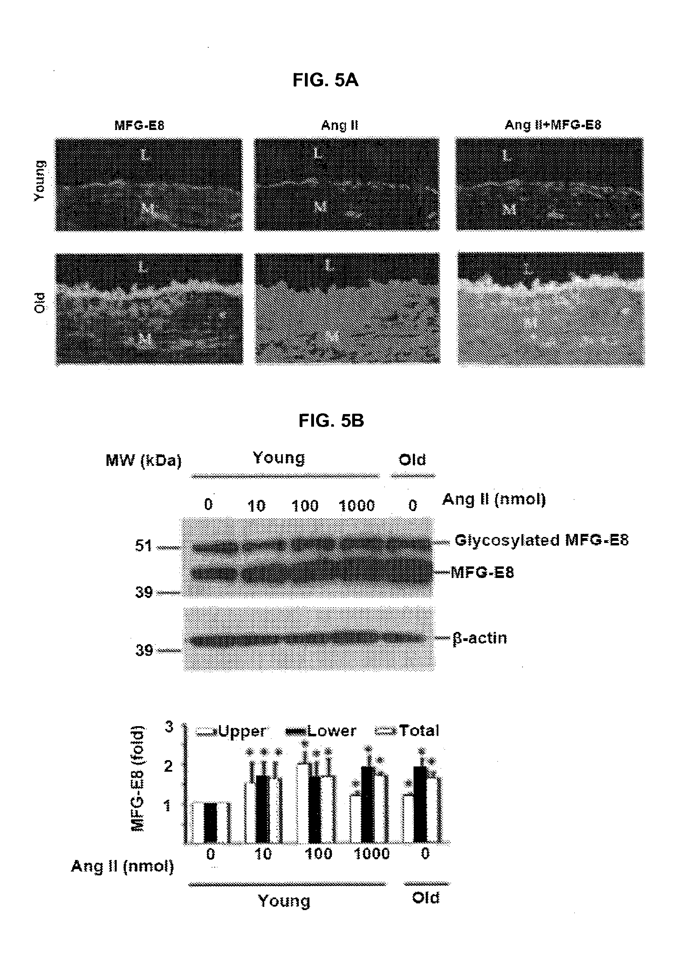

**FIG. 5A** and **5B** illustrate that Ang II and MFG-E8 colocalize *in vivo* and *in vitro.* FIG. 5A is a digital image of immunofluorescence staining for MFG-E8 and Ang II and a merged image in the young and old rat aortic wall. Nuclei were counterstained with DAPI (original magnification X 400). L=lumen; M=media. Staining in the merged images represents staining overlap. FIG. 5B, upper panel, is a digital image of representative one dimensional gel electrophoresis (1DE) Western blots of MFG-E8 in smooth muscle cells isolated from young or old rat aorta treated with or without Ang II with the indicated concentration levels (upper panels). The MFG-E8 immunoreactivity doublet is the N-linked glycosylated form (upper bands) and the unglycosylated form (lower bands), as shown by deglycosylation of the sample with PNGase F, which results in the enhancement of the lower molecular weight (MW) band. Average data (FIG. 5B, lower panel). *p<0.05 old untreated and young treated cells versus young control cells.

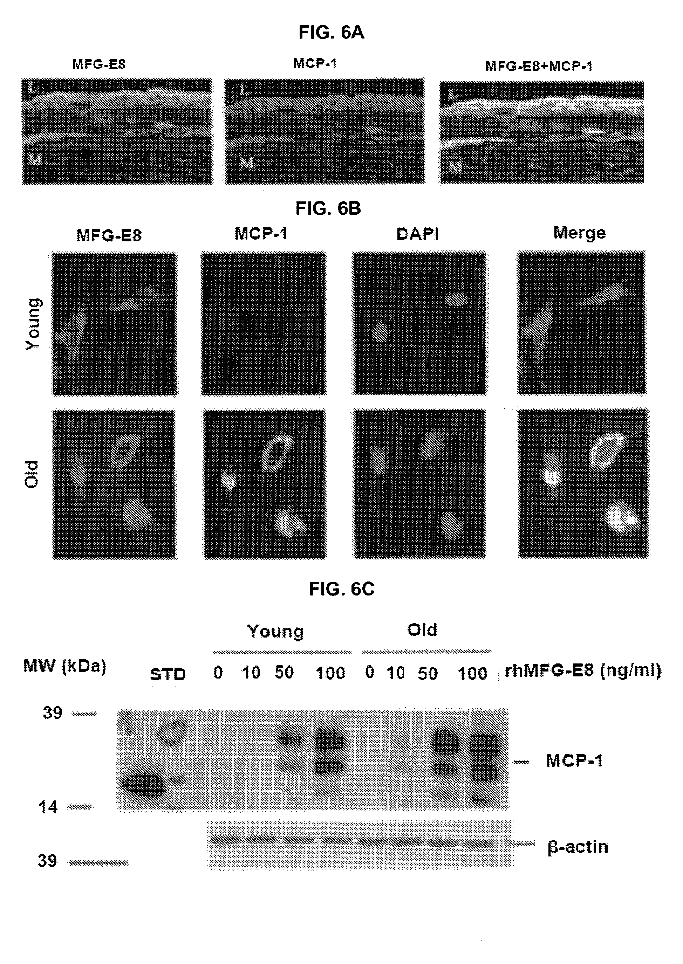

**FIG. 6A-6C** show the interaction between MFG-E8 and MCP-1 *in vivo* and *in vitro.* FIG. 6A is a set of digital images of immunofluorescence staining for MFG-E8 and MCP-1 and a merged image in old human aorta. Staining in the merged image represents staining overlap. Nuclei were counterstained with DAPI (Original magnification X200). L=lumen; M=media. FIG. 6B is a set of digital images showing immunofluorescence photomicrographs (x400) for MFG-E8, MCP-1, nucleic (DAPI), and colocalization of MFG-E8 and MCP-1 (merged images) within VSMCs. FIG. 6C is a digital image of representative Western blots showing that the functional dimer of MCP-1 increases in vascular smooth muscle cells (VSMCs) treated with MFG-E8 in both young and old cells in a dose-dependent manner.

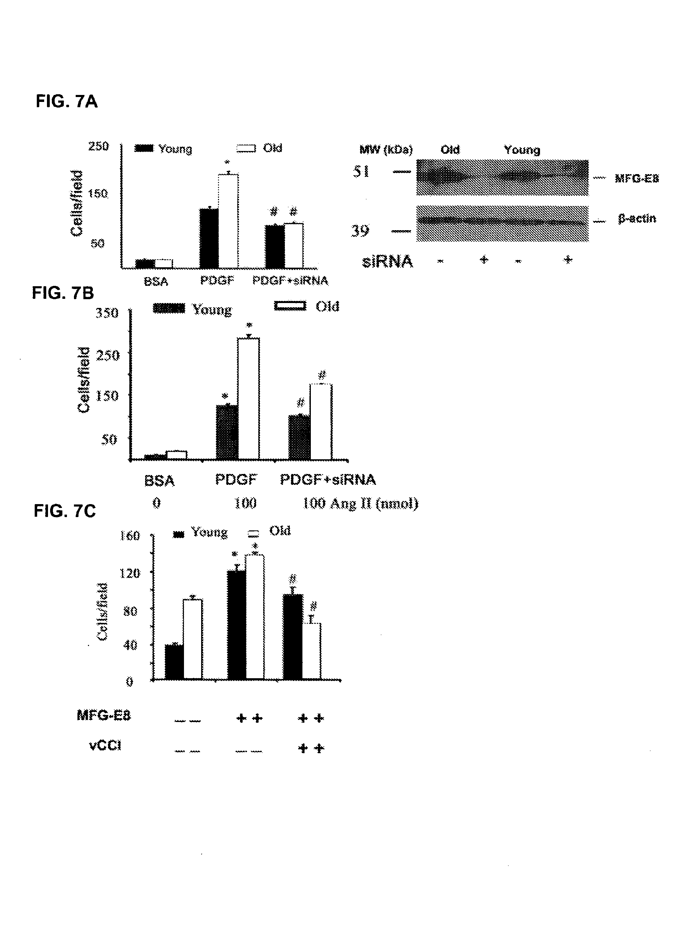

**FIG. 7A-7C** is a set of bar graphs and Western blots of a VSMC invasion assay in a modified Boyden chamber analysis. FIG. 7A shows the average invasion analysis of VSMC with and without siRNA targeting MFG-E8 (left panel). *p<0.05, old versus young cells chemoattracted by PDGF (10ng/ml); #p<0.05, young and old cells with MFG-E8 silencing versus sham silencing, respectively. Representative Western blots (right panel) show that the majority of MFG-E8 protein expressed in old and young VSMCs is knocked down *via* siRNA silencing. FIG. 7B shows the average invasion analysis of VSMCs with and without siRNA of MFG-E8 and Ang II treatment. *p<0.05, old versus young cells when treated with Ang II (100 nmol) and chemoattracted by PDGF (10ng/ml); #p<0.05, young and old cells with MFG-E8 silencing versus sham silencing, respectively. FIG. 7C shows the average invasion analysis of VSMC treated with and without MFG-E8 plus vCCI (a CCR2 blocker). *p<0.05 young and old cells treated with MFG-E8 (50ng/ml) versus untreated controls respectively; #p<0.05, young and old cells treated with MFG-E8 plus vCCI versus MFG-E8 treated, respectively.

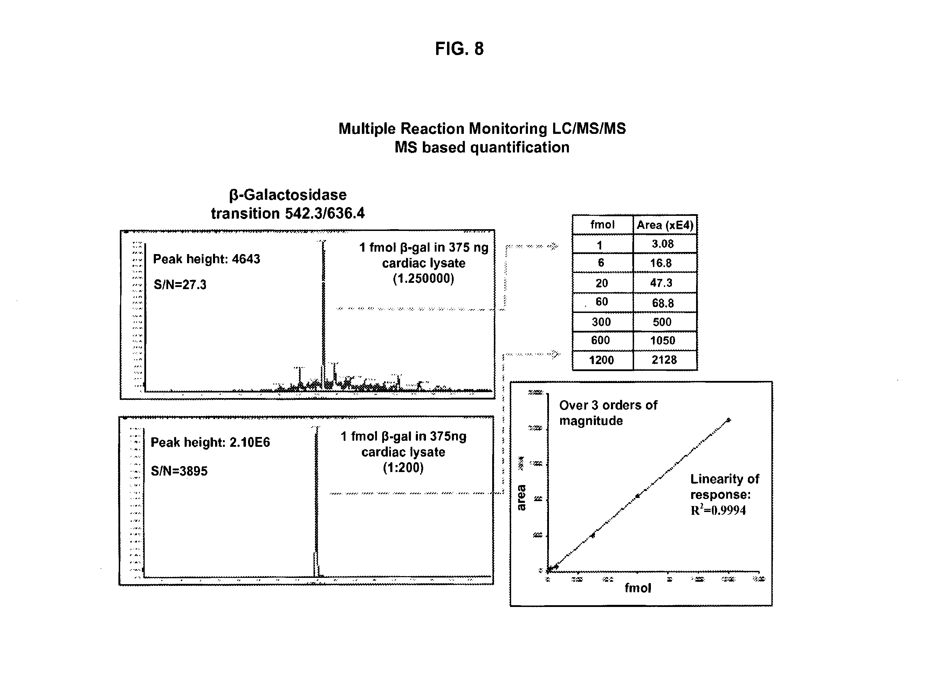

**FIG. 8** is a schematic representation of multiple reaction monitoring (MRM) MS-based quantitative studies that may be performed on serum samples from a number of different age groups, to determine the presence and amount of circulating forms of MFG-E8 present in serum. MRM can be used for any protein or combination of proteins. In addition, the status of post-translational modification as well as total protein concentration can be determined.

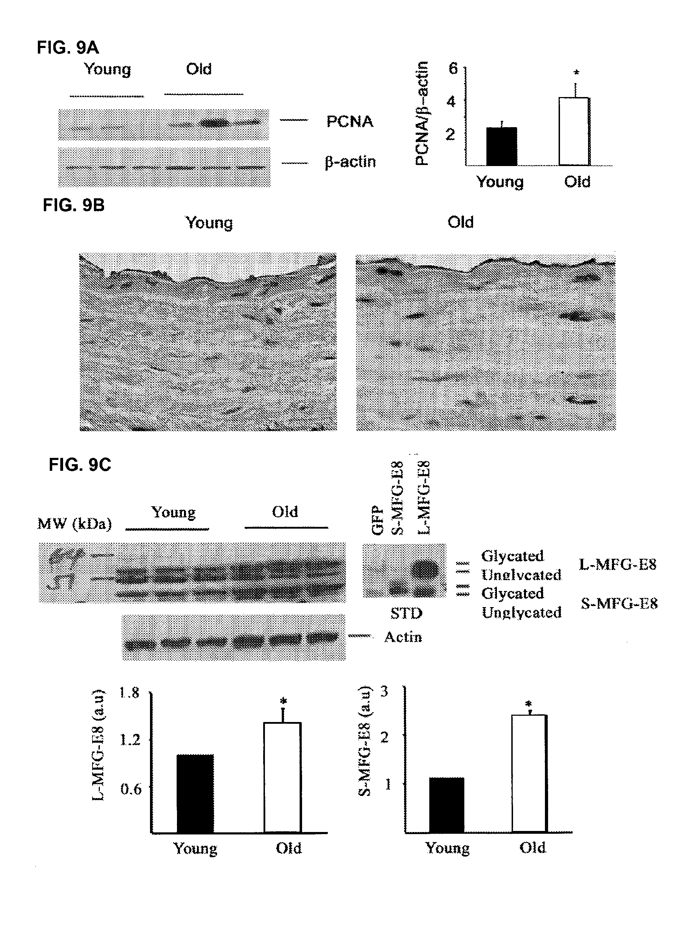

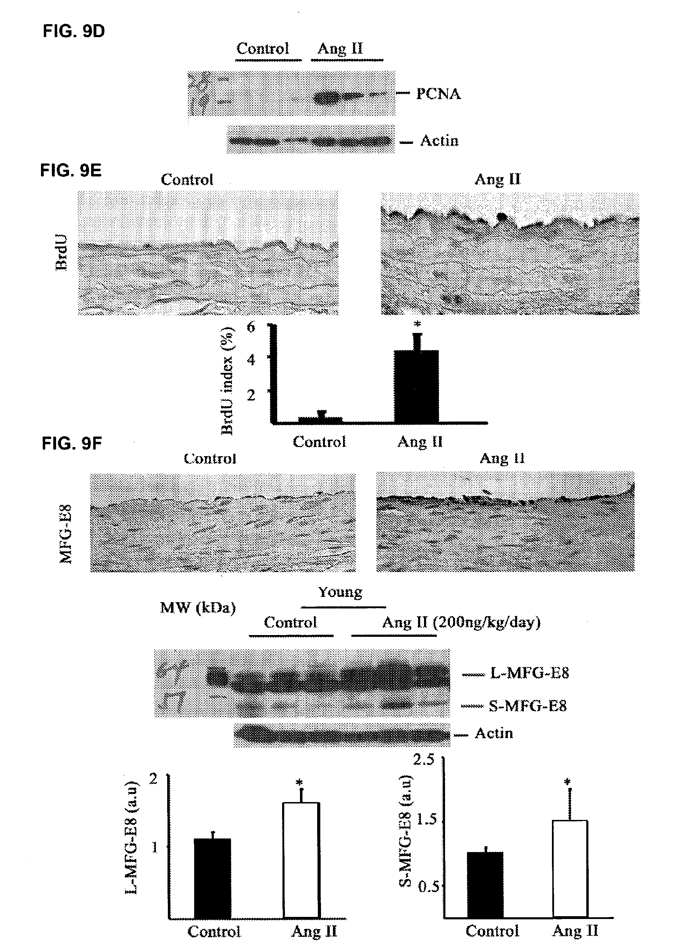

**FIG. 9A-9F** is a set of bar graphs and digital images showing alterations in VSMC proliferation. FIG. 9A is a Western blot showing the change in PCNA expression with aging (left panel) and average data (right panel). FIG. 9B is a series of photomicrographs of immunostaining for PCNA in aortic walls from young and old rats (X400). FIG. 9C is a series of three Western blots of MFG-E8 and average data (two bar graphs). Right panel demonstrates that Long and Short forms of MFG-E8 (L-MFG-8 and S-MFG-E8) were expressed in VSMC infected with adenovirus serving as a positive control. FIG. 9D are Western blots of PCNA expression in arterial wall of young rats infused Ang II. FIG. 9E is a series of digital images representing immunohistostaining for BrdU (upper panels) and average data. FIG. 9F is a series of digital images representing immunohistostaining for MFG-E8 (upper panels), Western blots of MFG-E8 (middle panel) and average data (lower panels). *p< 0.05, old versus young.

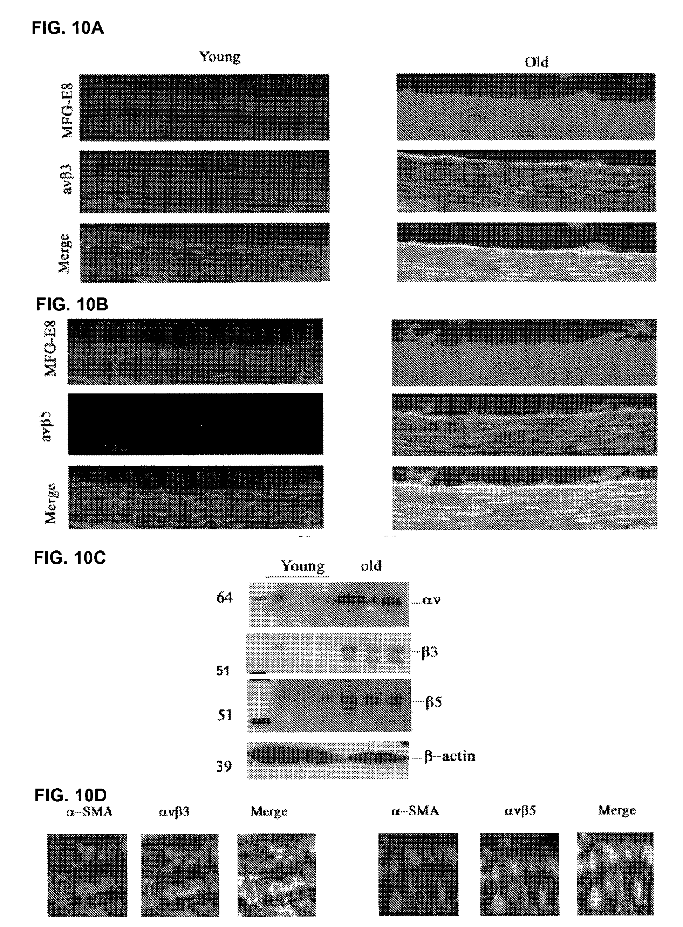

**FIG. 10A-10D** is a set of digital images showing expression of MFG-E8 and integrin receptors within the aortic wall. FIG. 10A is a series of digital images of aortic fluorescence micrographs of MFG-E8 (upper panels) and αvβ3 staining (middle panels) and merged (lower panels) from young (left panels) and old (right panels) (X400). FIG. 10B is a series digital images of aortic fluorescence micrographs of MFG-E8 (upper panels) and αvβ5 staining (middle panels) and merged (lower panels) from young (left panels) and old (right panels). FIG. 10C is a series of Western blots of αv, β3, and β5 (X400). FIG. 10D is a series of digital images of aortic medial fluorescence micrographs of α-SMA and αvβ3 or αvβ5 staining and merged (X1000).

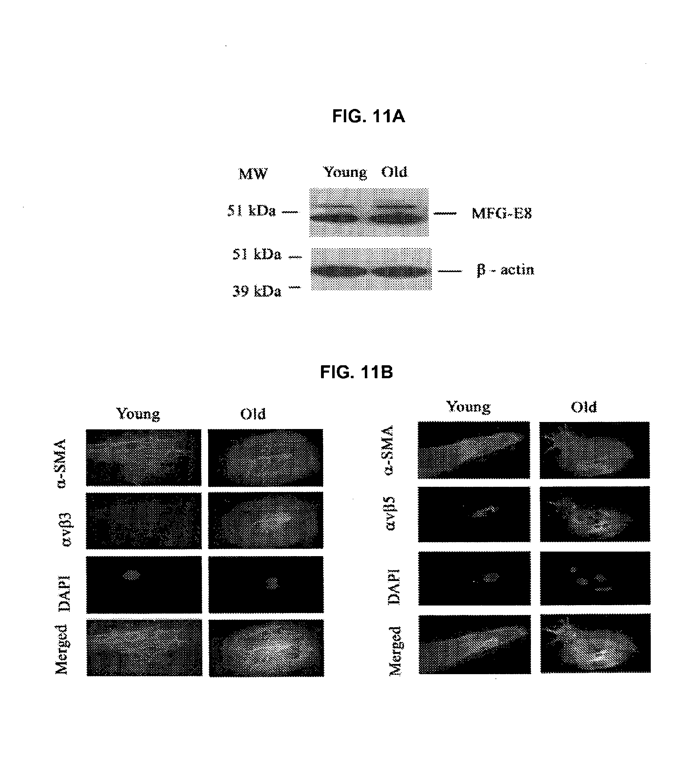

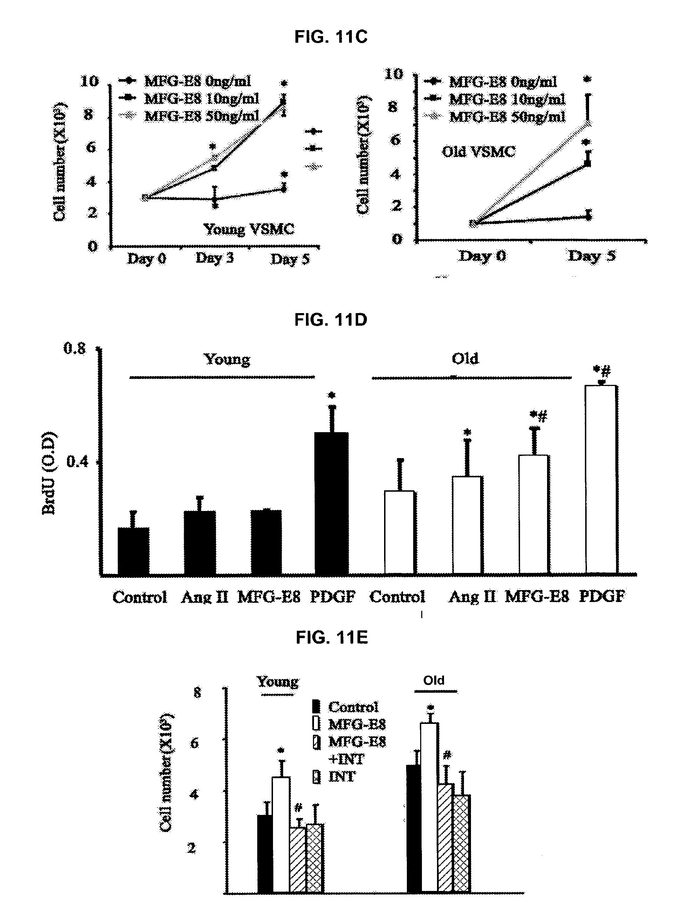

**FIG. 11A**-**11E** is a set of digital images and graphs showing that MFG-E8 promotes proliferation of VSMC via integrin signaling. FIG. 11A are representative Western blots of VSMC MFG-E8. FIG. 11B is a set of digital images of VSMC fluorescence macrographs of α-SMA, αvβ3 or αvβ5, DAPI, and merged (X400). FIG. 11C is a set of graphs showing a proliferative assay by a MTT assay of young (left panel) and old VSMC (right panel), treated with various concentrations, or without, MFG-E8. * p<0.05, one-way ANOVA. FIG. 11D is a graph showing a proliferative assay by BrdU incorporation of young (left panel) and old VSMC (right panel) treated with or without Ang II, MFG-E8, and PDGF (positive control). *p<0.05, old versus young control, #p<0.05, old versus corresponding young group. FIG. 11E is a graph showing proliferation of young VSMC treated with or without MFG-E8 and neutralizing integrin antibodies. *p<0.05 versus control, #p,0.05 versus MFG-E8 treatment.

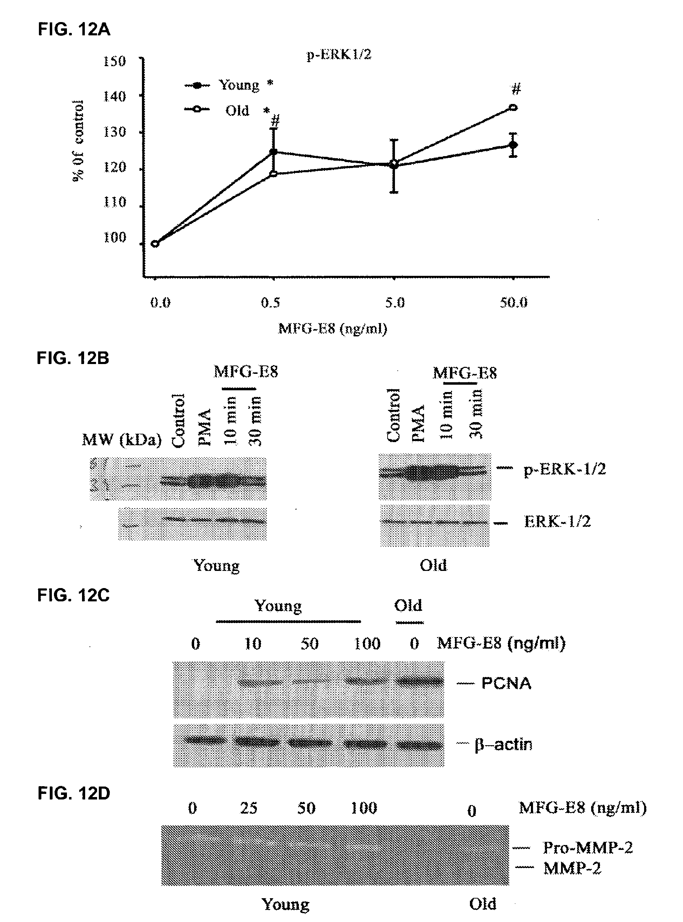

**FIG. 12A-12D** is a set of graphs and Western blots showing that increased MFG-E8 triggers proliferation-associated signaling molecule expression. FIG. 12A is a line graph showing the effects of MFG-E8 on p-ERK1/2, as determined by an ELISA assay. *p<0.05 one-way ANOVA. FIG. 12B is a set of Western blots showing the effects of MFG-E8 on p-ERK1/2. FIG. 12C is a set of Western blots showing the effects of MFG-E8 on PCNA expression. FIG. 12D is a polyacrylamide gel electrophoresis (PAGE) gelatin zymogram.

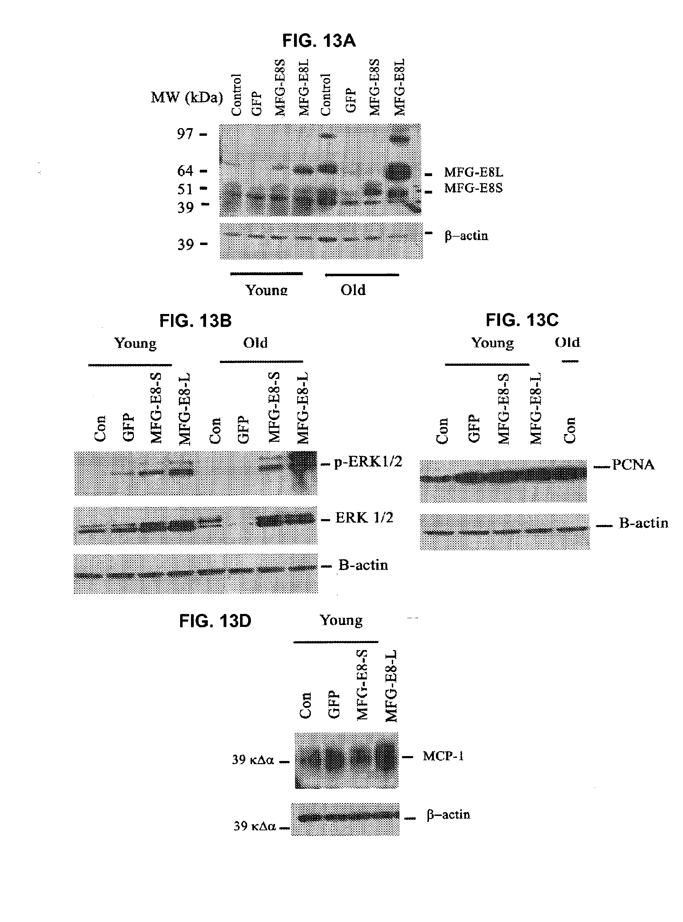

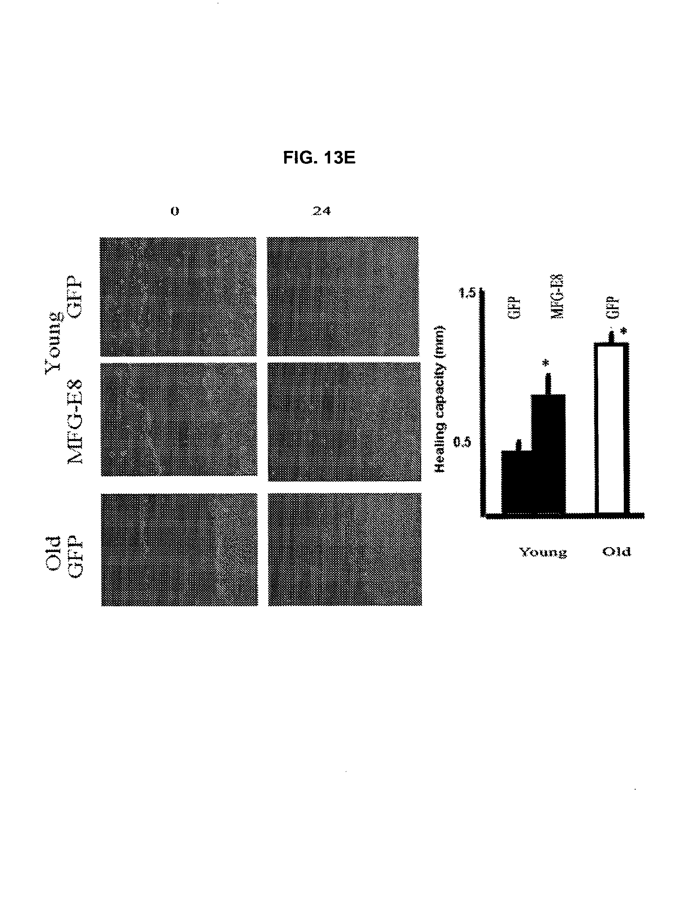

**FIG. 13A-13E** is a set of digital images showing that over-expression of ectopic MFG-E8 drives the progression of the cell cycle, and enhances proinflammation and wound-healing capacity. FIG. 13A shows the efficiency of MFGE8 expression in young and old VSMC infected with adenovirus infection. FIG. 13B shows Western blots demonstrating the effect of MFG-E8 over-expression on ERK1/2 phosphorylation. FIG. 13C is a series of Western blots of PCNA abundance. FIG. 13D is a series of Western blots of MCP-1 in young VSMC infected with adenovirus. FIG. 13E is a series of photographs of a wound-healing assay and average data. *p<0.05 versus young.

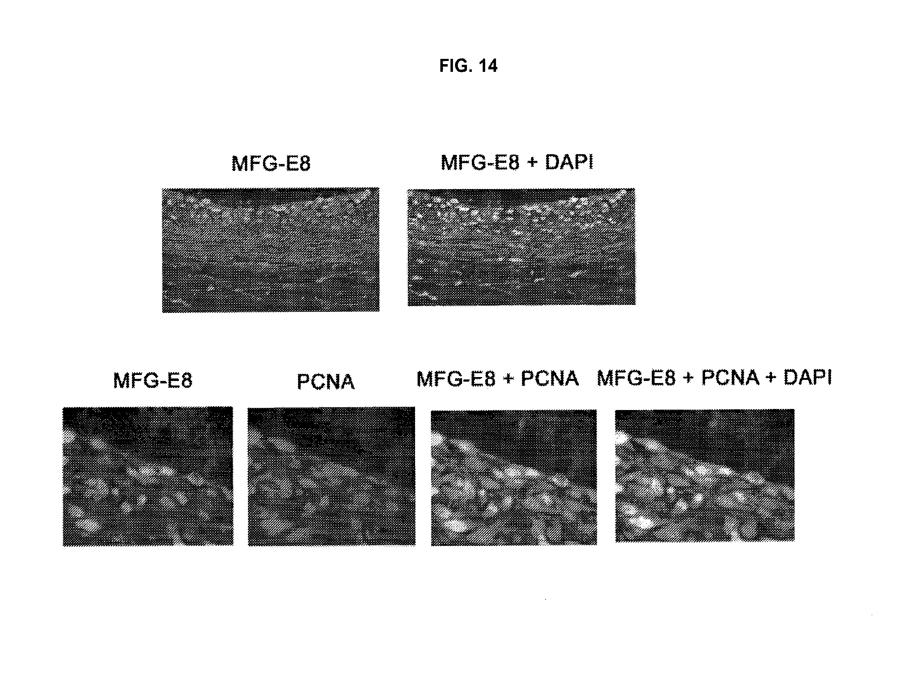

**FIG. 14** is a set of digital images showing that MFG-E8 expression is up-regulated post angioplasty within the neointima. The figure shows representative immunofluorescence micrographs of MFG-E8, PCNA, and DAPI staining in injured carotid arteries.

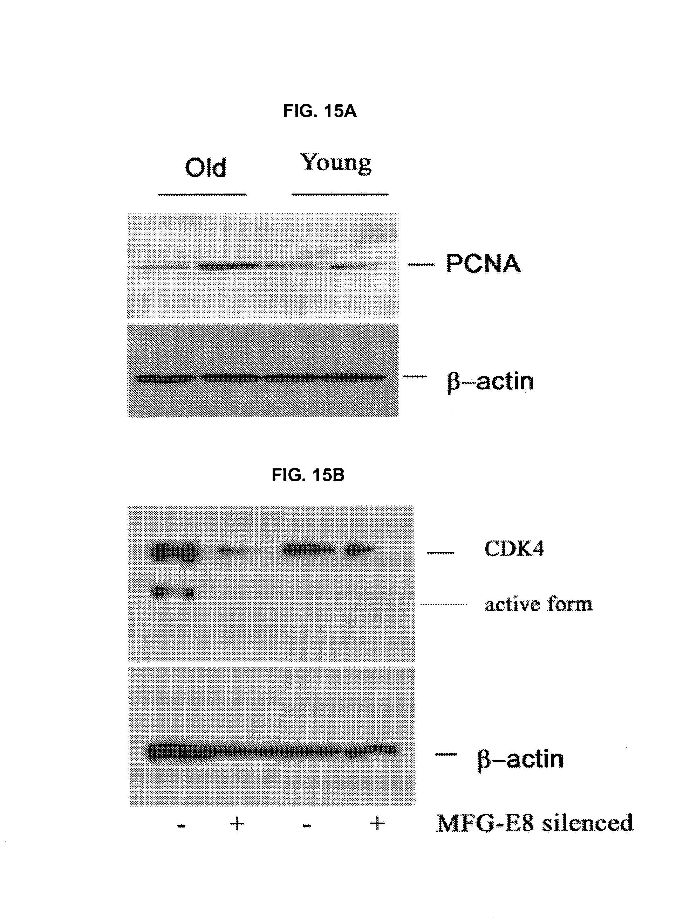

**FIG. 15A** and **15B** is a set of Western blots showing that silencing MFG-E8 affects a withdrawal from the cell cycle. FIG. 15A is a representative Western blot of PCNA expression in old and young VSMC in the presence of MFG-E8 siRNA. FIG. 15B is a representative Western blot of CDK-4 expression in old and young VSMC in the presence of MFG-E8 siRNA.

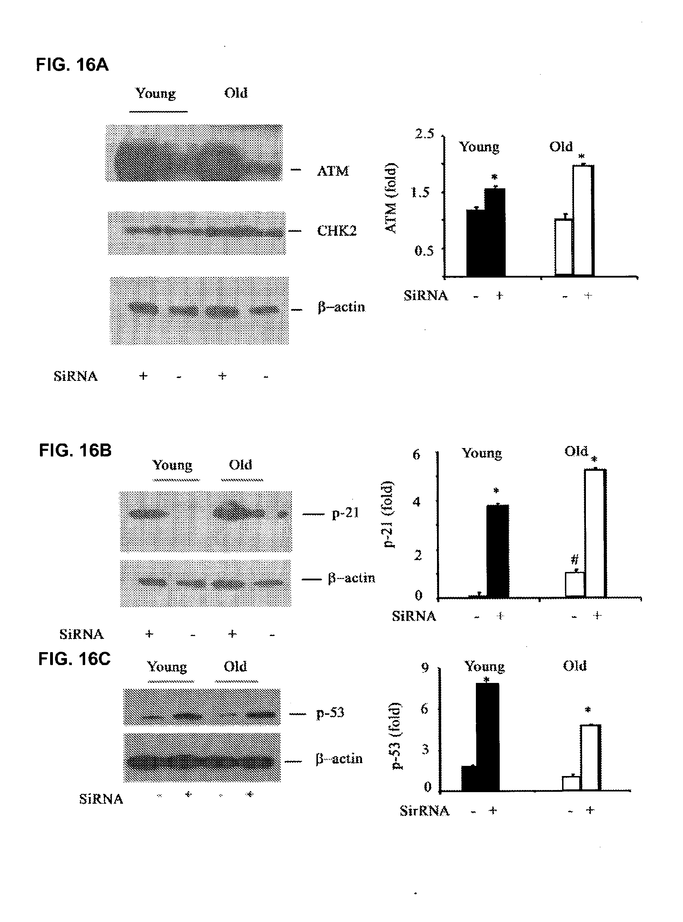

**FIG. 16A-16C** is a series of bar graphs and Western blots showing that silencing MFG-E8 induces senescence-associated signaling molecule expression. FIG. 16A is a series of representative Western blots of ATM and CHK2 expression (left panels) in the presence of MFG-E8 siRNA and average data of ATM (right panel). FIGs. 16B and 16C are representative Western blots of p21 and p53 (left panels) and average data (right panels). *p<0.05 silencing versus age-matched control VSMC; #p<0.05, old versus young VSMC.

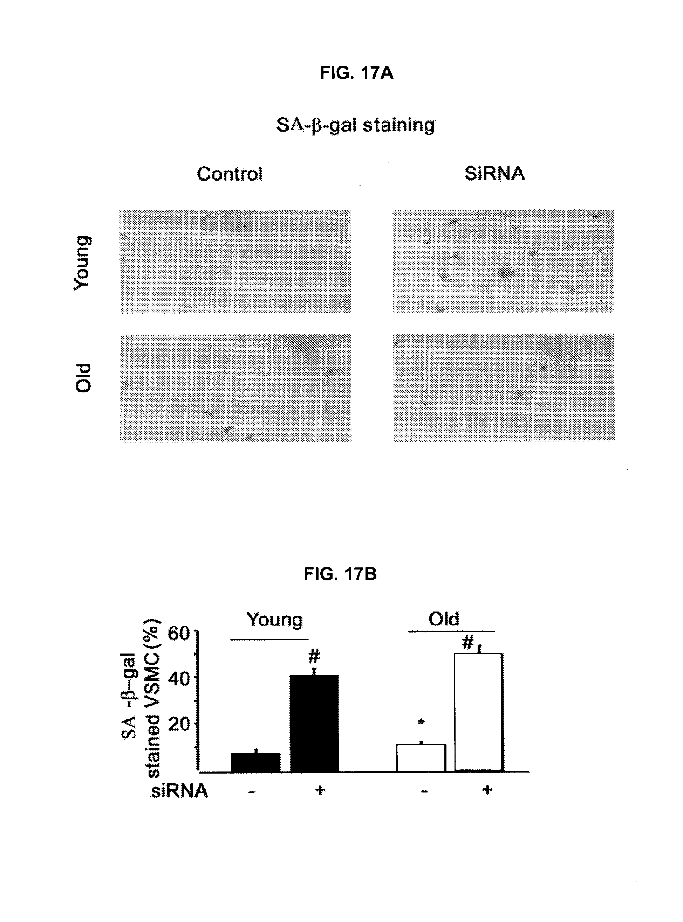

**FIG. 17A** and **17B** is a series of digital images and bar graphs showing that silencing MFG-E8 increases SA-β-gal activity. FIG. 17A is a series of photomicrographs of βgal staining of young (upper panels) and old (lower panels). FIG. 17B is a series of graphs showing average data. *p< 0.05, old versus young VSMC; #p<0.05 silencing versus age-matched VSMC.

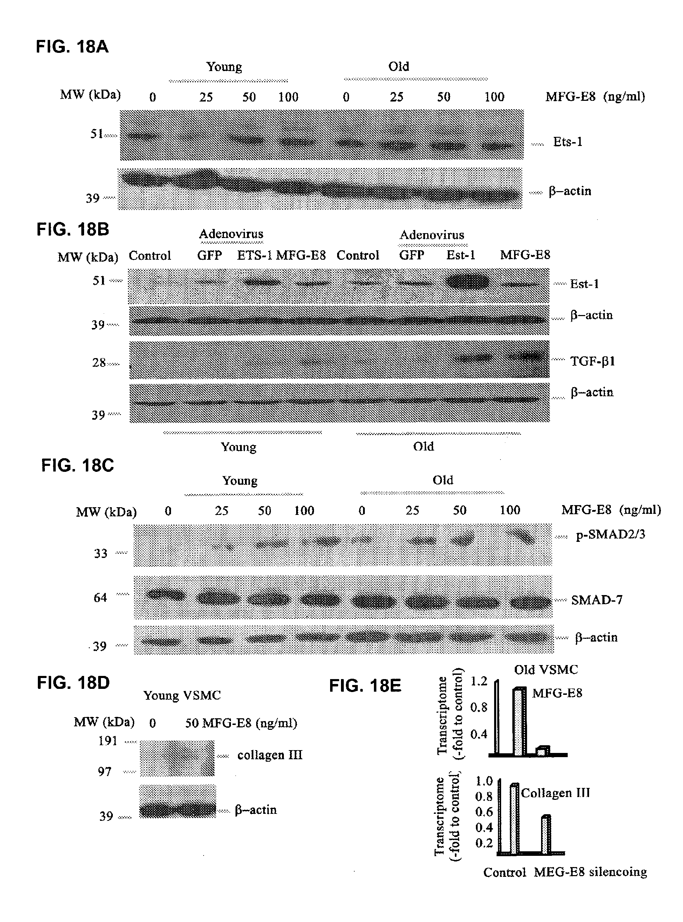

**FIG. 18A-18E** are a series of Western blots and bar graphs showing the effect of exogenous MFG-E8 treatment or over-expression of MFG-E8. FIG. 18A is a Western blot showing that exogenous MFG-E8 treatment or over-expression significantly increases transcription factor Ets-1 expression in both young and old VSMCs. FIG. 18B is a Western blot showing that MFG-E8 treatment and Ets-1 over-expression markedly enhances the activity of TGF-β1, a powerful profibrogenic cytokine. FIG. 18C is a Western blot showing that treatment of young and old VSMCs with MFG-E8 increases phosphorylation of SMA2/3, but did not have an effect on SMAD7 expression. FIG. 18D is a Western blot showing that administration of MFG-E8 to young VSMCs increases collagen III production. In contrast, MFG-E8 knockdown decreases type III collagen gene transcription (FIG. 18E).

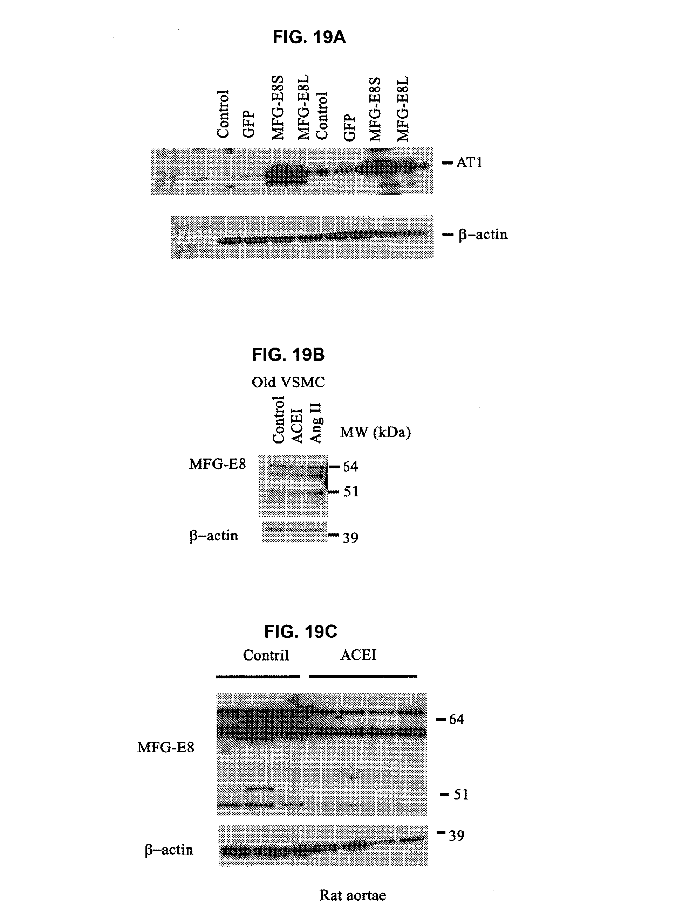

**FIG. 19A-C** is a series of Western blots showing the effect of over-expression of MFG-E8. FIG. 19A is a Western blot showing increases in AT1 protein expression in both young and old VSMCs in the presence of both MFG-E8S and MFG-E8L. FIG. 19B is a Western blot showing that angiotensin II (Ang II) strongly increases MFG-E8 expression. In contrast, angiotension converting enzyme (ACEI), Benazepril Hydrochloride, decreases MFG-E8 expression, particularly the short form, in old VSMC. FIG. 19C shows that strikingly, a chronic administration of Benazepril Hydrochloride (ACEI) to 8-month-old FXBN rats for 12 months via daily gavage, markedly decreased MFG-E8 expression, compared placebo animals.

### SEQUENCE LISTING

The nucleic and amino acid sequences listed in the accompanying sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. In the accompanying sequence listing:
SEQ ID NOs: 1, 2, and 9-14 are amino acid sequences of exemplary MFG-E8 fragment peptides.
SEQ ID NOs: 3 and 4 are nucleic acid sequences of PCR primers.
SEQ ID NO: 5 is an exemplary amino acid sequence of human MFG-E8.
SEQ ID NO: 6 is an exemplary nucleic acid sequence of human MFG-E8.
SEQ ID NO: 7 is an exemplary amino acid sequence of rat MFG-E8.
SEQ ID NO: 8 is an exemplary nucleic acid sequence of rat MFG-E8.

### DETAILED DESCRIPTION

### I. Abbreviations

- **1DE:**: 1-dimensional gel electrophoresis
- **2DE:**: 2-Dimensional gel electrophoresis
- **AGT:**: angiotensinogen
- **Ang II:**: angiotensin II
- **SCX:**: strong cation exchange chromatography
- **DIGE:**: differentially-expressed proteins
- **DTT:**: dithiothreitol
- **dsFv:**: disulfide stabilized Fv proteins
- **EKG:**: electrocardiogram
- **iTRAQ™:**: isobaric tag for relative and absolute quantitation
- **LC:**: liquid chromatography
- **L-MFG-E8:**: long form of MFG-E8
- **MCP-1:**: monocyte chemoattractant protein-1
- **MFG-E8:**: milk fat globule epidermal growth factor-VIII
- **MRM:**: multiple reaction monitoring
- **MS:**: mass spectroscopic
- **MTT:**: methylthiazoletetrazolium
- **PAD:**: peripheral artery disease
- **PCNA:**: proliferating cell nuclear antigen
- **PCR:**: polymerase chain reaction
- **PDGF:**: platelet-derived growth factor
- **PMT:**: photomultiplier tube
- **PTMs:**: post translational modifications
- **PVD:**: peripheral vascular disease
- **Q-RT-PCR:**: quantitative real-time PCR
- **RT:**: room temperature
- **RT-PCR:**: reverse-transcription-polymerase chain reaction
- **scFv:**: single chain Fv proteins
- **S-MFG-E8:**: short form of MFG-E8
- **SNP**: single nucleotide polymorphism
- **VSMCs:**: vascular smooth muscle cells

### II. Explanation of Terms

Unless otherwise noted, technical terms are used according to conventional usage. Definitions of common terms in molecular biology may be found in Benjamin Lewin, Genes VII, published by Oxford University Press, 2000 (ISBN 019879276X); Kendrew et al. (eds.), The Encyclopedia of Molecular Biology, published by Blackwell Publishers, 1994 (ISBN 0632021829); Robert A. Meyers (ed.), Molecular Biology and Biotechnology: a Comprehensive Desk Reference, published by Wiley, John & Sons, Inc., 1995 (ISBN 0471186341); and George P. Rédei, Encyclopedic Dictionary of Genetics, Genomics, and Proteomics, 2nd Edition, 2003 (ISBN: 0-471-26821-6).

As used herein, the singular terms "a," "an," and "the" include plural referents unless context clearly indicates otherwise. Similarly, the word "or" is intended to include "and" unless the context clearly indicates otherwise. Also, as used herein, the term "comprises" means "includes." Hence "comprising A or B" means including A, B, or A and B. It is further to be understood that all base sizes or amino acid sizes, and all molecular weight or molecular mass values, given for nucleic acids or polypeptides are approximate, and are provided for descriptive purposes, unless otherwise indicated. Although many methods and materials similar or equivalent to those described herein can be used, particular suitable methods and materials are described below. In case of conflict, the present specification, including explanations of terms, will control. In addition, the materials, methods, and examples are illustrative only and not intended to be limiting.

To facilitate review of the various embodiments of the invention, the following explanations of specific terms are provided:
**Age-associated vascular disorder:** A disorder of the blood vessels that is characterized by a remodeling of the vascular tissue as a function of age of the subject. While an age-associated vascular disorder can naturally occur as a result of the aging process, in some examples a subject exhibits premature aging of the vascular tissue. Such a subject could be diagnosed as having, or as predisposed to having, an age-associated vascular disease as the subject ages. Examples of age-associated vascular disorders include cardiovascular diseases, such as hypertension, atherosclerosis, peripheral vascular disease, coronary artery disease, myocardial ischemia, myocardial infarction (and ischemia or infarction of other organs) and stroke, arterial restenosis after angioplasty, and coronary heart disease, such as myocardial infarction, heart failure, and thrombosis.
**Amplifying a nucleic acid molecule:** To increase the number of copies of a nucleic acid molecule, such as a gene or fragment of a gene, for instance a nucleic acid molecule encoding a gene listed in Table 1, or a sub-sequence thereof. The resulting products are called amplification products.

An example *of in vitro* amplification is the polymerase chain reaction (PCR), in which a sample, such as a biological sample obtained from a subject, is contacted with a pair of oligonucleotide primers, under conditions that allow for hybridization of the primers to a nucleic acid molecule in the sample. The primers are extended under suitable conditions, dissociated from the template, and then re-annealed, extended, and dissociated to amplify the number of copies of the nucleic acid molecule. Other examples of *in vitro* amplification techniques include quantitative real-time RT-PCR, strand displacement amplification (see U.S. Patent No. 5,744,311); transcription-free isothermal amplification (see U.S. Patent No. 6,033,881); repair chain reaction amplification (see WO 90/01069); ligase chain reaction amplification (see EP-A-320 308); gap filling ligase chain reaction amplification (see U.S. Patent No. 5,427,930); coupled ligase detection and PCR (see U.S. Patent No. 6,027,889); and NASBA™ RNA transcription-free amplification (see U.S. Patent No. 6,025,134).

**Animal:** A living multicellular vertebrate organism, a category that includes, for example, mammals and birds. A "mammal" includes both human and non-human mammals, such as mice, rats, non-human primates, and humans. "Subject" includes both human and animal subjects.

**Antibody:** A polypeptide ligand comprising at least a light chain or heavy chain immunoglobulin variable region, which specifically recognizes and binds an epitope of an antigen, such as a protein gene product of any of the genes listed in Table 1 or a fragment thereof. Antibodies can be composed of a heavy and/or a light chain, each of which has a variable region, termed the variable heavy (V_{H}) region and the variable light (V_{L}) region. Together, the V_{H} region and the V_{L} region are responsible for binding the antigen recognized by the antibody. This includes intact immunoglobulins and the variants and portions of them well known in the art, such as Fab' fragments, F(ab)'₂ fragments, single chain Fv proteins ("scFv"), and disulfide stabilized Fv proteins ("dsFv"). The term also includes recombinant forms such as chimeric antibodies (for example, humanized antibodies) and heteroconjugate antibodies (such as, bispecific antibodies). See also, Pierce Catalog and Handbook, 1994-1995 (Pierce Chemical Co., Rockford, IL); Kuby, Immunology, 3rd Ed., W.H. Freeman & Co., New York, 1997.

**Arterial remodeling:** A lesion-specific process involving either enlargement or shrinkage of atherosclerotic arteries. Artery remodeling forms part of the large spectrum of vessel remodeling, and includes geometrical and structural changes of the vessel wall during the development (aging) and pathogenesis of quintessential cardiovascular diseases such as hypertension, atherosclerosis, and stroke. Positive remodeling (expansion) of early lesions maintains lumen size despite plaque accumulation. In contrast, negative remodeling (shrinkage) contributes to luminal stenosis independent of plaque accumulation.

**Atherosclerosis:** The progressive narrowing and hardening of a blood vessel over time. Atherosclerosis is a common form of arteriosclerosis in which deposits of yellowish plaques (atheromas) containing cholesterol, lipoid material, and lipophages are formed within the intima and inner media of large and medium-sized arteries. Treatment of atherosclerosis includes reversing or slowing the progression of atherosclerosis, for example as measured by the development of an atherosclerotic lesion, a change in the presence of existing atherosclerotic lesions, and/or functional signs of the disease, such as improvement in cardiovascular function as measured by signs (such as peripheral capillary refill), symptoms (such as chest pain and intermittent claudication), or laboratory evidence (such as that obtained by EKG, angiography, or other imaging techniques).

**Biological sample:** Any solid or fluid sample obtained from, excreted by or secreted by any living organism, including without limitation, multicellular organisms (such as animals, including samples from a healthy or apparently healthy human subject, or a human patient affected by a condition or disease to be diagnosed or investigated, such as an age-associated vascular disorder, for example atherosclerosis).

A biological sample can be a biological fluid obtained from, for example, blood, plasma, serum, urine, bile, ascites, saliva, cerebrospinal fluid, aqueous or vitreous humor, or any bodily secretion, a transudate, an exudate. A biological sample can also be a sample obtained from any organ or tissue (including a biopsy or autopsy specimen, such as a vascular biopsy) or can comprise a cell (whether a primary cell or cultured cell) or medium conditioned by any cell, tissue or organ. A biological sample can also be a sample that has been chemically treated, for example a sample which is fixed (such as fixed in formalin or any other chemical fixative known in the art) and/or paraffin embedded.

**Blood vessel:** The vessels through which blood circulates. In general, blood vessels are elastic tubular channels that are lined with endothelium. Blood vessels include the arteries, veins, and capillaries. Specific, non-limiting examples of a blood vessel include a vena cava, a thoracic aorta, a saphanous vein, a mammary artery, the brachial artery, and a capillary. In another embodiment, a blood vessel includes the smaller arteries and veins. In yet another embodiment, a blood vessel is a capillary of the microvascular circulation.

**Cardiovascular:** Pertaining to the heart and/or blood vessels.

**Cerebral ischemia or ischemic stroke:** A condition that occurs when an artery to the brain is partially or completely blocked such that the oxygen demand of the tissue exceeds the oxygen supplied. Deprived of oxygen and other nutrients following an ischemic stroke, the brain suffers damage and cell death as a result of the stroke.

Ischemic stroke can be caused by several different kinds of diseases. The most common problem is narrowing of the arteries in the neck or head. This is most often caused by atherosclerosis, or gradual cholesterol deposition. If the arteries become too narrow, blood cells may collect in them and form blood clots (thrombi). These blood clots can block the artery where they are formed (thrombosis), or can dislodge and become trapped in arteries closer to the brain (embolism). Also cerebral stroke can occur when atherosclerotic plaque separates away partially from the vessel wall and occludes the flow of blood through the blood vessel.

Another cause of stroke is blood clots in the heart, which can occur as a result of irregular heartbeat (for example, atrial fibrillation), heart attack, prior mural clot formation within a heart chamber and abnormalities of the heart valves such as occurs from rheumatic fever. While these are the most common causes of ischemic stroke, there are many other possible causes. Examples include use of street drugs, traumatic injury to the blood vessels of the neck, or disorders of blood clotting.

Ischemic stroke is by far the most common kind of stroke, accounting for about 80% of all strokes. Stroke can affect people of all ages, including children. Many people with ischemic strokes are older (60 or more years old, or 65 years or older), and the risk of stroke increases with older ages. At each age, stroke is more common in men than women, and it is more common among African-Americans than white Americans. Many people with stroke have other problems or conditions which put them at higher risk for stroke, such as high blood pressure (hypertension), heart disease, smoking, or diabetes.

**Chromatography:** Any of various techniques for the qualitative or quantitative separation of the components of mixtures of compounds, all of which are characterized by the use of a mobile phase (gas or liquid) moving relative to a stationary phase (liquid or solid). The differences between the rates of migration of the compounds between the two phases affect the separation. When a mixture a passed through a stationary phase, a molecule or molecules of interest (such as fragment peptides) can be separated from other molecules in the mixture and isolated. Examples of methods of chromatographic separation include gas-chromatography, liquid chromatography, capillary-action chromatography such as paper chromatography, thin layer chromatography (TLC), column chromatography, fast protein liquid chromatography (FPLC), high performance liquid chromatography (HPLC), nano flow HPLC, chromatography based on separation of the physical properties of proteins including hydrophobicity (reversed phase), charge (ion exchange), size/mass (size exclusion or gel filtration chromatography), biospecificity (affinity chromatography), amongst others. Chromatography also encompasses electrophoretic separation, as well as matrix- or chip-based formats.

**Contacting:** Placement in direct physical association, including both in a solid and a liquid form. For example, placing a liquid in direct physical association with another liquid, a liquid in direct physical association with a solid and a solid in direct physical association with another solid.

**Coronary Artery Disease:** In coronary artery disease, the coronary arteries become narrowed (stenosed) or blocked (occluded) by a gradual build-up of fat (cholesterol) within or on the artery wall, which reduces blood flow to the heart muscle. This build-up is called atherosclerotic plaque or simply plaque. Narrowing of the coronary arteries can also occur as a result of the development of undesirable blood clots (thrombi) in the arteries.

If plaque narrows the lumen or channel of the artery, it may make it difficult for adequate quantities of blood to flow to the heart muscle. If the build-up reduces flow only mildly, there may be no noticeable symptoms at rest, but symptoms such as chest pressure may occur with increased activity or stress. Other symptoms include heartburn, nausea, vomiting, shortness of breath and heavy sweating.

When flow is significantly reduced and the heart muscle does not receive enough blood flow to meet its needs (cardiac ischemia), severe symptoms such as chest pain (angina pectoris), heart attack (myocardial infarction), or rhythm disturbances (arrhythmias) may occur. A heart attack usually is the result of a completely blocked artery, which may damage the heart muscle.

There are three conventional ways to treat coronary artery disease: medication, surgery, and minimally invasive interventional procedures such as stent implantation, percutaneous transluminal coronary angioplasty (PTCA), intravascular radiotherapy, atherectomy and excimer (or exciplex) laser. The purpose of these treatments is to eliminate or reduce narrowing of the coronary blood vessels and hence eliminate or reduce symptoms, and in the case of coronary artery disease, decrease the risk of heart attack.

**Corresponding:** The term "corresponding" is a relative term indicating similarity in position, purpose or structure. In one embodiment, a peptide standard "corresponding" to a specific protein amino acid sequence (such as a protein gene product of a gene listed in Table 1) has an amino acid sequence identical to a portion of the protein (regardless of whether or not they have the same mass). Such a "peptide standard" can be used to quantitate the amount of a fragment peptide of identical sequence, such as a peptide fragment of any of the protein gene products of a gene listed in Table 1. In other embodiments, mass spectral signals in a mass spectrum that are due to corresponding peptides of identical structure but differing masses are "corresponding" mass spectral signals. A mass spectral signal due to a particular peptide is also referred to as a signal corresponding to the peptide. In other examples, a gene product of a gene listed in Table 1 corresponds to the protein or nucleic acid molecule expressed from the gene.

**DNA (deoxyribonucleic acid):** A long chain polymer which includes the genetic material of most living organisms. The repeating units in DNA polymers are four different nucleotides, each of which includes one of the four bases, adenine, guanine, cytosine and thymine bound to a deoxyribose sugar to which a phosphate group is attached. Triplets of nucleotides, referred to as codons, in DNA molecules code for amino acid in a polypeptide. The term codon is also used for the corresponding (and complementary) sequences of three nucleotides in the mRNA into which the DNA sequence is transcribed.

**Detecting expression of a gene product:** Detection of a level of expression in either a qualitative or quantitative manner, for example by detecting nucleic acid (such as mRNA) or protein. In some examples, it is the detection of a gene product of a gene listed in Table 1.

**Diagnosis:** The process of identifying a disease by its signs, symptoms and/or results of various tests. The conclusion reached through that process is also called a "diagnosis." In some examples, a subject is diagnosed with an age-associated vascular disorder.

**Differential expression or altered expression (quantitative differences):** A difference, such as an increase or decrease, in the amount of a gene product, such as messenger RNA, protein or both. In some examples, the difference is relative to a control or reference value, such as an amount of gene expression in tissue not affected by a disease, such as from sample isolated from a different subject who does not have an age-associated vascular disorder. Detecting differential expression can include measuring a change in gene or protein expression, such as a change in expression of one or more genes, such as the genes listed in Table 1.

**Expression:** The process by which the coded information of a gene is converted into an operational, non-operational, or structural part of a cell, for example to a gene product, such as an mRNA or protein. Gene expression can be influenced by external signals. For instance, exposure of a cell to a hormone may stimulate expression of a hormone-induced gene. Different types of cells can respond differently to an identical signal. Expression of a gene also can be regulated anywhere in the pathway from DNA to RNA to protein. Regulation can include modifying transcription, translation, RNA transport and processing, degradation of intermediary molecules such as mRNA, or through activation, inactivation, compartmentalization or degradation of specific protein molecules after they are produced.

The expression of a nucleic acid molecule can be altered relative to a normal (wild type) nucleic acid molecule, or the level of the nucleic acid in a subject responding to a treatment. The term "alterations in gene expression" (e.g., differential expression) includes but is not limited to: (1) over-expression; (2) under-expression; or (3) suppression of expression. Alterations in the expression of a nucleic acid molecule can be associated with, and in fact cause, a change in expression of the corresponding protein.

**Fragment peptide:** A peptide generated by cleavage of a protein or polypeptide with a protein cleavage agent, for example in a protein digest, or other fragmentation, such as fragmentation in a mass spectrometer. Fragment peptides include peptides produced by treatment of a protein with one or more endoproteases such as trypsin, chymotrypsin, endoprotease argC, endoprotease aspN, endoprotease gluC, and endoprotease lysC, as well as peptides produced by cleavage using chemical agents, such as cyanogen bromide, formic acid, and thiotrifluoroacetic acid. This can also be an endogenous cleavage that occurs in the tissue or body fluid that gives rise to a shorter fragment than the protein initially synthesized *in vivo.* Thus, fragments can be endogenous or exogenously created. In some examples, the endogenous protein fragments are differential markers of disease state. Thus, specific endogenous fragments could be diagnostic. The endogenous fragments can be diagnostic. One or more cleavage peptides (which can be referred to as fragment peptides) from a particular protein can be mass identifiers for the protein. In another example, a fragment peptide is generated during mass spectrometry (MS) analysis in the mass spectrometer. Representative fragment peptides include fragment peptides of the protein gene products of the genes listed in Table 1. In specific examples, a fragment peptide is a fragment peptide of MFG-E8, such as any one of SEQ ID NOs: 1, 2 and 9-12.

**Gene expression profile (or fingerprint):** Differential or altered gene expression can be detected by changes in the detectable amount of gene expression (such as cDNA or mRNA) or by changes in the detectable amount of proteins expressed by those genes. A gene expression profile can be a distinct or identifiable pattern of gene expression, for instance a pattern of high and low expression of a defined set of genes. In some examples, as few as one or two genes provides a profile, but more genes can be used in a profile, for example at least 5, at least 10, at least 15, at least 20, at least 30, at least 40, or even all of the genes listed in Table 1. A gene expression profile (also referred to as a fingerprint) can be linked to a tissue (including blood or blood fractions) or cell type, or to a distinct or identifiable condition that influences gene expression in a predictable way (such as disease or aging). Gene expression profiles can include relative, as well as absolute, expression levels of specific genes, and can be viewed in the context of a test sample compared to a baseline or control sample profile (such as a sample from a subject who does not have an age-associated vascular condition). In one example, a gene expression profile in a subject is read on an array (such as a nucleic acid array described below). In some examples, a gene expression profile is determined from a MS analysis of a biological sample obtained from a subject. In some examples, a gene expression profile can be used to diagnose or prognose an age-associated vascular disorder in a subject.

**Hybridization:** To form base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the Na⁺ concentration) of the hybridization buffer will determine the stringency of hybridization. Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11).

In particular examples, probes or primers can hybridize to one or more molecules (such as mRNA or cDNA molecules), for example under very high or high stringency conditions.

The following is an exemplary set of hybridization conditions and is not limiting:

| Verv High Stringency (detects sequences that share at least 90% identity) | |
|---|---|
| Hybridization: | 5x SSC at 65°C for 16 hours |
| Wash twice: | 2x SSC at room temperature (RT) for 15 minutes each |
| Wash twice: | 0.5x SSC at 65°C for 20 minutes each |
| | |

| High Stringency (detects sequences that share at least 80% identity) | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |
| | |

| Low Stringency (detects sequences that share at least 50% identity) | |
|---|---|
| Hybridization: | 6x SSC at RT to 55°C for 16-20 hours |
| Wash at least twice: | 2x-3x SSC at RT to 55°C for 20-30 minutes each. |

**Isolated:** An "isolated" biological component (such as a nucleic acid, peptide or protein, cell, tissue or blood fraction) has been substantially separated, produced apart from, or purified away from other biological components of the organism in which the component naturally occurs, that is, other chromosomal and extrachromosomal DNA and RNA, and proteins or other tissues. Nucleic acids, peptides and proteins which have been "isolated" thus include nucleic acids and proteins purified by standard or non-standard purification methods. The term also embraces nucleic acids, peptides and proteins prepared by recombinant expression in a host cell as well as chemically synthesized peptides and nucleic acids. In some examples, proteins, peptides and fragments thereof are isolated from a biological sample, for example prior to analysis, such as MS analysis. **Isotopic analog:** A molecule that differs from another molecule in the relative isotopic abundance of an atom it contains. For example, peptide sequences containing identical sequences of amino acids, but differing in the isotopic abundance of an atom, are isotopic analogs of each other. The term "isotopic analog" is a relative term that does necessarily not imply that the isotopic analog necessarily contains an isotope that is present in less or greater abundance in nature. For example, a mass identifier containing a natural abundance of ¹²C and ¹³C or N¹³ and N¹⁵ is an isotopic analog of a corresponding mass identifier having non-natural abundances of these isotopes, and vice versa.

**Isotopically-labeled:** A molecule that includes one or more stable heavy isotopes in a greater-than-natural abundance. Heavy stable isotopes include, for example ²H, ¹³C, ¹⁵N, ³⁴S, ¹⁷O, and ¹⁸O. Other labeling reagents can be used as long as there is a mass or sensor difference from the endogenous protein or peptide.

**Label:** An agent capable of detection, for example by ELISA, spectrophotometry, flow cytometry, or microscopy. For example, a label can be attached to a nucleic acid molecule or protein, thereby permitting detection of the nucleic acid molecule or protein. For example, a nucleic acid molecule, peptide or an antibody that specifically binds to a molecule can include a label. Examples of labels include, but are not limited to, radioactive isotopes, heavy stable isotopes, enzyme substrates, co-factors, ligands, chemiluminescent agents, fluorophores, haptens, enzymes, and combinations thereof. Methods for labeling and guidance in the choice of labels appropriate for various purposes are discussed for example in Sambrook et al. (Molecular Cloning: A Laboratory Manual, Cold Spring Harbor, New York, 1989) and Ausubel et al. (In Current Protocols in Molecular Biology, John Wiley & Sons, New York, 1998).

**Mass spectrometry:** A method wherein a sample is analyzed by generating gas phase ions from the sample, which are then separated according to their mass-to-charge ratio (m/z) and detected. Methods of generating gas phase ions from a sample include electrospray ionization (ESI), matrix-assisted laser desorption-ionization (MALDI), surface-enhanced laser desorption-ionization (SELDI), chemical ionization, and electron-impact ionization (EI). Separation of ions according to their m/z ratio can be accomplished with any type of mass analyzer, including quadrupole mass analyzers (Q), time-of-flight (TOF) mass analyzers, magnetic sector mass analyzers, 3D and linear ion traps (IT), Fourier-transform ion cyclotron resonance (FT-ICR) analyzers, and combinations thereof (for example, a quadrupole-time-of-flight analyzer, or Q-TOF analyzer), or Orbtiratp (LTQ-Orbitrap) or a triple quadropole LC/MS/MS such as the Q-trap MS instrument. Prior to separation, the sample (protein or resulting peptides) may be subjected to one or more dimensions of chromatographic separation, for example, one or more dimensions of liquid chromatography (based on hydrophobicity or mass/size), gel-electrophoretic, biospecificity (antibody) or specific post-translational modifications (for example, glycosylation or phosphorylation) separation.

**Multiple reaction monitoring (MRM):** A mass spectrometry based method in which absolute quantification of a targeted protein(s) can be obtained. In this method external or internal standards are used. Often a known quantity of a synthetic stable isotopically labeled peptide matching each of the targeted peptides that represent unique the protein is added into each sample being quantified. Comparison of the peak of the endogenous peptide to the labeled standard peptide allows absolute quantitation. MRM can be multiplexed easily, allowing multiple phosphorylation sites and/or multiple proteins to be assessed simultaneously.

**Milk fat globule epidermal growth factor-VIII (MFG-E8):** A secreted glycoprotein. Generally, MFG-E8 includes a signal sequence, two epidermal growth factor (EGF) domains, a proline/threonine (PT)-rich domain, and two factor VIII-homologous domains (C1 and C2). The second EGF domain contains an RGD (arginine-glycine-aspartate) motif that can be recognized by some members of the integrin family. MFG-E8 includes polypeptides encoded by nucleic acid sequences having single nucleotide polymorphisms (SNPs), or polypeptides having amino acid polymorphisms or co- and post-translational modifications.

Human MFG-E8 is 387 amino acids in length (386 amino acids in length after removal of the initiating methionine) and is roughly ∼43,000 Da. An exemplary amino acid sequence of human MFG-E8 is available at GENBANK® at accession no. NP_005919 as of January 19,2009; SEQ ID NO: 5). Asparagine residues at positions 238, 325, 329, and 350 of SEQ ID NO: 5 are potential N-linked glycosylation sites; residues 1-24 comprise a signal sequence. MFG-E8 or lactadherin 24-387 (amino acid residues 24-387 of SEQ ID NO: 5) is produced by removal of the signal sequence and is also known as a secreted or bioavailable form of MFG-E8. MFG-E8 can also be processed into two shorter forms, the lactadherin short form (amino acid residues 202-387 of SEQ ID NO: 5) and medin (amino acid residues 268-317 of SEQ ID NO: 5).

An exemplary nucleic acid sequence of human MFG-E8 is available at GENBANK® at accession no. NM_005928 (as of January 19, 2009; SEQ ID NO: 6).

An exemplary amino acid sequence of rat MFG-E8 is available at GENBANK® at accession no. NP_001035276 (as of January 19, 2009; SEQ ID NO: 7).

An exemplary nucleic acid sequence of rat MFG-E8 is available at GENBANK® at accession no. NM_001040186 (as of January 19, 2009; SEQ ID NO: 8).

**Nucleic acid array:** An arrangement of nucleic acids (such as DNA or RNA) in assigned locations on a matrix, such as that found in cDNA arrays, or oligonucleotide arrays, sometimes referred to as an array.

**Nucleotide:** Includes, but is not limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. A nucleotide sequence refers to the sequence of bases in a polynucleotide. An "oligonucleotide" is a plurality of joined nucleotides joined by native phosphodiester bonds, between about 6 and about 300 nucleotides in length, for example about 6 to 300 contiguous nucleotides of a nucleic acid molecule encoding a protein of interest. An oligonucleotide analog refers to moieties that function similarly to oligonucleotides but have non-naturally occurring portions. For example, oligonucleotide analogs can contain non-naturally occurring portions, such as altered sugar moieties or inter-sugar linkages, such as a phosphorothioate oligodeoxynucleotide.

Particular oligonucleotides and oligonucleotide analogs can include linear sequences up to about 200 nucleotides in length, for example a sequence (such as DNA or RNA) that is at least 6 nucleotides, for example at least 8, at least 10, at least 15, at least 20, at least 21, at least 25, at least 30, at least 35, at least 40, at least 45, at least 50, at least 100 or even at least 200 nucleotides long, or from about 6 to about 50 nucleotides, for example about 10-25 nucleotides, such as 12, 15 or 20 nucleotides. In particular examples, an oligonucleotide includes these numbers of contiguous nucleotides encoding a portion of a protein of interest, such as any one of the proteins gene products of the genes listed in Table 1. Such an oligonucleotide can be used on a nucleic acid array, or as primers or probes to detect the presence of the nucleic acid molecule encoding the protein of interest.

**Peptide/Protein/Polypeptide:** All of these terms refer to a polymer of amino acids and/or amino acid analogs that are joined by peptide bonds or peptide bond mimetics, regardless of length or post-translational modification (such as glycosylation, methylation, ubiquitination, phosphorylation, or the like). "Post-translational modification" is the chemical modification of a polypeptide after its translation, for example by mono-ubiquitination, glycosylation, methylation, phosphorylation, or the like. In some examples, a polypeptide is a protein gene product of a gene listed in Table 1 or a fragment thereof, such as MFG-E8 or a fragment thereof.

**Predictable mass difference:** A difference in the molecular mass of two molecules or ions (such as two peptides, or peptide ions) that can be calculated from the molecular formulas and isotopic contents of the two molecules or ions. Although predictable mass differences exist between molecules or ions of differing molecular formulas, they also can exist between two molecules or ions that have the same molecular formula but include different isotopes of their constituent atoms. A predictable mass difference is present between two molecules or ions of the same formula when a known number of atoms of one or more type in one molecule or ion are replaced by lighter or heavier isotopes of those atoms in the other molecule or ion. For example, replacement of a ¹²C atom in a molecule with a ¹³C atom (or vice versa) provides a predictable mass difference of about 1 atomic mass unit (amu), replacement of a ¹⁴N atom with a ¹⁵N atom (or vice versa) provides a predictable mass difference of about 1 amu, and replacement of a ¹H atom with a ²H (or vice versa) provides a predictable mass difference of about 1 amu. Such differences between the masses of particular atoms in two different molecules or ions are summed over all of the atoms in the two molecules or ions to provide a predictable mass difference between the two molecules or ions. Thus, for example, if two molecules have the formula C₆H₆, where one molecule includes 6 ¹³C atoms and the other includes 6 ¹²C atoms, the predictable mass difference between the two molecules is about 6 amu (1 amu difference/carbon atom).

**Peripheral Vascular Disease (PVD):** A condition in which the arteries and/or veins that carry blood to and from the arms, legs, soft tissues and vital organs of the body, including the heart and brain, become narrowed or occluded. This interferes with the normal flow of blood, sometimes causing pain but often causing no readily detectable symptoms. With progression of PVD, significant loss of blood flow to tissue and organs can lead to tissue death, necrosis and organ death.

The most common cause of PVD is atherosclerosis, a gradual process in which cholesterol and scar tissue build up, forming plaques that occlude the blood vessels. In some cases, PVD may be caused by blood clots that lodge in the arteries and restrict blood flow.

PVD affects about one in 20 people over the age of 50, or 8 million people in the United States. More than half the people with PVD experience leg pain, numbness or other symptoms, but many people dismiss these signs as a normal part of aging and do not seek medical help.

The most common symptom of PVD is painful cramping in the leg or hip, particularly when walking. This symptom, also known as claudication, occurs when there is not enough blood flowing to the leg muscles during exercise, such that ischemia occurs. The pain typically goes away when the muscles are rested.

Other symptoms may include numbness, tingling or weakness in the leg. In severe cases, people with PVD may experience a burning or aching pain in an extremity such as the foot or toes while resting, or may develop a sore on the leg or foot that does not heal. People with PVD also may experience a cooling or color change in the skin of the legs or feet, or loss of hair on the legs. In extreme cases, untreated PVD can lead to gangrene, a serious condition that may require amputation of a leg, foot or toes. People with PVD are also at higher risk for heart disease and stroke.

Typically most symptomatic PVD is ascribed to peripheral artery disease (PAD) denoting the above described pathology predominantly in arteries. The term PVD includes this symptomology and pathology in all classes of blood vessels.

**Post-translational modifications (qualitative differences):** Proteins can be altered by a chemical modification after translation. Examples of post-translational modifications include addition of functional groups (such as acetate, phosphate, lipids, or carbohydrates), removal of a portion of a protein (such as a signal sequence or the initial methionine residue) or formation of alternatively spliced variants, and formation of a disulfide bond. Proteins can also be modified by co-translational modifications. The modifications can be disease specific and differences in the extent of the modification can be diagnostic.

**Primers:** Short nucleic acid molecules, for instance DNA oligonucleotides 10-100 nucleotides in length, such as about 15, 20, 25, 30 or 50 nucleotides or more in length, such as this number of contiguous nucleotides of a nucleotide sequence encoding a protein of interest or other nucleic acid molecule. Primers can be annealed to a complementary target DNA strand by nucleic acid hybridization to form a hybrid between the primer and the target DNA strand. Primer pairs can be used for amplification of a nucleic acid sequence, such as by PCR or other nucleic acid amplification methods known in the art.

Methods for preparing and using nucleic acid primers are described, for example, in Sarnbrook et al. (In Molecular- Cloning: A Laboratory Manual, CSHL, New York, 1989), Ausubel et al. (In Current Protocols in Molecular Biology, John Wiley & Sons, New York, 1998), and Innis et al. (PCR Protocols, A Guicle to Methods anal Applications, Academic Press, Inc., San Diego, CA, 1990). PCR primer pairs can be derived from a known sequence, for example, by using computer programs intended for that purpose such as Primer (Version 0.5, © 1991, Whitehead Institute for Biomedical Research, Cambridge, MA). One of ordinary skill in the art will appreciate that the specificity of a particular primer increases with its length.

In one example, a primer includes at least 15 consecutive nucleotides of a nucleotide molecule, such as at least 18 consecutive nucleotides, at least 20, at least 25, at least 30, at least 35, at least 40, at least 45, at least 50 or more consecutive nucleotides of a nucleotide sequence (such as a gene, mRNA or cDNA). Such primers can be used to amplify a nucleotide sequence of interest encoding a protein, such as the proteins listed in Table 1, for example using PCR.

**Probe:** A short sequence of nucleotides, such as at least 8, at least 10, at least 15, at least 20, at least 21, at least 25, at least 30, at least 35, at least 40, at least 45, at least 50, at least 55, at least 60, at least 65, at least 70, at least 75, at least 80, at least 85, at least 90, at least 95 or even greater than 100 nucleotides in length, used to detect the presence of a complementary sequence by molecular hybridization. In particular examples, oligonucleotide probes include a label that permits detection of oligonucleotide probe:target sequence hybridization complexes. Such an oligonucleotide probe can also be used on a nucleic acid array, for example to detect a nucleic acid molecule in a biological sample contacted to the array. In some examples, a probe is used to detect the presence of a nucleic acid molecule gene product of a gene listed in Table 1.

**Prognosis:** The probable course or outcome of a disease process. By way of example, the prognosis of a subject can indicate the likelihood of a subject to have or develop an age-related vascular disorder, such as atherosclerosis.

**Protein cleavage agent:** Includes both enzyme and chemical agents capable of cleaving peptide bonds in a protein or peptide. Examples of proteolytic enzymes include endoproteases such as trypsin, chymotrypsin, endoprotease argC, endoprotease aspN, endoprotease gluC, and endoprotease lysC. Examples of chemical protein cleavage agents include cyanogen bromide, formic acid, and thiotrifluoroacetic acid. The specific bonds cleaved by an endoprotease or a chemical protein cleavage agents may be more specifically referred to as "endoprotease cleavage sites" and "chemical protein cleavage agent sites," respectively. Proteins typically contain one or more intrinsic protein cleavage agent sites that are recognized by one or more protein cleavage agents by virtue of the amino acid sequence of the protein.

**Purified:** The term "purified" does not require absolute purity; rather, it is intended as a relative term. Thus, for example, a purified protein preparation is one in which the protein referred to is more pure than the protein in its natural environment within a cell. For example, a preparation of a protein is purified such that the protein represents at least 50% of the total protein content of the preparation. Similarly, a purified oligonucleotide preparation is one in which the oligonucleotide is more pure than in an environment including a complex mixture of oligonucleotides.

**Quantitative real-time PCR (or Q-RT-PCR):** A method for determining the level of specific DNA or RNA molecules in a biological sample, for example a DNA or RNA gene product of a gene listed in Table 1. The accumulation of PCR product is measured at each cycle of a PCR reaction and is compared with a standard curve or quantitated relative to a control DNA or RNA. Quantitative real-time PCR is based on the use of fluorescent dyes or probes to measure the accumulation of PCR product. This may be accomplished through a TAQMAN® assay, where a fluorescently labeled probe is displaced during DNA synthesis by Taq polymerase, resulting in fluorescence, or by inclusion in the PCR reaction of a fluorescent dye such as SYBR® Green, which binds non-specifically to the accumulating doublestranded DNA.

If a standard curve is used to quantitate DNA or RNA, a series of samples containing known amounts of DNA or RNA are run simultaneously with unknown samples. The resulting fluorescence measured from the unknowns may be compared with that from the known samples in order to calculate the quantity of DNA or RNA in the sample. One application of this method is to quantify the expression of an mRNA in one or more samples from subjects.

Quantitative real-time PCR may also be used to determine the relative quantity of a specified RNA present in a sample in comparison to a control sample when knowing the absolute copy number is not necessary. One application of this method is to determine the number of copies of an mRNA in a sample from a subject. The PCR product generated is assessed to determine how many PCR cycles are required for the PCR product to be detectable.

**Reference:** A standard against which an experiment or treatment can be measured or evaluated against. In some examples, a reference is a reference level of expression of a gene product of a gene of interest, such as a gene listed in Table 1. A reference can be a standard value or the amount of a substance, such as a specific protein or mRNA in a reference sample, such as the amount expressed in a sample taken from a healthy and/or young subject. A "statistical reference" is a standard based on the statistical analysis among a population. In some examples, a statistical reference is the expression of a gene listed in Table 1 in a population of subjects that does not have an age-related vascular disorder. In some examples, the population from which a statistical reference is calculated is a population of young subjects, for example human subject between the ages of about 18-40, such as about 18-35, about 18-30, about 18-25, about 25-40, about 30-40, or about 20-35. A difference between a sample being tested and a reference can be an increase or conversely a decrease. The difference can be a qualitative difference, for example, but not limited to, a difference in color, shape, or location, or a quantitative difference, for example a statistically significant difference. In some examples, a quantitative difference is a decrease, relative to a control, of at least about 10%, such as at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 500%, or greater than 500%. In other examples, a quantitative difference is an increase, relative to a control, of at least about 10%, such as at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 500%, or greater than 500%.

**Sequence identity/similarity:** The identity/similarity between two or more nucleic acid sequences, or two or more amino acid sequences, is expressed in terms of the identity or similarity between the sequences. Sequence identity can be measured in terms of percentage identity; the higher the percentage, the more identical the sequences are. Sequence similarity can be measured in terms of percentage similarity (which takes into account conservative amino acid substitutions); the higher the percentage, the more similar the sequences are. Homologs or orthologs of nucleic acid or amino acid sequences possess a relatively high degree of sequence identity/similarity when aligned using standard methods. This homology is more significant when the orthologous proteins or cDNAs are derived from species which are more closely related (such as human and mouse sequences), compared to species more distantly related (such as human and *C*. *elegans* sequences).

Methods of alignment of sequences for comparison are well known in the art. Various programs and alignment algorithms are described in: Smith & Waterman, Adv. Appl. Math. 2:482-489, 1981; Needleman & Wunsch, J. Mol. Biol. 48:443-453, 1970; Pearson & Lipman, Proc. Natl. Acad. Sci. USA 85:2444-2448, 1988; Higgins & Sharp, Gene, 73:237-244, 1988; Higgins & Sharp, CABIOS 5:151-153, 1989; Corpet et al., Nuc. Acids Res. 16:10881-10890, 1988; Huang et al. Computer Appls. in the Biosciences 8, 155-165, 1992; and Pearson et al., Meth. Mol. Bio. 24:307-331, 1994. Altschul et al., J. Mol. Biol. 215:403-410, 1990, presents a detailed consideration of sequence alignment methods and homology calculations.

The NCBI Basic Local Alignment Search Tool (BLAST) (Altschul et al., J. Mol. Biol. 215:403-10, 1990) is available from several sources, including the National Center for Biological Information (NCBI, National Library of Medicine, Building 38A, Room 8N805, Bethesda, MD 20894) and on the Internet, for use in connection with the sequence analysis programs blastp, blastn, blastx, tblastn and tblastx. Additional information can be found at the NCBI web site.

BLASTN is used to compare nucleic acid sequences, while BLASTP is used to compare amino acid sequences. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. For example, a target sequence containing a 20-nucleotide region that aligns with 20 consecutive nucleotides from an identified sequence as follows contains a region that shares 75 percent sequence identity to that identified sequence (that is, 15÷20*100=75). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer. In another example, For comparisons of amino acid sequences of greater than about 30 amino acids, the Blast 2 sequences function is employed using the default BLOSUM62 matrix set to default parameters, (gap existence cost of 11, and a per residue gap cost of 1). Homologs are typically characterized by possession of at least 70% sequence identity counted over the full-length alignment with an amino acid sequence using the NCBI Basic Blast 2.0, gapped blastp with databases such as the nr or swissprot database. Queries searched with the blastn program are filtered with DUST (Hancock and Armstrong, Comput. Appl. Biosci. 10:67-70, 1994). In addition, a manual alignment can be performed. Proteins with even greater similarity will show increasing percentage identities when assessed by this method, such as at least about 75%, 80%, 85%, 90%, 95%, 98%, or 99% sequence identity to a protein gene product of a gene listed in Table 1.

When aligning short peptides (fewer than around 30 amino acids), the alignment is be performed using the Blast 2 sequences function, employing the PAM30 matrix set to default parameters (open gap 9, extension gap 1 penalties). When less than the entire sequence is being compared for sequence identity, homologs will typically possess at least 75% sequence identity over short windows of 10-20 amino acids, and can possess sequence identities of at least 85%, 90%, 95% or 98% depending on their identity to the reference sequence. Methods for determining sequence identity over such short windows are described at the NCBI web site.

One indication that two nucleic acid molecules are closely related is that the two molecules hybridize to each other under stringent conditions, as described above. Nucleic acid sequences that do not show a high degree of identity may nevertheless encode identical or similar (conserved) amino acid sequences, due to the degeneracy of the genetic code. Changes in a nucleic acid sequence can be made using this degeneracy to produce multiple nucleic acid molecules that all encode substantially the same protein. Such homologous nucleic acid sequences can, for example, possess at least about 60%, 70%, 80%, 90%, 95%, 98%, or 99% sequence identity to a nucleic acid of a gene listed in Table 1 is determined by this method. An alternative (and not necessarily cumulative) indication that two nucleic acid sequences are substantially identical is that the polypeptide which the first nucleic acid encodes is immunologically cross reactive with the polypeptide encoded by the second nucleic acid.

One of skill in the art will appreciate that the particular sequence identity ranges are provided for guidance only; it is possible that strongly significant homologs could be obtained that fall outside the ranges provided.

**Standard:** A substance or solution of a substance of known amount, purity or concentration. A standard can be compared (such as by spectrometric, chromatographic, or spectrophotometric analysis) to an unknown sample (of the same or similar substance) to determine the presence of the substance in the sample and/or determine the amount, purity or concentration of the unknown sample. In one embodiment a standard is a peptide standard. An internal standard is a compound that is added in a known amount to a sample prior to sample preparation and/or analysis and serves as a reference for calculating the concentrations of the components of the sample. Isotopically-labeled peptides are particularly useful as internal standards for peptide analysis since the chemical properties of the labeled peptide standards are almost identical to their non-labeled counterparts. Thus, during chemical sample preparation steps (such as chromatography, for example, HPLC) any loss of the non-labeled peptides is reflected in a similar loss of the labeled peptides. Alternatively, one could use external standards, for example in mass spectrometry analysis as long as the instrument reproducibility between runs was sufficient to ensure consistency between standard analysis.

**Vascular function assessment:** An assay that measures the function of the vascular system. Assays include measurement of a parameter of the blood, assays of arterial hyperplasia, vascular contractility measurements, brachial reactivity measurements, and morphometric measurements. Similarly, an endothelial cell assessment is a test that measures a function or parameter of an endothelial cell. "Decreased vascular function" indicates a decrease in any function of the blood vessels, as compared to a standard value or a control sample. Thus, in one example, decreased vascular function is a decrease in a vascular contractility, as compared to a known value for normal vascular contractility. In another example, decreased vascular function is the lower contractility of a blood vessel as compared to the contractility of a vessel known to not be affected by as a disease or a disorder. In a further example, decreased vascular function is a lower vascular contractility as compared to the contractility of a vessel from the same subject at an earlier time point.

Decreased vascular function is associated with atherosclerosis, myocardial infarction, intermittent claudication, bowel ischemia, retinal ischemia, transient ischemic attacks (TIAs), ischemic strokes, restenosis after angioplasty, transplant atherosclerosis, unstable angina, sudden death, and alterations in blood pressure.

**Vascular tissue:** Tissue consisting of, or containing, blood vessels as an essential part of a structure. Vascular tissue operates by means of, or is made up of an arrangement of, vessels. Vascular tissue includes the arteries, veins, capillaries, lacteals, microvasculature, etc. In one embodiment, vascular tissue includes a highly vascularized organ (for example, the lung). In another embodiment, vascular tissue is a blood vessel, or a portion thereof. Cells isolated from a vascular tissue are a population of cells isolated from the remaining components of the tissue, for example VSMCs.

**Vasculopathy:** A disease of the blood vessels. An "age-related vasculopathy: is a disease of the blood vessels that is associated with advanced age. One specific, non-limiting vasculopathy is atherosclerosis. Other vasculopathies include, but are not limited to, diabetic associated vasculopathy, hypertension associated vasculopathy, Burger's disease associated vasculopathy and scleroderma associated vasculopathy.

### III. Overview of Several Embodiments

Disclosed are methods for diagnosing or prognosing an age-associated vascular disorder in a subject. The methods include obtaining a biological sample from the subject and detecting altered expression a gene product of MFG-E8 or at least one gene listed in Table 1 (or a combination thereof) in the biological sample relative to a reference level, thereby diagnosing or prognosing an age-associated vascular disorder in the subject. In some examples, the reference level is the level of expression of the gene product in a biological sample obtained from the same subject at an earlier time point. In other examples, the reference level is the level of expression of the gene product in a biological sample obtained from another subject that does not have the age-associated vascular disorder. In other examples, the reference level is the level of expression of the gene product in a biological sample obtained from a younger individual. In still other examples, the reference level is a statistical reference value indicative of expression in a subject that does not have the age-associated vascular disorder.

In some embodiments of the disclosed method, the gene product of MFG-E8 or of the at least one other gene listed in Table 1 is a protein and detection of altered expression of the gene product includes detecting the MFG-E8 protein and/or other protein listed in Table 1. In particular embodiments, detecting a protein gene product of MFG-E8 and/or at least one other gene listed in Table 1 further comprises enrichment of the MFG-E8 protein or other protein listed in Table 1 in the biological sample. In some embodiments detecting a protein gene product of a gene listed in Table 1 includes producing a protein digest from the biological sample obtained from the subject and detecting a fragment of the protein in the protein digest (for example a serine protease digest, such as a trypsin digest), thereby detecting the protein in the biological sample. In some embodiments, the protein digest is fractionated prior to detecting the fragment of the protein listed in Table 1 in the protein digest, for example fractionated by chromatography, such as by one or more of gel electrophoresis, liquid chromatography, capillary electrophoresis, nano-reversed phase liquid chromatography, high performance liquid chromatography, phosphorylation enrichment affinity chromatography, glycosylation enrichment affinity chromatography, and protein affinity chromatography.

In some embodiments of the disclosed method, the protein gene product of MFG-E8 and/or at least one other gene listed in Table 1 or a peptide fragment thereof is detected using mass spectrometry, for example using tandem mass spectrometry. In other embodiments, detecting MFG-E8 or at least one other gene listed in Table 1 or a peptide fragment thereof involved contacting the MFG-E8 protein or other protein listed in Table 1 with an antibody that specifically binds the MFG-E8 protein or other protein listed in Table 1. In some examples, co- and post-translation modified forms of the protein, peptides, or fragments thereof are detected, for example, SNP and other polymorphisms, protein isoforms arising from different genes or the same genes and different splice variants. In some examples of the disclosed methods the protein or a fragment thereof is quantitated, for example by comparing an amount of the protein or fragment peptide thereof to a protein or peptide standard of known amount. In some embodiments, the peptide standard is an isotopically labeled peptide, such as a peptide isotopically labeled with ¹⁸O, ¹⁷O, ³⁴S, ¹⁵N, ¹³C, ²H or combinations thereof.

In some embodiments of the disclosed method, the gene product of the gene listed in Table 1 is a nucleic acid and detecting altered expression of the gene product includes detecting the nucleic acid. In specific examples mRNA encoding the gene is detected, for example using a RT-PCR, such as quantitative RT-PCR, or mass spectrometry. In some embodiments, detecting the nucleic acid involves using a microarray.

In other embodiments, the altered expression level of MFG-E8 or one or more other gene listed in Table 1 includes an increase in the expression level of MFG-E8 or the one or more other gene(s) listed in Table 1. In particular embodiments, the increased expression level of MFG-E8 or the other gene(s) listed in Table 1 is an increase in protein or nucleic acid level. In further embodiments, detecting the altered expression level includes detecting the expression level of MFG-E8 and at least one other gene listed in Table 1.

In some embodiments, the disclosed methods for diagnosing or prognosing an age-associated vascular disorder in a subject include obtaining a biological sample from the subject and detecting increased expression of MFG-E8 protein in the biological sample relative to a reference level. An increase in the expression of MFG-E8 diagnoses the subject as having or prognoses the subject as being at risk of having an age-associated vascular disorder. In some examples, the reference level is the level of expression of MFG-E8 protein in a biological sample obtained from the same subject at an earlier time point. In some examples, the reference level is the level of expression of MFG-E8 protein in a biological sample obtained from another subject that does not have the age-associated vascular disorder. In some examples, the reference level is the level of expression of MFG-E8 protein in a biological sample obtained from a younger individual. In some examples, the reference level is a statistical reference value indicative of expression in a subject that does not have the age-associated vascular disorder.

In some embodiments, the disclosed method includes producing a protein digest from the biological sample (for example a serine protease digest, such as a trypsin digest), optionally fractionating the protein digest (for example using chromatography), and detecting a fragment of MFG-E8 in the protein digest, thereby detecting MFG-E8 in the biological sample. In some non-limiting examples, the peptide fragment of MFG-E8 comprises the amino acid sequence of any one of SEQ ID NO: 1, SEQ ID NO: 2, SEQ ID NO: 9, SEQ ID NO: 10, SEQ ID NO: 11 or SEQ ID NO: 12. In some non-limiting examples, the peptide fragment of MFG-E8 consists of the amino acid sequence of any one of SEQ ID NO: 1, SEQ ID NO: 2, SEQ ID NO: 9, SEQ ID NO: 10, SEQ ID NO: 11 or SEQ ID NO: 12.

In some embodiments, the fragment of MFG-E8 is detected using mass spectrometry, such as tandem mass spectrometry.

In some embodiments, the MFG-E8 protein or a fragment thereof in the biological sample is quantitated. In some examples, quantitating the MFG-E8 includes comparing an amount of the MFG-E8 protein of fragment peptide thereof to a protein or peptide standard of known amount. In some examples, the peptide standard comprises a peptide having the amino acid sequence of any one of SEQ ID NO: 1, SEQ ID NO: 2, SEQ ID NO: 9, SEQ ID NO: 10, SEQ ID NO: 11 or SEQ ID NO: 12. In some examples, the peptide standard consists of a peptide having the amino acid sequence of any one of SEQ ID NO: 1, SEQ ID NO: 2, SEQ ID NO: 9, SEQ ID NO: 10, SEQ ID NO: 11 or SEQ ID NO: 12. In some examples, the peptide standard is isotopically labeled, for example isotopically labeled with one or more heavy stable isotopes selected from ¹⁸O, ¹⁷O, ³⁴S, ¹⁵N, ¹³C, ²H or combinations thereof.

In some embodiments of the disclosed methods, the biological sample comprises a blood sample, a urine sample, a serum sample, an ascites sample, a saliva sample, a cell, or a portion of a tissue sample. In specific examples, the biological sample comprises a serum sample or a plasma sample. In other examples, the tissue sample is obtained by biopsy. In some examples, the tissue sample includes vascular tissue, such as aortic tissue.

### IV. Methods of Diagnosing a Subject with an Age-Associated Vascular Disorder

It is disclosed herein that the expression of 50 genes (listed in Table 1) changes in vascular tissue as the vascular tissue ages. In other words, the expression of these 46 genes changes as a function of age of the vascular tissue. This discovery was made using comprehensive proteomic analysis with a combination of gel-based (2DE) and mass spectrometry (MS)-based (iTRAQ™ isotopic labeling). These 46 differentially expressed genes are markers for age-associated vascular disorders, such as such as hypertension, atherosclerosis, stroke, and arterial restenosis after angioplasty.

Methods are disclosed herein to determine if a subject has or may develop an age-associated vascular disorder. The subject may be young or aged. In some embodiments, the subject is young and exhibits premature aging of the vascular tissue. In other embodiments, the subject is young and is at risk for developing a vascular disorder that is similar to, or the same as, an age-associated vascular disorder. The disclosed methods can be used for an initial diagnosis, or to follow the progression of an age-related vascular disorder, for example as the subject ages or following treatment designed to ameliorate the age-related vascular disorder.

The methods diagnosing or prognosing an age-associated vascular disorder in a subject include obtaining a biological sample from the subject and detecting altered expression of at least one gene product of a gene listed in protein Table 1, for example a protein gene product or nucleic acid gene product of a gene listed in Table 1, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the genes listed in Table 1. The presence of altered expression of at least one gene product of a gene listed in Table 1 relative to a control level indicates that the subject has an age-associated vascular disorder or is a risk of developing an age-associated vascular disorder. In some examples gene product of a subset of the genes listed in Table 1 are detected. In some examples expression of one or more of the subset of genes listed in Table 1 that increase in expression in as a function of age is detected (such as one or more of MFGM, HTRA1, ITM2B, CSRP2, PLF4, IBP7, RBP1, ARPC4, CNN1, CNN3, TGFB3, CLUS, MMP2, PGS1, FINC, SPRL1, VTNC, KNT1, POSTN, APOE, TRI47, ADCY5, GTR4, or FRIL1). In some examples expression of one or more of the subset of genes listed in Table 1 that decrease in expression in as a function of age is detected (such as one or more of KCRM, GPDA, COX5B, GLNA, ODO1, DECR, THIM, HCDH, ECHA, ACSL1, IDHP, PYC, SLK, CO1A1, CO1A2, APOA1, MUP, TTHY, A1AT, TRI47, A1I3, CAH3, EST2, SPA3K, SPA3L, FETUA, or FETUB). In some examples, expression of the subset of genes that are classified in Table 1 as being involved in apoptosis/cell cycle/proliferation is detected (such as, HTRA1, ITM2B, CSRP2, IBP7,and/or PLF4). In some examples, expression of the subset of genes that are classified in Table 1 as being involved in cell metabolism is detected (such as, KCRM, GPDA, COX5B, and/or GLNA). In some examples, expression of the subset of genes that are classified in Table 1 as being involved in lipid metabolism/citric acid cycle is detected (such as, ODO1, IDHP and/or PYC). In some examples, expression of the subset of genes that are classified in Table 1 as being involved in lipid metabolism/fatty acid beta-oxidation is detected (such as, DECR, THIM, HCDH, ECHA, and/or ACSL1). In some examples, expression of the subset of genes that are classified in Table 1 as being involved in cytoskeleton/invasion/migration is detected (such as, RBP1, SLK, ARPC4, CNN1, and/or CNN3). In some examples, expression of the subset of genes that are classified in Table 1 as being involved in cytoskeleton/ invasion/migration/adhesion is detected (such as, TGFB3, CLUS, MMP2, PGS1, CO1A1, CO1A2, FINC, SPRL1, VTNC, APOA1, KNT1, POSTN, APOE, FETUA, FETUB, and/or MUP). In some examples, expression of the subset of genes that are classified in Table 1 as being involved in inflammation/oxidation is detected (such as, TRI47, A1I3, FRIL1, CAH3, EST2, SPA3K, and/or SPA3L). In some examples, the protein gene products are differentially post-translationally modified on samples taken from old versus young subjects, thus differences in post translational modification of a gene product listed in Table 1, such as one more of MFGM, CNN3, KNT1, POSTN, A1I3, are used to diagnose an age-related disorder.

In one example, increased expression of milk fat globule Epidermal growth factor-VIII's (MFG-E8; lactadherin) was conserved across mammalian species including rat aorta non-human primates and humans. This increased expression of MFG-E8 enables VSMC cell invasion and VSMC replicative capacity and is a component of vascular-remodeling that leads to age-related vascular disorders for example cardiovascular diseases, such as hypertension, atherosclerosis, and stroke, and arterial restenosis after angioplasty.

Exemplary methods of detecting and quantifying gene products, such as protein gene products and nucleic acid gene products are given below in sections A and B respectively. The presence and/or quantity of the gene product of the genes listed in Table 1 in can be determined in different types of biological samples, for example a solid biological sample obtained from a subject, such as a tissue sample, or a fluid sample obtained from a subject. In particular embodiments, a biological sample obtained from the subject is a blood sample, a urine sample, a serum sample, an ascites sample, a saliva sample, a cell, or portion of tissue, although any biological sample of interest can be used. Tissue samples, such as a portion of a tissue, can be obtained by a variety of invasive, minimally invasive, and/or non-invasive methods. In some examples, the biological sample is a blood fraction, such as serum. In some examples a biological sample obtained from a subject is vascular tissue, for example aortic tissue. It will appreciated that any method of obtaining tissue from a subject can be utilized, and that the selection of the method used will depend upon various factors such as the type of tissue, age of the subject, or procedures available to the practitioner. The tissue sample can be obtained by a variety of procedures including, but not limited to, surgical excision, aspiration, or biopsy.

References that can be used with the disclosed methods include the level of expression of a gene product listed in Table 1, in a biological sample obtained from the same subject at an earlier time point, in a biological sample obtained from another subject that does not have the age-associated vascular disorder, in a biological sample obtained from a younger individual, or a statistical reference value indicative of expression in a subject that does not have the age-associated vascular disorder.

The methods disclosed herein are particularly suited to monitoring the progression of an age-related vascular disorder in a subject. Monitoring an age-related vascular disorder in a subject can involve detecting the expression of one or more of the genes listed in Table 1 in a biological sample obtained from a subject at a first time point and comparing that expression to the expression of the same gene at a later time point. An alteration in gene expression at the second time point can indicate that the subject is showing signs of age-related vascular disorder progression, for example, an increase in the expression of MFG-E8 at the second time point would indicate that the subject is progressing, and conversely a decrease in the expression of MFG-E8 at the second time point would indicate that the subject is regressing, for example in response to a therapeutic treatment.

### A. Evaluation of Proteins

In some embodiments, detection of altered expression of a gene product of one or more genes listed in Table 1 includes detecting a protein encoded by one or more of the genes listed in Table 1 or a fragment or modified form (including all SNP, or amino acid polymorphisms and co- and post-translational modifications of the protein) thereof, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the genes listed in Table 1 in a biological sample can be evaluated using these methods.

### i. Proteins, Fragments, Modified Forms and Detection Thereof

Fragment peptides, such as fragments of a protein gene product of a gene listed in Table 1, for example MFG-E8 fragment peptides, can be obtained by proteolytic cleavage of proteins present in the biological sample with a protein cleavage agent, such as in a protein digest. The full-length protein or a modified form thereof can be in some instances also used. The fragment peptides are excised from the full-length protein. By way of example, a MGF-E8 fragment peptide is removed from the full-length protein such that it does not include the full-length MGF-E8 protein. Fragment peptides (such as fragments of the protein gene products of the genes listed in Table 1, for example MFG-E8 fragment peptides) include peptides produced by treatment of a protein with one or more endoproteases, such as trypsin, chymotrypsin, endoprotease argC, endoprotease aspN, endoprotease gluC, and endoprotease lysC, as well as peptides produced by chemical cleavage reactions, such as those that employ cyanogen bromide, formic acid, and thiotrifluoroacetic acid, as is well known to those of ordinary skill in the art. In some embodiments, the proteolytic cleavage agent is serine protease. In one embodiment, the proteolytic cleavage agent is trypsin, and the resulting digest is a trypsin digest. Endogenous proteases can cleavage *peptides in vivo*; the resultant peptides can also be used in the assay.

Fragment peptides of the protein gene products of the genes listed in Table 1, for example MFG-E8 fragment peptides, can be uniquely associated with the full-length protein sequence from which they are excised. Thus, these peptides can be used to determine the presence of the full-length protein in a biological sample, such as a biological sample obtained from a subject. Identification of the peptide sequence that is uniquely associated with the full-length polypeptide sequence in a sample identifies the full-length polypeptide sequence in the sample. In other words, a fragment peptide that is uniquely associated with a full-length protein is a mass identifier that contains enough sequence information to discriminate between the full-length protein, from which the mass identifier is derived, and other proteins in the sample.

Mass identifiers are peptides (or a set of peptides) having a particular sequence(s) that is (are) uniquely generated from a protein of interest (such as MFG-E8) by treatment with a particular protein cleavage agent. Detection of a mass identifier for a protein of interest in a sample unambiguously identifies the presence of the protein of interest in a sample treated with the protein cleavage agent. Determination of the concentration or amount of the mass identifier in a sample also determines the concentration or amount of the protein of interest in the sample either directly (by direct proportion) or after multiplying the concentration of the mass identifier by the number of such mass identifiers generated per protein of interest. Mass identifiers can be identified by treating proteins with a protein cleavage agent *in vivo, in vitro* or *in silico.* Various methods and algorithms for determining a mass identifier for a protein of interest are known, but all have in common that peptide sequences obtained by digestion (actual or theoretical) of a protein of interest with a protein cleavage agent (such as an endoprotease or a model of an endoprotease's cleavage specificity) are compared to peptide sequences obtained by digestion of other known proteins with the same cleavage agent to determine one or more peptide sequences that are uniquely produced from the protein of interest (such as MGF-E8).

The fragment peptides useful in the disclosed examples are from about 6 to about 45 amino acid residues in length, such as about 6 amino acids, about 7 amino acids, about 8 amino acids, about 9 amino acids, about 10 amino acids, about 11 amino acids, about 12 amino acids, about 13 amino acids, about 14 amino acids, about 15 amino acids, about 16 amino acids, about 17 amino acids, about 18 amino acids, about 19 amino acids, about 20 amino acids, about 21 amino acids, about 22 amino acids, about 25 amino acids, about 30 amino acids, about 35 amino acids, about 40 amino acids, or about 45 amino acids in length. In some embodiments, the fragment peptide is a MFG-E8 fragment peptide. In some embodiments, the MFG-E8 fragment peptide does not include an amino acid sequence corresponding to medin (amino acid residues 268-317 of SEQ ID NO: 5). In some embodiments, the MFG-E8 fragment peptide corresponds to an amino acid sequence in MFG-E8 and includes an amino acid sequence as set forth as SEQ ID NO: 1, SEQ ID NO: 2, SEQ ID NO: 9, SEQ ID NO: 10, SEQ ID NO: 11, or SEQ ID NO: 12. In some embodiments, the MFG-E8 fragment peptide consists of an amino acid sequence as set forth as one of SEQ ID NO: 1, SEQ ID NO: 2, SEQ ID NO: 9, SEQ ID NO: 10, SEQ ID NO: 11, or SEQ ID NO: 12.

Fragment peptides, such as peptide fragments of the protein gene products of the genes listed in Table 1, for example, MFG-E8 fragment peptides, can be detected by any method that allows for the detection and identification of peptides. Methods particularly suited for the detection and identification of peptides, such as MFG-E8 fragment peptides, are mass spectrometric methods. In certain embodiments, the fragment peptides of the protein gene products of the genes listed in Table 1, such as MGF-E8 fragment peptides, are detected with mass spectrometry. In certain embodiments, the fragment peptides of the protein gene products of the genes listed in Table 1, such as MGF-E8 fragment peptides, are detected with tandem mass spectrometry. It some embodiments, the fragment peptides of the protein gene products of the genes listed in Table 1, such as MGF-E8 fragment peptides are detected by detection of ion fragments generated from fragment peptides (for example, by collision using tandem mass spectrometry). Exemplary mass spectrometric methods that can be used in the disclosed methods are found below, although it is contemplated that any mass spectrometric technique that identifies peptides could be used.

Enzymatic digestion of complex mixtures of proteins followed by mass spectrometric based analysis of the digest is well known in the art (see for example, U.S. Patent No. 6,940,065 and Yates et al., J. Protein Chem., 16: 495-497, 1997). Prior to mass spectrometric analysis, it can be advantageous to fractionate the protein digest, for example by subjecting the protein digest to chromatography. Methods of fractionation of a protein sample are well known in the art, and include without limitation chromatographic methods, such as gas chromatography, paper chromatography, thin layer chromatography (TLC), liquid chromatography, column chromatography, fast protein liquid chromatography (FPLC), ion exchange chromatography, size exclusion chromatography, affinity chromatography, high performance liquid chromatography (HPLC), polyacrylamide gel electrophoresis (PAGE), capillary electrophoresis (CE), and reverse phase high performance liquid chromatography (RP-HPLC), amongst others.

In some examples of the disclosed methods, the fragment peptides generated from a protein digest are labeled with a mass identifier, for example using iTRAQ® regents (Applied Biosystems). Multiple samples can be run simultaneously using different iTRAQ® reagents that label the individual samples with different mass identifiers. By way of example, sample one can be labeled with a mass identifier (or mass tag) that has a molecular weight of 114 amu, while sample two mass identifier (or mass tag) can have a molecular weight of 117. When the samples are combined and subjected to mass spectrometric analysis, a fragment peptide from sample two will have a predictable mass difference of three amu, compared to the same fragment peptide from sample one. In other words a peptide of identical sequence in sample one and sample two will be three amu heavier. This predictable mass difference can be used both to identify the peptide fragments (and hence the protein from which they were excised) and the relative quantities of each peptide in the samples.

Aspects of the disclosed methods relate to quantitating the amount of the fragment peptides of the protein gene products of the genes listed in Table 1, such as MGF-E8 fragment peptides, present in the biological sample. The quantity of a fragment peptide present in the biological sample is proportional to the amount of the full length protein (from which the fragment peptide is derived) present in the sample prior to digestion. Thus, the disclosed method allows for the quantitation of the full length protein in the biological sample.

Protein expression levels can be quantified by mass spectrometry if peptide standards of known concentration are available. Methods for quantitating a fragment peptide include comparing an amount of the fragment peptide to a peptide standard of known amount. Typically, the peptide standards are isotopically labeled peptides, and these are added in known amounts to a non-labeled protein digest. However, non-isotopically labeled peptide standards also can be used. In one embodiment, the non-isotopically labeled peptide standard is a peptide with a randomized sequence. By way of example, the change in relative peak intensity before and after the addition of a peptide standard can be used to calculate the amount of a fragment peptide present in a biological sample, thus providing quantification of the full length protein in the sample. When a non-isotopically labeled peptide standard is used, a mass spectrum of the protein digest is obtained in the presence and absence of the non-isotopically labeled peptide standard. The ratio of the intensity of the signals with and without the addition of the non-isotopically labeled peptide standard reflects the relative amounts (or concentrations) of the fragment peptide present in a biological sample, and thus the amount of the full length protein present in the sample. It is understood that the spectra of the protein digest either with and without the peptide standard can be obtained in any order.

When an isotopically labeled peptides a used, typically the combined sample (peptide standard plus protein digest) is analyzed by mass spectrometry, and the ratios of the mass spectral signal intensities for the peptide standard and the sample peptides are measured. In some examples, the peptide standard is isotopically labeled and the peptides in the digest are not labeled. In some examples, the peptides in the digest are isotopically labeled and the peptide standard is not labeled. In other examples, the peptide standard is labeled with a different mass identifier (or mass tag) than the mass identifier (or mass tag) that labels the peptides in the digest, such that both the peptide standard and the peptides in the digest are labeled.

Typically, the peptide standard is added to the biological sample prior to the protein digest, however in some circumstances it may be advantageous to add the peptide standard after proteolytic digest. A mass spectrum of a sample containing both sample peptides and the added peptide standard typically includes one or more pairs of separated signals that are due to a sample peptide and its corresponding peptide standard. The ratio of the intensity of the signals in each pair reflects the relative amounts (or concentrations) of each peptide present in the sample. Since the amount (or concentration) of the peptide standard is known, the amount (or concentration) of the sample peptide can be calculated by multiplying the ratio of the intensity of the signal for the sample peptide to the intensity of the signal for the peptide standard by the known amount (or concentration) of the peptide standard. Furthermore, since the sample peptides are present in amounts (or concentrations) that are the same as (or related by a known ratio to) the amounts (or concentrations) of the proteins originally in the sample, a determination of the amounts (or concentrations) of the sample peptides also permits a determination of the amounts (or concentrations) of the proteins in the sample. Since the concentrations of the peptide standards are known, the concentration of the sample peptides (and the proteins they are derived from, such as full-length protein gene products of the genes listed in Table 1, for example full length MFG-E8) can be calculated using the ratios. However, external calibrants and standards can be used instead to the internal standard. External standards are run in between samples that are being quantified. The number of standards and their frequency depends on the reproducibility of the MS platform.

Peptide standards useful in the disclosed method correspond to an amino acid sequence of about 6 to about 45 amino acid residues of the proteins listed in Table , such as about 6 amino acids, about 7 amino acids, about 8 amino acids, about 9 amino acids, about 10 amino acids, about 11 amino acids, about 12 amino acids, about 13 amino acids, about 14 amino acids, about 15 amino acids, about 16 amino acids, about 17 amino acids, about 18 amino acids, about 19 amino acids, about 20 amino acids, about 21 amino acids, about 22 amino acids, about 25 amino acids, about 30 amino acids, about 35 amino acids, about 40 amino acids, or about 45 amino acids. In some embodiments, the peptide standard corresponds to an amino acid sequence in MFG-E8. In some embodiments, the peptide standard does not correspond to an amino acid sequence of medin (amino acid residues 268-317 of SEQ ID NO: 5). In some embodiments, the peptide standard corresponds to an amino acid sequence in MFG-E8 and includes an amino acid sequence as set forth as SEQ ID NO: 1, SEQ ID NO: 2, SEQ ID NO: 9, SEQ ID NO: 10, SEQ ID NO: 11, or SEQ ID NO: 12. In some embodiments, the peptide standard consists of an amino acid sequence as set forth as one of SEQ ID NO: 1, SEQ ID NO: 2, SEQ ID NO: 9, SEQ ID NO: 10, SEQ ID NO: 11, or SEQ ID NO: 12. In some embodiments, the peptide standard is labeled with an isotope, such as a heavy stable isotope. Examples of particularly useful heavy stable isotopes are ¹⁸O, ¹⁷O, ³⁴S, ¹⁵N, ¹³C, and ²H. Peptide standards can be labeled with one or more isotopes, for example a labeled peptide can contain ¹⁸O, ¹⁷O, ¹⁵N, ³⁴S, ¹³C, and ²H or any combination thereof. Methods of labeling peptides with heavy isotopes are well known in the art and exemplary methods are given below.

### ii. Immunodetection of Proteins

The availability of antibodies specific to one or more of the protein gene products (or a fragment or modified form thereof) of the genes listed in Table 1, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the protein gene products of the genes listed in Table 1, facilitates the detection and quantitation of these proteins by one of a number of immunoassay methods that are well known in the art, such as those presented in Harlow and Lane (Antibodies, A Laboratory Manual, CSHL, New York, 1988). Methods of producing antibodies are also known in the art.

Any standard immunoassay format (such as ELISA, Western blot, or RIA assay) can be used to measure protein levels. Thus, the level of one or more of the protein gene products (or a fragment or modified form thereof) of the genes listed in Table 1, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the protein gene products of the genes listed in Table 1, in a biological sample can be evaluated using these methods.

Immunohistochemical techniques can also be utilized for detection and quantification. General guidance regarding such techniques can be found in Bancroft and Stevens (Theory and Practice of Histological Techniques, Churchill Livingstone, 1982) and Ausubel et al. (Current Protocols in Molecular Biology, John Wiley & Sons, New York, 1998). Quantitation of the protein encoded by any of the genes listed in Table 1 can be achieved by immunoassay. A statistically significant increase or decrease in the amount can be evaluated using statistical methods disclosed herein and/or known in the art.

In other examples, the antibody that specifically binds a protein gene product of a gene listed in Table 1 is directly labeled with a detectable label. In another example, each antibody that specifically binds a protein encoded by a gene listed in Table 1 is unlabeled and a second antibody or other molecule that can bind the first antibody that specifically binds the protein encoded by a gene listed in Table 1 is labeled. As is well known to one of skill in the art, a second antibody is chosen that is able to specifically bind the specific species and class of the first antibody. For example, if the first antibody is a human IgG, then the secondary antibody can be an anti-human-IgG. Other molecules that can bind to antibodies include, without limitation, Protein A and Protein G, both of which are available commercially.

Suitable labels for an antibody or secondary antibody include various enzymes, prosthetic groups, fluorescent materials, luminescent materials, magnetic agents and radioactive materials. Non-limiting examples of suitable enzymes include horseradish peroxidase, alkaline phosphatase, beta-galactosidase, or acetylcholinesterase. Non-limiting examples of suitable prosthetic group complexes include streptavidin/biotin and avidin/biotin. Non-limiting examples of suitable fluorescent materials include umbelliferone, fluorescein, fluorescein isothiocyanate, rhodamine, dichlorotriazinylamine fluorescein, dansyl chloride or phycoerythrin. A non-limiting exemplary luminescent material is luminol; a non-limiting exemplary magnetic agent is gadolinium, and non-limiting exemplary radioactive labels include ¹²⁵I, ¹³¹I, ³⁵S or ³H.

In an alternative example, proteins encoded by the genes listed in Table 1 can be assayed in a biological sample by a competition immunoassay utilizing standards of a protein encoded by a gene listed in Table 1 labeled with a detectable substance and an unlabeled antibody that specifically binds the desired protein encoded by a gene listed in Table 1. In this assay, the sample, the labeled standards, and the antibody that specifically binds the desired protein encoded by a gene listed in Table 1 are combined and the amount of labeled standard bound to the unlabeled antibody is determined. The amount of protein encoded by a gene listed in Table 1 in the biological sample is inversely proportional to the amount of labeled standard bound to the antibody that specifically binds the protein encoded by a gene listed in Table 1. Other capture and detecting reagents than antibodies can be used including aptamers and peptoids.

### iii. Other Methods of Protein Detection

One can also measure intact mass of proteins or endogenously produced peptides (a common commercial method is SELDI) in which the fragment or protein is captured on a chip, surface, or matrix based on a intrinsic protein character such as hydrophobicity, biospecificity (for example an antibody) to enrich for the fragment or protein of interest. Samples enriched for the fragment or protein of interest can be analyzed directly. For example, MALDI TOF or MALDI TOF TOF instruments can be used to assess the presence of the analyte. However, other MS instruments can also be used that enhance analyte capture or desorption. For example a triple quadropole MS which will target the specific peptide or protein can be used. In other embodiments, the sample enriched for the fragment or protein of interest is subjected to a protein digest, for example a serine protease digest, prior to further analysis.

Briefly, enrichment strategies (if needed) allow one to selectively capture analytes (protein or peptides of interest, such as proteins encoded by genes listed in Table 1). Chromatographic surfaces or matrixes can be composed of hydrophobic, hydrophilic, ion exchange, immobilized metal, or other chemistries. For example, the surface chemistry can include binding functionalities based on oxygen-dependent, carbon-dependent, sulfur-dependent, and/or nitrogen-dependent means of covalent or noncovalent immobilization of analytes. The activated surfaces are used to covalently immobilize specific "bait" molecules such as antibodies, receptors, or oligonucleotides often used for biomolecular interaction studies such as protein-protein and protein-DNA interactions.

The surface chemistry allows the bound analytes to be retained and unbound materials to be washed away. Subsequently, analytes bound to the surface can be desorbed and analyzed by any of several means, for example using mass spectrometry. When the analyte is ionized in the process of desorption, such as in laser desorption/ionization mass spectrometry, the detector can be an ion detector. Mass spectrometers generally include means for determining the time-of-flight of desorbed ions. This information is converted to mass. However, one need not determine the mass of desorbed ions to resolve and detect them: the fact that ionized analytes strike the detector at different times provides detection and resolution of them. Alternatively, the analyte can be detectably labeled (for example, with a fluorophore or radioactive isotope). In these cases, the detector can be a fluorescence or radioactivity detector. A plurality of detection means can be implemented in series to fully interrogate the analyte components and function associated with retained molecules at each location in the array. In some examples an array is on the surface of a substrate. In some examples, an array can also be a gel matrix.

Therefore, in a particular example, the chromatographic surface includes antibodies that specifically bind one or more of the protein gene products (or a fragment or modified form thereof) of the genes listed in Table 1, such as 1,2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the protein gene products of the genes listed in Table 1.

In another example, antibodies are immobilized onto the surface using a bacterial Fc binding support (Hess et al., J Chromatogr B Analyt Technol Biomed Life Sci. 815(1-2):65-75, 2005). The chromatographic surface is incubated with a sample. The antigens present in the sample can recognize the antibodies on the chromatographic surface. The unbound proteins and mass spectrometric interfering compounds are washed away and the proteins that are retained on the chromatographic surface are analyzed and detected by MALDI TOF. Quantification is required using standards.

In some examples, enrichment is not required and by specifically targeting the mass of the peptide one can isolate these peptides within the mass spectrometer. This is done using methods such as, but not exclusively, multiple reaction monitoring.

### iv. Mass Spectrometric Methods

Mass spectrometry is particularly suited to the identification of peptides or proteins from biological samples, such as the protein gene products of the genes listed in Table 1 or fragments thereof, such as proteolytic cleavage fragments, or modified forms thereof. Mass spectrometry also is particularly useful in the quantitation of peptides in a biological sample, for example using isotopically labeled peptide standards. The application of mass spectrometric techniques to identify proteins in biological samples is known in the art and is described, for example, in Akhilesh et al., Nature, 405:837-846, 2000; Dutt et al., Curr. Opin. Biotechnol., 11:176-179, 2000; Gygi et al., Curr. Opin. Chem. Biol., 4 (5): 489-94, 2000; Gygi et al., Anal. Chem., 72 (6): 1112-8, 2000; and Anderson et al., Curr. Opin. Biotechnol., 11:408-412, 2000.

Separation of ions according to their m/z ratio can be accomplished with any type of mass analyzer, including quadrupole mass analyzers (Q), time-of-flight (TOF) mass analyzers (for example, linear or reflecting) analyzers, magnetic sector mass analyzers, 3D and linear ion traps (IT), Fourier-transform ion cyclotron resonance (FT-ICR) analyzers, Orbitrap analyzers (like LTQ-Orbitrap LC/MS/MS) and combinations thereof (for example, a quadrupole-time-of-flight analyzer, or Q-TOF analyzer). As well triple quadropole instrument can be used such as the Q-trap.

In some embodiments, the mass spectrometric technique is tandem mass spectrometry (MS/MS) and the presence of a peptide of a protein gene product of a gene listed in Table 1 or a fragment thereof is detected. Typically, in tandem mass spectrometry a protein gene product of a gene listed in Table 1 or a fragment or modified form thereof entering the tandem mass spectrometer is selected and subjected to collision induced dissociation (CID). The spectrum of the resulting fragment ion is recorded in the second stage of the mass spectrometry, as a so-called CID or ETD spectrum. Because the CID or ETD process usually causes fragmentation at peptide bonds and different amino acids for the most part yield peaks of different masses, a CID or ETD spectrum alone often provides enough information to determine the presence of a peptide such as a fragment peptide of a protein gene product of a gene listed in Table 1. Suitable mass spectrometer systems for MS/MS include an ion fragment r and one, two, or more mass spectrometers, such as those described above. Examples of suitable ion fragmentors include, but are not limited to, collision cells (in which ions are fragmented by causing them to collide with neutral gas molecules), photo dissociation cells (in which ions are fragmented by irradiating them with a beam of photons), and surface dissociation fragmentor (in which ions are fragmented by colliding them with a solid or a liquid surface). Suitable mass spectrometer systems can also include ion reflectors.

Prior to mass spectrometry, the sample can be subjected to one or more dimensions of chromatographic separation, for example, one or more dimensions of liquid or size exclusion chromatography. Representative examples of chromatographic separation include paper chromatography, thin layer chromatography (TLC), liquid chromatography, column chromatography, high performance liquid chromatography (HPLC), fast protein liquid chromatography (FPLC), ion exchange chromatography, size exclusion chromatography, affinity chromatography, high performance liquid chromatography (HPLC), nano-reverse phase liquid chromatography (nano-RPLC), polyacrylamide gel electrophoresis (PAGE), capillary electrophoresis (CE), reverse phase high performance liquid chromatography (RP-HPLC) or other suitable chromatographic techniques. Thus, in some embodiments, the mass spectrometric technique is directly or indirectly coupled with a one, two or three dimensional liquid chromatography technique, such as column chromatography, high performance liquid chromatography (HPLC or FPLC), reversed phase, ion exchange chromatography, size exclusion chromatography, affinity chromatography (such as protein or peptide affinity chromatography, immunoaffinity chromatography, lectin affinity chromatography, etc.), or one, two or three dimensional polyacrylamide gel electrophoresis (PAGE), or one or two dimensional capillary electrophoresis (CE) to further resolve the biological sample prior to mass spectrometric analysis.

### v. Multiple Reaction Monitoring

In multiple reaction monitoring (MRM), the challenge is the selection of the correct tryptic peptides to use as markers for the abundance of specific proteins of interest, such as the protein gene products of the genes listed in Table 1. This selection is relatively straightforward if the protein has been identified by MS, which is the case for the proteins listed in Table 1, such that the peptides are observable in a mass spectrometer (for example an LTQ Orbitrap).

The process of establishing an MRM assay for a protein consists of a number of steps: 1) selection of the appropriate peptide(s) unique to the protein of interest and showing high MS signal response (prototypic peptides) which will help maximize the sensitivity of the assay; 2) selection of predominant peptide fragments specific (MS/MS) for the parent peptide, (useful MRM transition); 3) for each peptide-fragment pair, optimization of specific MS parameters (for example, the collision energy) to maximize the signal response/sensitivity; 4) validation of the MRM assay to confirm peptide identity, for example by acquiring a full MS2 spectrum of the peptide in the triple quadrupole MS instrument used for MRM; 5) extraction of the final "coordinates" of the MRM assay, including the selected peptide and peptide fragments, the corresponding mass-to-charge ratios, the fragment intensity ratios, the associated collision energy, and the chromatographic elution time to be optionally used in time-constrained MRM analyses. In some examples, isotopically labeled internal peptide standards (with known concentrations determined by amino acid analysis) are used to facilitate absolute quantitation of selected peptides.

Specific SNP or co- or post-translational modifications can also be targeted, such as phosphorylation or cleavage at a specific residue. In this case both the modified and unmodified peptides are targeted. In the case of endogenous proteolysis, MRM assay can be carried out directly on the sample without *in vitro* digestion. In some examples, such as for very low abundant proteins or peptides, the sample is enriched (for example using MARS columns or other techniques known in the art). This can be done using methods already described. However in serum or plasma (and other body fluids), this could mean the depletion or removal of the top abundant proteins, such as albumin or immunoglobulins. There are chemical and affinity based methods that can be used. For example, proteins or protein fragments can also be enriched based on post-translational modification, for example using antibodies specific for the post-translational modification. For example MFG-E8 could be isolated for analysis based on its N-linked glyco modification. This can be done a number of ways including biotinylation, antibodies, and lectins.

### vi. Isotopically-Labeled Peptides

In some embodiments, the peptide standard is labeled with an isotope, such as a heavy stable isotope. Examples of particularly useful heavy stable isotopes are ¹⁸O, ¹⁷O, ³⁴S, ¹⁵N, ¹³C, and ²H. Useful isotopically labeled peptide standards have a predictable number of sites where the heavy isotope replaces a non-heavy isotope yielding a peptide with a predictable mass difference from a peptide that does not have any heavy isotopes incorporated. Thus, the isotopic-labeling of the peptide standards yields separate distinct mass spectrometric signals from peptides obtained from the biological samples. These isotopically labeled peptide standards can be used to quantify proteins (or a fragment or modified form thereof) present in biological samples.

Stable isotopes can be incorporated into peptides either biologically (*in vivo*) or chemically (*in vitro).* Most often synthetic peptides are made in which an amino acid with a stable isotope is incorporated. However, there are methods for providing isotopically-labeled peptide standards are to express the peptides in a host cell (such as *E. coli*) grown on an isotopically-altered medium. Alternatively, peptides can be expressed in cell free systems. Isotopically-altered medium is a growth medium that is enriched in one or more stable heavy isotopes of an element or elements relative to their natural isotopic abundances. For example, a growth medium including greater amounts of ²H, ¹³C, ³⁴S, ¹⁵N, ¹⁷O, and/or ¹⁸O than are found in nature is an isotopically-altered medium. Enrichment of the medium with stable heavy isotopes can be partial (where both heavy and light isotopes of a particular element are present in the medium), or uniform (where substantially only heavy isotopes of a particular element are present, such as greater than 90%, 95%, 98% or 99% of the atoms of an element are the heavy isotope). Stable heavy isotopes can be added to the medium in any form. For example, the isotopes can be added in the form of a simple chemical substance such as ¹⁵NH₃, ¹³C-glucose, ²H₂O, or can be added in the form of a more complex substance such as an isotopically-altered amino acid (for example, amino acids labeled with ²H, ¹³C, ³⁴S, ¹⁵N, ¹⁷O, and/or ¹⁸O, such as deuterium-enriched leucine, serine, and/or tyrosine). Uniform labeling media refers to a growth medium wherein substantially all atoms (such as greater than 90%, 95%, 98% or 99% of all atoms) of a particular element present in the medium are present in the form of a particular isotope of the element. A uniformly-labeled growth medium provides a particular type of atom substantially in the form of a single isotope of the atom. For example, a medium that provides nitrogen in the form of ¹⁵NH₃ as the sole nitrogen source for host cells grown on the medium is a uniformly-labeled media. Thus, a peptide standard expressed in host cells grown in media with ¹⁵NH₃ as the sole nitrogen source will be uniformly isotopically labeled with ¹⁵N.

In some instances peptides expressed in a host cell are unstable and prone to degradation (see, for example, Lindhout et al., Protein Science, 12: 1786-1791, 2003). One solution to the stability problem is to express peptides in a fusion construct with a larger fusion protein partner. The fusion protein approach has been applied to produce a single peptide or a few copies of a single peptide (a homopolymer of peptides) as part of the fusion protein (see, Jones et al., Biochemistry, 39: 1870-1878, 2000; Majerle et al., J. Biomol. NMR, 18: 145-151, 2000; Sharon et al., Protein Expr. Purif., 24: 374-383, 2002; and Lindhout et al., Protein Science, 12: 1786-1791, 2003). The peptide standard is then excised from the fusion construct, for example by proteolytic cleavage with an endopeptidase such as trypsin. Peptide standards can also be produced from the protein gene products of the genes listed in Table 1 or a portion thereof that is expressed in an isotopically-altered medium (for example, in a host cell). The resulting protein or a portion thereof can be digested with an endoprotease (such as trypsin) to excise fragment peptides that can be used as peptide standards. Peptide standards, such as a peptide corresponding to a portion of the protein gene products of the genes listed in Table 1, can be subsequently purified and quantitated for use as a peptide standard of known molecular weight and concentration.

Isotopically-labeled peptide standards of known concentration and molecular weight can be synthesized from isotopically labeled amino acids. Peptide synthesis is well known in the art (see, for example, Atherton and Sheppard, Solid Phase Peptide Synthesis: a Practical Approach published by published by Oxford University Press, USA, and Chan and White Fmoc Solid Phase Peptide Synthesis: A Practical Approach, published by Oxford University Press, USA).

In other examples, such as employed in the iTRAQ® methods isotopic labels are incorporated into a peptide by chemical modification of the peptide with a chemical reagents that that are differentially isotopically labeled.

### B. Evaluating Nucleic Acids

Gene expression can be evaluated by detecting mRNA transcribed from a gene of interest in a biological sample, or cDNA reverse transcribed from such mRNA, thereby detecting the mRNA indirectly. Thus, the disclosed methods can include evaluating mRNA encoding one or more of the protein gene products (or a fragment or modified form thereof) of the genes listed in Table 1, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50, of the protein gene products of the genes listed in Table 1. In particular examples, mRNA encoding MFG-E8 is evaluated. In some examples, the mRNA or cDNA is quantitated, for example using a PCR and/or array based method.

### i. Evaluation of RNA

RNA can be isolated from a biological sample isolated from a subject of interest, such as a subject being evaluated for an age-associated vascular disorder, using methods well known to one skilled in the art, including commercially available kits. General methods for mRNA extraction are well known in the art and are disclosed in standard textbooks of molecular biology, including Ausubel et al., Current Protocols of Molecular Biology, John Wiley and Sons (1998). Methods for RNA extraction from paraffin embedded tissues are disclosed, for example, in Rupp and Locker, Biotechniques. 6(1):56-60, 1987, and De Andres et al., BioTechniques 18:42-44, 1995. In one example, RNA isolation can be performed using a purification kit, buffer set and protease from commercial manufacturers, such as QIAGEN®, according to the manufacturer's instructions. For example, total RNA from cells (such as those obtained from a subject) can be isolated using QIAGEN® RNeasy mini-columns. Other commercially available RNA isolation kits include MASTERPURE® Complete DNA and RNA Purification Kit (EPICENTRE® Madison, Wis.), and Paraffin Block RNA Isolation Kit (Ambion®, Inc.). Total RNA from tissue samples can be isolated using RNA Stat-60 (Tel-Test). RNA prepared from tumor or other biological sample can be isolated, for example, by cesium chloride density gradient centrifugation.

Methods of gene expression profiling include methods based on hybridization analysis of polynucleotides, methods based on sequencing of polynucleotides, and other genomics-based methods. In some examples, mRNA expression in a sample is quantified using northern blotting or *in situ* hybridization (Parker & Barnes, Methods in Molecular Biology 106:247-283, 1999); RNAse protection assays (Hod, Biotechniques 13:852-4, 1992); and PCR-based methods, such as reverse transcription polymerase chain reaction (RT-PCR) (Weis et al., Trends in Genetics 8:263-4, 1992). Alternatively, antibodies can be employed that can recognize specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE), and gene expression analysis by massively parallel signature sequencing (MPSS). In one example, reverse-transcription (RT)-PCR can be used to compare mRNA levels in different samples, to characterize patterns of gene expression, to discriminate between closely related mRNAs, and to analyze RNA structure.

Methods for quantitating mRNA are well known in the art. In one example, the method utilizes RT-PCR. Generally, the first step in gene expression profiling by RT-PCR is the reverse transcription of the RNA template into cDNA using a reverse transcriptase, followed by its exponential amplification in a PCR reaction. Two commonly used reverse transcriptases are avian myeloblastosis virus reverse transcriptase (AMV-RT) and Moloney murine leukemia virus reverse transcriptase (MMLV-RT). The reverse transcription step is typically primed using specific primers, random hexamers, oligo-dT primers, or a combination thereof, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Two oligonucleotide primers are used to generate an amplicon typical of a PCR reaction. A third oligonucleotide, or probe, is designed to detect nucleotide sequence located between the two PCR primers. In quantitative RT-PCR, the probe can be labeled, for example with a fluorescent molecule, such that the amount of fluorescent signal emitted by the probe, when bound to the amplicon, is used as a measure of the amount of amplicon generated in a sample.

Real-time PCR, such as TaqMan® Real-time PCR, is used to monitor the progress of a PCR reaction and measure the amount of amplicon generated over the course of a specific time period (in real time). The amount of amplicon generated can also be quantified in a real-time RT-PCR reaction. Typically real-time RT-PCR utilizes the 5'-nuclease activity of Taq or Tth polymerase to hydrolyze a hybridization probe bound to its target amplicon, but any enzyme with equivalent 5' nuclease activity can be used. In some embodiments, the probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together, as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

RT-PCR can be performed using commercially available equipment, such as, for example, ABI PRISM 7700® Sequence Detection System® (Perkin-Elmer-Applied Biosystems, Foster City, CA), or Lightcycler (Roche Molecular Biochemicals, Mannheim, Germany). In one example, the 5' nuclease procedure is run on a real-time quantitative PCR device such as the ABI PRISM 7700® Sequence Detection System®. The system includes of thermocycler, laser, charge-coupled device (CCD), camera and computer. The system amplifies samples in a 96-well format on a thermocycler. During amplification, laser-induced fluorescent signal is collected in real-time through fiber optic cables for all 96 wells, and detected at the CCD. The system includes software for running the instrument and for analyzing the data.

The point when the fluorescent signal is first recorded as statistically significant is the threshold cycle (Ct). Thus, in some examples, 5'-nuclease assay data are initially expressed as Ct, or the threshold cycle. As discussed above, fluorescence values are recorded during every cycle and represent the amount of product amplified to that point in the amplification reaction.

To minimize errors and the effect of sample-to-sample variation, RT-PCR is can be performed using an internal standard. The ideal internal standard is expressed at a constant level among different tissues, and is unaffected by the experimental treatment. RNAs commonly used to normalize patterns of gene expression are mRNAs for the housekeeping genes glyceraldehyde-3-phosphate-dehydrogenase (GAPDH), beta-actin, and 18S ribosomal RNA.

A variation of RT-PCR is real time RT-PCR, which measures PCR product accumulation through a dual-labeled fluorogenic probe (e.g. TAQMAN® probe). Real time PCR is compatible both with quantitative competitive PCR, where internal competitor for each target sequence is used for normalization, and with quantitative comparative PCR using a normalization gene contained within the sample, or a housekeeping gene for RT-PCR (see Held et al., Genome Research 6:986-994, 1996). Quantitative PCR is also described in U.S. Patent No. 5,538,848. Related probes and quantitative amplification procedures are described in U.S. Patent No. 5,716,784 and U.S. Patent No. 5,723,591. Instruments for carrying out quantitative PCR in microtiter plates are available from PE Applied Biosystems, 850 Lincoln Centre Drive, Foster City, CA 94404 under the trademark ABI PRISM® 7700.

The primers used for the amplification are selected so as to amplify a unique segment of the gene of interest, such as mRNA encoding the protein gene products (or a fragment or modified form thereof) of the genes listed in Table 1, such as 1,2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the protein gene products of the genes listed in Table 1. In some examples, primers are selected that amplify a segment of an MFG-E8 nucleic acid, such as a segment of human MFG-E8 that does not include the segment of MFG-E8 encoding medin.

An alternative quantitative nucleic acid amplification procedure is described in U.S. Patent No. 5,219,727. In this procedure, the amount of a target sequence in a sample is determined by simultaneously amplifying the target sequence and an internal standard nucleic acid segment. The amount of amplified DNA from each segment is determined and compared to a standard curve to determine the amount of the target nucleic acid segment that was present in the sample prior to amplification.

As discussed above, in some embodiments of this method, the expression of a "house kneeping" gene or "internal control" can also be evaluated. These terms include any constitutively or globally expressed gene whose presence enables an assessment of mRNA levels of genes of interest. Such an assessment includes a determination of the overall constitutive level of gene transcription and a control for variations in RNA recovery.

In some examples, gene expression is identified or confirmed using the microarray technique. Thus, the expression profile can be measured in either fresh or paraffin-embedded tissue, using microarray technology. In this method, nucleic acid sequences of interest (including cDNAs and oligonucleotides) are plated, or arrayed, on a microchip substrate. The arrayed sequences are then hybridized with specific DNA probes from biological samples obtained from subjects.

In a specific embodiment of the microarray technique, PCR amplified inserts of cDNA clones are applied to a substrate in a dense array. Probes for the nucleotide sequences encoding the genes listed in Table 1 are applied to the substrate. The microarrayed nucleic acids are suitable for hybridization under stringent conditions.

Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern of one or more the protein gene products of the genes listed in Table 1, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the of the genes listed in Table 1. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):10614-9, 1996). Microarray analysis can be performed by commercially available equipment, following manufacturer's protocols, such as are supplied with Affymetrix® GenChip® technology, or Incyte's microarray technology.

Serial analysis of gene expression (SAGE) is another method that allows the simultaneous and quantitative analysis of a large number of gene transcripts, without the need of providing an individual hybridization probe for each transcript. First, a short sequence tag (about 10-14 base pairs) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, for example, Velculescu et al., Science 270:484-7, 1995; and Velculescu et al., Cell 88:243-51, 1997.

Yet another approach for detecting and quantitating RNA involves mass spectrometry. RNAs can be detected if they are enzymatically digested to nucleosides and the resulting mixture analyzed using liquid chromatography-mass spectrometry (LC-MS) approaches. A number of MS-based approaches also have been developed to reveal the presence of modified nucleosides that also identify the sequence locations of any modified nucleosides within the original RNA. The analytical strategy is based upon the unique attributes of post-transcriptionally modified nucleosides, as post-transcriptionally modified nucleosides have a higher molecular mass than their unmodified counterpart (e.g., methylation results in a 14 Dalton mass increase). Because MS is sensitive to changes in molecular mass, the presence of such modifications in oligonucleotides, such as endonuclease digestion products or intact RNAs, is noted by this increase in molecular mass. The analytical strategy for recognizing modified nucleosides is predicated on calculating predicted molecular masses from the gene or cDNA sequence of the RNA of interest. Subsequent analysis by MS is then used to determine whether the predicted masses are present, which signify no post-transcriptionally modified nucleosides are in that RNA sample, or if anomalous masses are detected, which can then be attributed to the presence of one or more post-transcriptionally modified nucleosides, assuming the original gene sequence was correct.

Quantitation generally involves total hydrolysis of RNA to nucleosides and subsequent quantification using LC/MS. As stable isotope labeling in combination with MS has become a routine quantification approach in proteomics, MS-based quantification of RNA at the oligonucleotide level using ¹⁸O labelling has been demonstrated in the manner similar to that performed for proteomics. One RNA sample is digested with RNase T1 in ¹⁸O-labelled ('heavy') water and the second RNA sample is digested with RNase T1 in normal ('light') water. The two samples are then combined and analyzed by MALDI-MS. Relative ion abundances of the light- and heavy-water digestion products, which are separated by 2 daltons due to the isotopic mass difference of ¹⁸O compared with ¹⁶O, reveal relative quantification information about the two RNA samples. This approach allows relative quantitation of the original intact RNA and is readily applicable to the quantitative determination of post-transcriptional modifications of mRNA.

### ii. Arrays

Arrays are disclosed herein that include oligonucleotide probes for one or more of the nucleic acids encoding the genes listed in Table 1, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or all 50 of the genes listed in Table 1.

The methods in accordance with the present disclosure take advantage of the fact that under appropriate conditions oligonucleotides form base-paired duplexes with nucleic acid molecules that have a complementary base sequence. Thus, the arrays disclosed herein can be used to detect, identify and/or quantitate one or more of the nucleic acid gene products of one or more of the genes listed in Table 1 by hybridizing with a nucleic acid gene product of a gene listed in Table 1 or a subsequence thereof.

The stability of the duplex is dependent on a number of factors, including the length of the oligonucleotides, the base composition, and the composition of the solution in which hybridization is effected. The effects of base composition on duplex stability can be reduced by carrying out the hybridization in particular solutions, for example in the presence of high concentrations of tertiary or quaternary amines.

The thermal stability of the duplex is also dependent on the degree of sequence similarity between the sequences. By carrying out the hybridization at temperatures close to the anticipated melting temperatures Tₘs of the type of duplexes expected to be formed between the target sequences, such as a nucleic acid sequence of a gene listed in Table 1 and the oligonucleotides bound to the array, the rate of formation of mis-matched duplexes can be substantially reduced.

The length of each oligonucleotide sequence employed in the array can be selected to optimize binding to an mRNA. An optimum length for use with a particular marker nucleic acid sequence under specific screening conditions can be determined empirically. Thus, the length for each individual element of the set of oligonucleotide sequences included in the array can be optimized for screening. In various examples, oligonucleotide probes are from about 20 to about 35 nucleotides in length or about 25 to about 40 nucleotides in length.

The oligonucleotide probe sequences forming the array can be directly linked to the support, for example via the 5'- or 3'-end of the probe. In one example, the oligonucleotides are bound to the solid support by the 5' end. However, one of skill in the art can determine whether the use of the 3' end or the 5' end of the oligonucleotide is suitable for bonding to the solid support. In general, the internal complementarity of an oligonucleotide probe in the region of the 3' end and the 5' end determines binding to the support. Alternatively, the oligonucleotide probes can be attached to the support by sequences such as oligonucleotides or other molecules that serve as spacers or linkers to the solid support.

In particular examples, the array is a microarray formed from glass (silicon dioxide). Suitable silicon dioxide types for the solid support include, but are not limited to: aluminosilicate, borosilicate, silica, soda lime, zinc titania and fused silica (for example see Schena, Micraoarray Analysis. John Wiley & Sons, Inc, Hoboken, New Jersey, 2003). The attachment of nucleic acids to the surface of the glass can be achieved by methods known in the art, for example by surface treatments that form from an organic polymer. Particular examples include, but are not limited to: polypropylene, polyethylene, polybutylene, polyisobutylene, polybutadiene, polyisoprene, polyvinylpyrrolidine, polytetrafluroethylene, polyvinylidene difluroide, polyfluoroethylene-propylene, polyethylenevinyl alcohol, polymethylpentene, polycholorotrifluoroethylene, polysulfornes, hydroxylated biaxially oriented polypropylene, aminated biaxially oriented polypropylene, thiolated biaxially oriented polypropylene, etyleneacrylic acid, thylene methacrylic acid, and blends of copolymers thereof (see U.S. Patent No. 5,985,567), organosilane compounds that provide chemically active amine or aldehyde groups, epoxy or polylysine treatment of the microarray. Another example of a solid support surface is polypropylene.

In general, suitable characteristics of the material that can be used to form the solid support surface include: being amenable to surface activation such that upon activation, the surface of the support is capable of covalently attaching a biomolecule such as an oligonucleotide thereto; amenability to "in situ" synthesis of biomolecules; being chemically inert such that at the areas on the support not occupied by the oligonucleotides are not amenable to non-specific binding, or when non-specific binding occurs, such materials can be readily removed from the surface without removing the oligonucleotides.

In one example, the surface treatment is amine-containing silane derivatives. Attachment of nucleic acids to an amine surface occurs via interactions between negatively charged phosphate groups on the DNA backbone and positively charged amino groups (Schena, Microarray Analysis. John Wiley & Sons, Inc, Hoboken, New Jersey, 2003). In another example, reactive aldehyde groups are used as surface treatment. Attachment to the aldehyde surface is achieved by the addition of 5'-amine group or amino linker to the DNA of interest. Binding occurs when the nonbonding electron pair on the amine linker acts as a nucleophile that attacks the electropositive carbon atom of the aldehyde group.

A wide variety of array formats can be employed in accordance with the present disclosure. One example includes a linear array of oligonucleotide bands, generally referred to in the art as a dipstick. Another suitable format includes a two-dimensional pattern of discrete cells (such as 4096 squares in a 64 by 64 array). As is appreciated by those skilled in the art, other array formats including, but not limited to slot (rectangular) and circular arrays are equally suitable for use (see U.S. Patent No. 5,981,185). In one example, the array is formed on a polymer medium, which is a thread, membrane or film. An example of an organic polymer medium is a polypropylene sheet having a thickness on the order of about 1 mil. (0.001 inch) to about 20 mil., although the thickness of the film is not critical and can be varied over a fairly broad range. Biaxially oriented polypropylene (BOPP) films are also suitable in this regard; in addition to their durability, BOPP films exhibit low background fluorescence. In a particular example, the array is a solid phase, Allele-Specific Oligonucleotides (ASO) based nucleic acid array.

The array formats of the present disclosure can be included in a variety of different types of formats. A "format" includes any format to which the solid support can be affixed, such as microtiter plates, test tubes, inorganic sheets, dipsticks, and the like. For example, when the solid support is a polypropylene thread, one or more polypropylene threads can be affixed to a plastic dipstick-type device; polypropylene membranes can be affixed to glass slides. The particular format is, in and of itself, unimportant. All that is necessary is that the solid support can be affixed thereto without affecting the functional behavior of the solid support or any biopolymer absorbed thereon, and that the format (such as the dipstick or slide) is stable to any materials into which the device is introduced (such as clinical samples and hybridization solutions).

The arrays of the present disclosure can be prepared by a variety of approaches. In one example, oligonucleotide or protein sequences are synthesized separately and then attached to a solid support (see U.S. Patent No. 6,013,789). In another example, sequences are synthesized directly onto the support to provide the desired array (see U.S. Patent No. 5,554,501). Suitable methods for covalently coupling oligonucleotides and proteins to a solid support and for directly synthesizing the oligonucleotides or proteins onto the support are known to those working in the field; a summary of suitable methods can be found in Matson et al., Anal. Biochem. 217:306-10, 1994. In one example, the oligonucleotides are synthesized onto the support using conventional chemical techniques for preparing oligonucleotides on solid supports (such as see PCT Publication No. WO 85/01051 and PCT Publication No. WO 89/10977, or U.S. Patent No. 5,554,501).

A suitable array can be produced using automated means to synthesize oligonucleotides in the cells of the array by laying down the precursors for the four bases in a predetermined pattern. Briefly, a multiple-channel automated chemical delivery system is employed to create oligonucleotide probe populations in parallel rows (corresponding in number to the number of channels in the delivery system) across the substrate. Following completion of oligonucleotide synthesis in a first direction, the substrate can then be rotated by 90° to permit synthesis to proceed within a second (2°) set of rows that are now perpendicular to the first set. This process creates a multiple-channel array whose intersection generates a plurality of discrete cells.

In particular examples, the oligonucleotide probes on the array include one or more labels, which permit detection of oligonucleotide probe:target sequence hybridization complexes.

The following examples are provided to illustrate particular features of certain embodiments. However, the particular features described below should not be construed as limitations, but rather as examples from which equivalents will be recognized by those of ordinary skill in the relevant art. Some of the data presented in the following examples are included in Fu et al., Circ. Res., 104:1337-1346, 2009. The online version of Fu et al., Circ. Res., 104:1337-1346, 2009, including the accompanying Data Supplement, was published online on May 14,2009 with updates through October 26, 2009.

### EXAMPLES

### Example 1

### Materials and Methods

This example describes the preparation of arterial samples used in the Examples.

### Arterial Specimens:

*Rats:* Male Fisher 344 crossbred Brown Norway rats (FXBN), 8-month-old (8 mo) and 30-month-old (30 mo), were obtained from the National Institute on Aging Contract Colonies (Harlan Sprague Dawley, Inc, Indianapolis, IN). All of the animals were sacrificed by an overdose of sodium pentobarbital, and thoracic aortae were immediately removed, isolated, and processed as described previously by Wang et al. Arterioscler Thromb Vasc Biol 26:1503-1509, 2006.

*Nonhumans Primates:* Four young, (age range of 10.4±1.6 years) and four old, (age range of 22.2±3.4 years) male rhesus monkeys (*Macaca fascicularis*) were maintained according to methods previously described by Wang et al., Hypertension, 41:1308-1316,2003. Segments of the thoracic aorta were harvested as described above for rodents in Wang et al., Arterioscler Thromb Vasc Biol 26:1503-1509, 2006.

*Human Aorta Preparations:* Segments of descending thoracic aortae were procured within 24-48 hours of non-cardiac deaths due to external causes (homicide, suicide, or accident). Grossly normal intima and media of six Caucasian males were obtained. The samples were divided into two age groups: young (19±3 years, n=5), and old (67±10 years, n=5). The samples were obtained according to the methods previously described in Wang et al., Hypertension 50: 219-227, 2007.

*Preparation of Aortic Proteins:* The tissues from isolated aortae were cut into small pieces and homogenized with 8 M urea, 2 M thiourea, 4% 3[(3-cholamidopropyl)dimethylammonio]-propanesulfonic acid (CHAPS), 50 mM dithiothreitol (DTT). The crude extract was then centrifuged at 16,000 g at room temperature for 30 minutes. The supernatant fraction was immediately transferred to new pre-chilled tubes and snap-frozen in aliquots with liquid nitrogen and stored at -80 °C. Protein concentrations of these samples were determined by using the 2D-Quant-Kit (GE Healthcare) with bovine serum albumin (BSA) as a standard.

### Example 2

### Quantification and Identification of the Aortic Proteome by 2DE and iTRAQ™ MS Based Analysis

This example describes the methods used to determine the identity and relative expression level of proteins that are differentially expressed in the aorta of old and young rats.

Using 2DE DIGE, a 2DE gel map of 286 proteins was identified from rat aorta. It was observed that 18 of these proteins changed abundance with aging (see Table 1). Using iTRAQ™, 880 proteins were quantified with 50 proteins having differential abundance associated with aged aortas (see Table 1). The two methods produced complimentary results and therefore, increased the confidence of the results. In general, less than 5 % of the proteins identified in rat aorta exhibited different abundance between young and old rat aorta. The majority of these proteins have no significant post translational modifications (PTMs). Of the proteins found to differ with aging, some were shown to have a PTM. For example, acidic calponin 3 is a phosphoprotein, while MFG-E8, kininogen 1, and periostin were detected as N-linked glycoproteins (see Table 1).

Quantification and characterization of MFG-E8 by 2DE DIGE and iTRAQ™ in aging rat aorta show that MFG-E8 is more abundant in aged aorta than in young adult samples (2.53±0.15, n=8). iTRAQ™ spectra demonstrated that MFG-E8 is more abundant in aged than young adult aorta. Representative spectra of two peptides [VLPLSWHNR (SEQ ID NO: 1; amino acid residues 409-417 of MFG-E8) and CLVTEDTQR (SEQ ID NO: 2; amino acid residues 79-87 of MFG-E8)] indicate the abundance of MFG-E8 is ∼2.4 fold increased in old versus young aorta.

To ensure statistical significance for data obtained from the young and old rats, the following design was applied to the analysis of the rat aortic proteome. Equal amounts (50 µg) of young (Cy3), old (Cy5), and the mixture (Cy2) of the individually labeled samples were loaded per gel, resulting in 75 µg of protein from the young rats (young samples) and the old rats (old samples). The young and old samples were randomly paired. In addition, a mixture of all young samples and a mixture of all old rat samples were also paired to statistically evaluate the protein abundance variation between the young and the old animal samples.

Protein labeling was performed using the CyDyes, DIGE Fluors developed for fluorescence 2D DIGE technology (GE Healthcare), according to the manufacturer's recommended protocol. Each sample was covalently labeled with a different fluorophore, Cy2 (a mixture of young and old aorta extract), Cy3 (young), and Cy5 (old). The labeling mixture was incubated on ice in the dark for 30 minutes and centrifuged (16000 g, room temp (RT), 5 minutes). The reaction was terminated by adding 0.8 µl of 10 mM lysine (Sigma-Aldrich), vortexed and incubated on ice for 10 minutes. Each of the labeled protein samples was combined and rehydration buffer added (8 M urea, 2% w/v CHAPS, 0.005% bromphenol blue 20 mM DTT and 1% IPG buffer) (GE Healthcare).

Labeled protein samples were subjected to 2D gel electrophoresis following protocols for pH 4-7 and 6-11 (Graham et al., Proteomics 5(9):2309-14, 2005; Kane et al. Proteomics 6(21):5683-5687, 2006). Eighteen cm Immobiline™ Dry-Strips (pH4-7) (GE Health Care) were actively rehydrated at 50V for 12 hours, followed by electrophoresis at 150V for 1.5 hour, 300V for 2 hours, 600V for 1hour, 1000V for 1 hour, a 2 hour rapid ramp up to 8000V, and finally 800V for 5 hours. For pH 6-11 strips, the strips were first rehydrated for 12 hours followed by paper-bridge loading at 200V for 8 hours. The strips were removed to strip-holders and electrophoresis started from 300V as indicated above. Ten percent Bis-Tris polyacrylamide gels (Bio-Rad) were used for second dimension SDS-PAGE. After SDS-PAGE, cyanine dye-labeled proteins were visualized using a Typhoon™ 9410 imager (GE Healthcare). Cy2 images were scanned using a 488 nm laser and an emission filter of 520 nm band pass BP 40 emission filter. Cy3 images were scanned using a 532 nm laser and a 580 nm BP 30 emission filter. Cy5 images were scanned using a 633 nm laser and a 670 nm BP 30 emission filter. All gels were scanned at 100 µm (pixel size) resolution. The photomultiplier tube (PMT) was set to 550 V by using normal sensitivity. The scanned gels were analyzed using the DeCyder software V4.0 (GE Healthcare). All Cy2 results were defined as standards when comparing gels from young and old samples.

To prepare gel spots for protein identification purpose, 400 microgram of proteins were subjected to 2D gel electrophoresis as indicated above without Cye dye labelling. At the end of the run, the gels were removed and soaked in a solution containing acetic acid/methanol/water (5:45:50, v/v/v) for at least 1 hour for fixation, followed by another hour of methanol/water and three washes for 5 minutes in water. Staining was performed with silver nitrate. An Epson 10000XL Fastsilver Imager (Agilent, Wilmington, DE) was used to visualize silver stained gel slabs. After the gels were scanned, the images were exported as TIFF files. The TIFF files of 2DE images of young and old rat aorta proteins were compared using Decyder software (GE Healthcare).

After gel image acquisition, the spots of interest were excised. In some examples, the spots were stored at -80 °C at this time or directly subjected to in-gel tryptic digestion. After digestion, peptide samples could be concentrated and stored at 80 °C or dissolved with 10 µl 0.1% TFA in water for desalting with ZipTip_{C18} pipette tips (Millipore, Bellerica, MA). In some examples, the eluted samples were used directly for mass spectrometry analysis with a MALDI-TOF mass spectrometer or dried by Speed Vac and redissolved in 1% TFA for analysis using electrospray on a Linear Trap Quadrupole (LTQ) ion trap mass spectrometer.

Routinely, the samples were first analyzed using the MALDI mass spectrometer. The instrument was operated in the reflection positive ion mode. The laser power was set at 1350-1450 on the manufacturer's scale of 0 to 5000. The monoisotopic peaks of trypsin autolysis products, m/z 842.51 and 2211.11, were used for internal calibration of each spectrum. If these trypsin autolysis peaks were not detected, melletin and angiotensin II were used as external calibrants. An aliquot of 0.5 µl of sample solution was put on the plate and covered with 0.5 µl of 50 mM alpha-hydroxycinnamic acid in 0.1% trifluoro acetic acid (TFA), 70% acetonitrile, then dried before analysis. Spectra were recorded by accumulating 100-200 laser shots, depending on the quality of the sample. Mascot search programs available on the world wild web at matrixscience.com were used to obtain protein identifications for the experiments. The SwissProt database was searched for peptides. If no conclusive identification could be acquired, the sample would be analyzed using nanospray tandem mass spectrometry to identify proteins by microsequencing.

In some examples the instrument used for acquiring tandem mass spectrometry was a LTQ ion trap mass spectrometer equipped with an on-line microcapillary high-pressure liquid chromatography (HPLC) (Eksigent, CA). Peptides were trapped on a peptide trap (3 cm, 75 µm, packed with irregular size particles 5-15 µm, 120 Å, YMC ODS-AQ) and further separated on a reverse-phase analytical column packed with 10 cm of C18 beads (360x75 µm, 5 µm, 120 Å, YMC ODS-AQ, Waters, Milford, MA) with a 10 µm emitter tip (New Objective, Woburn, MA) attached. The HPLC gradient was 5-40% B for 30 minutes (A, 0.1% formic acid; B, 90% acetonitrile in 0.1% formic acid) and then quickly bumped up to 90% B before coming back to initial conditions. The flow rate was 300 nL/minute.

In some examples the instrument used for acquiring tandem mass spectrometry was a LCQ DecaXP plus ion trap mass spectrometer equipped with an in-line microcapillary HPLC (ThermoElectron, San Jose, CA) that uses two C18 peptide captrap cartridges for high throughput (Michrom BioResources Inc., Auburn CA). The reverse-phase column was a PicoFrit 75 µm I.D. x 10 cm fused capillary with a 15 µm nanoelectrospray tip and packed with 5 µm, 300 Å BioBasic C18 particles (New Objective Inc., Woburn, MA). The samples were dried and resuspended in 10 µL 0.1% formic acid. Samples were injected using a Surveyor autosampler (ThermoElectron). After injecting 10 µL of sample, the peptides were eluted using a linear gradient of water and acetonitrile, both acidified with 0.1% (v/v) formic acid. The gradient increased from 5% acetonitrile, to 60% in 55 minute. The column was then washed with 98% acetonitrile for 5 minutes and water for 26 minute at a constant flow rate of 275 nL/minute. The ion-trap mass spectrometer was operated in a data-dependent mode in which a full MS scan was followed by MS/MS scans of the three most intensive ions. The ions were automatically selected for collision-induced dissociation (CID).

Pro-Q Diamond Phosphoprotein Stain (Invitrogen™) was used to detected phosphoproteins. To match phosphoproteins on the 2-D gel map, a small portion of the protein (50 µg) sample was labeled with Cy5 and mixed with 400 µg unlabeled proteins and were subjected to 2-D gel electrophoresis. The gel was stained with Pro-Q® Diamond Phosphoprotein Stain according to manufacturer's user manual (Agnetti et al., Pharmacol Res. 55(6):511-22, 2007). After staining, the proteins were visualized using a Typhoon™ 9410 imager (GE Healthcare). Phosphoproteins were scanned using a 532 nm laser and a 580 nm BP 30 emission filter (similar to Cy3). Total proteins were scanned using a 633 nm laser and a 670 nm BP 30 emission filter (Cy5).

To detect glycoproteins, 50 µg of sample mixture from old rats was treated with PNGase F (Davies et al., Methods in Molecular Biology, vol. 32: Basic Protein and Peptide Protocols (Walker, J. M., ed.), Humana, Totowa, NJ, pp. 129-141, 1994). The control sample (untreated) was labeled with Cy3 and treated sample was labeled with Cy5 and the combined samples were subjected to DIGE analysis.

For the iTRAQ™ trials used to identify and quantitate peptides present in rat aortic samples, two separate set of four samples each of rat aorta tissue were analyzed. Four samples were analyzed simultaneously in a single trial to identify the proteins present and differentially expressed in old versus your aortic tissue [two sets of four samples (2 old and 2 young) for a total of 4 young and 4 old rats]. Each sample from the two sets was independently digested and labeled with iTRAQ™ reagents. Each set (samples from 2 old and 2 young rats) was combined, purified and fractionated with Strong Cation Exchange Chromatography (SCX) and analyzed on a Qstar Pulsar™ (Applied Biosystems-MDS Sciex) mass spectrometer.

The samples (80 µg each) were dissolved in 20 µL of 0.1% SDS in 500 mM TEAB, reduced, alkylated and digested with trypsin with a protein to enzyme ratio 20:1 at 37 °C overnight. The iTRAQ™ reagent was dissolved in 70 µL of ethanol and added to the digest. The mixture was incubated at room temperature for 1 hour. Samples from young rats were labeled with label 114 (young 1), old with 115 (old 1), young with 116 (young 2) and old with 117 (old 2), respectively. After labeling, the samples were mixed and dried down to a volume of 50 µL. The combined peptide mixture was fractionated by SCX chromatography on an Agilent HPLC System using a PolySulfoethyl A column (2.1x100mm, 5µm, 300Å, PolyLC, Columbia, MD). Samples were dissolved in 1 mL of SCX loading buffer (25% v/v acetonitrile, 10mM PH₂PO₄, pH 2.8), pH was adjusted to 2.8 by adding 1 N phosphoric acid. The whole sample was loaded and washed isocratically for 30 minutes at 200µL/minute. Peptides were eluted with a gradient of 0-500mM KCl (25% v/v acetonitrile, 10 mM KH₂PO₄, pH 2.8) over 40 minutes at a flow rate of 200µL/minute. The absorbance at 214 nm was monitored and 32 SCX fractions were collected along the gradient.

Each SCX fraction was dried down and dissolved in 0.1% formic acid. The resulting fractions were analyzed on Qstar Pulsar™ (Applied Biosystems-MDS Sciex) interfaced with an Eksigent nano-LC system. Peptides were separated on a reverse-phase column packed with 10 cm of C18 beads (360x75 µm, 5 µm, 120 Å, YMC ODS-AQ, waters, Milford, MA) with an emitter tip (New Objective, Woburn, MA) attached. The HPLC gradient was 5-40% B for 60 minute (A, 0.1% formic acid; B, 90% acetonitrile in 0.1% formic acid) and the flow rate was 300nL/minute. Survey scans were acquired from m/z 350-1200 with up to three precursors selected for MS/MS using a dynamic exclusion of 45 seconds. Rolling collision energy was used to promote fragmentation and the collision energy range was ∼20% higher than that used for unlabeled peptides due to iTRAQ™ tags.

The MS/MS spectra were extracted and searched against the SwissProt database using Protein Pilots™ software (Applied Biosystems) with Paragon™ method and the following parameters: all species or rat as species, trypsin as enzyme (one missed cleavage allowed), cysteine static modification with methylmethanethiosulfate and iTRAQ™ (peptide labeled at N-terminal and Lysine) as sample type. The raw peptide identification results from the Paragon™ Algorithm (Applied Biosystems) searches were further processed by the Pro Group™ Algorithm (Applied Biosystems) within the ProteinPilot™ software suit before they were displayed. The Pro Group™ Algorithm uses the peptide identification results to determine the minimal set of proteins that can be reported for a given protein confidence threshold. The protein identification confidence threshold cutoff for this study was at least 95% confidence and at least one peptide identified with confidence. The protein abundance ratios were calculated using two set of iTRAQ™ data (see Table 1). Table 1 shows the differential expression of protein in old versus young rats. Column one is the protein classification, column two is the common protein name, column three is gene name (using the SwissProt convention), column four is the level of differential expression of the listed proteins as determined from iTRAQ™ analysis, column five is the level of differential expression of the listed proteins as determined from DIGE analysis, column six is a site of possible post translational modification, column seven is an exemplary accession number of the listed protein (Homo sapiens) and column eight is an exemplary accession number of the listed protein (Rattus norvegicus), the sequence of each of which is as of January 19,2009.

**Table 1: Differentially abundant proteins identified by 2-D DIGE and iTRAQ**

| **Protein class** | **Protein name** | **Gene (1)** | **iTRAQ Old versus Young (2)** | **DIGE Old versus Young (3)** | **Post-translation modification (4)** | **Exemplary Accession Nos, (Homo sapiens)** | **Exemplary Accession Nos, (*Rattus norvegicus*)** |
|---|---|---|---|---|---|---|---|
| Apoptosis/cell | MFG-E8 | MFGM | 2.31±0.21 | 2.53±0.15 | Glycosylation | NP_005919 | NP_036943 |
| cycle/proliferation | | | | | Potential N-linked glycosylation at 61, 230, 280, 390 | NP_001108086 | |
| Apoptosis/cell | Serine protease | HTRA1 | 2.03±0.17 | ND | | NP_002766 | NP_062510 |
| cycle/proliferation | HTRA1 | | | | | | NP_113909 |
| Apoptosis/cell | Integral membrane protein 2B | ITM2B | 2.28±0.63 | ND | | NP_068839 | NP_00100696 |
| cycle/proliferation | | | | | | | 4 |
| Apoptosis/cell cycle/proliferation | Cysteine and glycine-rich protein 2 or Smooth muscle cell LIM protein | CS RP2 | 1.56±0.08 | ND | | NP_001312 | NP_803174 |
| Apoptosis/cell cycle/proliferation | Platelet factor 4 (CXCL4) | PLF4 | 3.09±1.5 | ND | | NP_002610 | NP_00100773 0 |
| Apoptosis/cell cycle/proliferation | Insulin-like growth factor binding protein 7 (IGFBP-7) | IBP7 | 1.40±0.2 | ND | | NP 001544 | N/A |
| Cell Metabolism | Creatine kinase M-type | KCRM | 0.58±0.02 | ND | | NP_001815 | NP_036662 |
| Cell Metabolism | Glycerol 3-P-dehydrogenase 1 | GPDA | 0.58±0.10 | 0.52±0.08 | | NP_005267 | NP_071551 |
| Cell Metabolism | Cytochrome c oxidase subunit 5B | COX5B | 0.66±0.02 | ND | | NP_001853 | NP_446038 |
| Cell Metabolism | Glutamine synthetase | GLNA | 0.58±0.10 | | | NP_002056 | NP_058769 |
| | | | | | | NP_001028216 | |
| | | | | | | NP_001028228 | |
| Cell Metabolism; lipid metabolism /citric acid cycle | 2-oxoglutarate dehydrogenase E1 component | ODO1 | 0.67±0.11 | ND | | NP_001003941 | NP_00101746 |
| | | | | | | NP_002532 | 1 |
| Cell Metabolism; Lipid metabolism /Fatty acid beta-oxidation | 2,4-dienoyl-CoA reductase | DECR | 0.63±0.06 | ND | | NP_001350 | NP_476545 |
| Cell Metabolism; Lipid metabolism /Fatty acid beta-oxidation | 3-ketoacyl-CoA thiolase | THIM | 0.66±0.07 | 0.60±0.07 | | NP_006102 | NP_569117 |
| Cell Metabolism; Lipid metabolism /Fatty acid beta-oxidation | Hydroxyacyl-coenzyme A dehydrogenase | HCDH | 0.61±0.12 | ND | | NP_005318 | NP_476534 |
| Cell Metabolism; Lipid metabolism /Fatty acid beta-oxidation | Trifunctional enzyme subunit beta | ECHA | 0.66±0.07 | ND | | NP_000173 | NP_570839 |
| Cell Metabolism; Lipid metabolism /Fatty acid beta-oxidation | Long-chain-fatty-acid-CoA ligase 1 | ACSL1 | 0.63±0.12 | 0.62±0.07 | | NP_001986 | NP_036952 |
| Cell Metabolism; lipid metabolism /citric acid cycle | Isocitrate dehydrogenase [NADP] | IDHP | 0.75±0.05 | 0.65±0.05 | | NP_002159 | NP_00101418 3 |
| Cell Metabolism: lipid metabolism /citric acid cycle | Pyruvate carboxylase | PYC | 0.93±0.02 | 0.56±0.07 | | NP_000911 | NP_036876 |
| | | | | | | NP_001035806 | |
| | | | | | | NP_071504 | |
| Cytoskeleton/ invasion/migration | RalA-binding protein 1 | RBP1 | 1.49±0.02 | ND | | NP_006779 | NP_114456 |
| Cytoskeleton/ invasion/migration | Actin-related protein 2/3 complex subunit 4 | ARPC4 | 1.54±0.07 | ND | | NP-005709 | N/A |
| Cytoskeleton/ invasion/ migration | STE20-like serine/threonin e-protein kinase | SLK | 0.59±0.03 | ND | | NP_055535 | NP_062222 |
| Cytoskeleton/ invasion/ migration | Calponin-1 | CNN1 | 1.42±0.06 | 1.69±0.06 | | NP_001290 | NP_113935 |
| Cytoskeleton/ invasion/ migration | Calponin-3 | CNN3 | 1.45±0.07 | 1.75±0.06 | Phosphorylation Probable (by similarity) at 244 | NP_001830 | NP_062232 |
| Extracellular matrix, cytoskeletal/ invasion/ migration/ adhesion | Transforming growth factor beta-3 | TGFB3 | 2.22±0.21 | ND | | NP_003230 | NP_037306 |
| Extracellular matrix/ cell adhesion | Clusterin | CLUS | 1.89±0.26 | ND | | NP_001822 | NP_444180 |
| | | | | | | NP_976084 | |
| Extracellular matrix/ cell adhesion | 72 kDa type IV collagenase (MMP-2) | MMP2 | 1.40±0.11 | ND | | NP_004521 | NP_112316 |
| Extracellular matrix/ cell adhesion | Biglycan | PGS1 | 1.56±0.06 | ND | | NP_001702 | NP_058783 |
| Extracellular matrix/ cell adhesion | Collagen alpha-1(I) chain precursor⁵ | CO1A1 | 0.39±0.11 | 0.35±0.05 | | NP_000079 | NP-445756 |
| Extracellular matrix/ cell adhesion | Collagen alpha-2(I) chain precursor⁵ | CO1A2 | 0.54±0.14 | 0.45±0.06 | | NP_000080 | NP_445808 |
| Extracellular matrix/ cell adhesion | Fibronectin 1 | FINC | 1.68±0.10 | 2.1±0.19 | | NP_002017 | NP_062016 |
| | | | | | | NP_473375 | |
| | | | | | | NP_997639 | |
| | | | | | | NP_997640 | |
| | | | | | | NP_997641 | |
| | | | | | | NP_997647 | |
| Extracellular matrix/ cell adhesion | SPARC-like protein 1 | SPRL1 | 1.47±0.14 | ND | | NP_001121782 | NP_037078 |
| | | | | | | NP_004675 | |
| Extracellular matrix/ cell adhesion | Vitronectin | VTNC, | 1.91±0.09 | ND | | NP_000629 | NP_035837 |
| Extracellular matrix/ cell adhesion | Apolipoprotein A-I | APOA1 | 0.68±0.13 | 0.60±0.04 | | NP_000030 | NP_036870 |
| Extracellular matrix/ cell adhension | Kininogen 1 | KNT1 | 1.67±0.22 | 1.86±0.19 | Glycosylation Not known | NP_001095886 | NP_036828 NP_000884 |
| Extracellular matrix/ cell adhension | Periostin (OSF-2) | POSTN | 2.43±0.13 | 2.46±0.19 | Glycosylation Predicted N-linked glycosylation at 601 | NP_001129407 | NP_00110202 0 |
| | | | | | | NP_001129408 | |
| | | | | | | NP_006466 | |
| Extracellular matrix/cell adhesion | Apolipoprotein E | APOE | 1.55±0.29 | ND | | NP_000032 | NP_620183 |
| Extracellular matrix/cell adhesion | Fetuin-A; α-2-HS-glycoprotein | FETUA | 0.61±0.20 | ND | | NP_001613 | NP_037030 |
| Extracellular matrix/cell adhesion | Fetuin-B | FETUB | 0.66±0.05 | ND | | NP_055190 | NP_445800 |
| Miscellaneous | Adenylate cyclase type 5 | ADCY5 | 1.54±0.08 | ND | | NP-899200 | NP-072122 |
| Miscellaneous | Major urinary protein | MUP | 0.46±0.04 | ND | | N/A | NP_671747 |
| Miscellaneous | Transthyretin | TTHY | 0.59±0.18 | 0.63±0.12 | | NP_000362 | NP_036813 |
| Miscellaneous | Alpha-1-antiproteinase or Alpha-1-antitrypsin | A1AT | 0.66±0.09 | 0.58±0.09 | | NP_000286 | NP_071964 |
| | | | | | | NP_001002235 | |
| | | | | | | NP_001002236 | |
| | | | | | | NP_001121172 | |
| | | | | | | NP_001121173 | |
| | | | | | | NP_001121174 | |
| | | | | | | NP_001121175 | |
| | | | | | | NP_001121176 | |
| | | | | | | NP_001121177 | |
| | | | | | | NP_001121178 | |
| | | | | | | NP_001121179 | |
| Miscellaneous: | Tripartite motif-containing protein 47 | TRIP47 | 1.97±0.09 | | | NP_258411 | NP_00110305 5 |
| Miscellaneous: inflammation | Alpha-1-inhibitor III | A1I3 | 0.67±0.14 | 0.60±0.08 | Glycosylation | N/A | NP_00103306 4 |
| Miscellaneous: inflammation/oxidati on | Ferritin light chain | FRIL1 | 1.56±0.12 | 1.91±0.09 | | NP_000137 | NP_071945 |
| Miscellaneous: inflammation/oxidati on | Carbonic anhydrase 3 | CAH3 | 0.63±0.23 | ND | | NP_005172 | NP_062165 |
| Miscellaneous: inflammation/oxidati on | Liver carboxylestera se 1 | EST2 | 0.49±0.02 | ND | | NP_003860 | NP_058700 |
| | | | | | | NP_932327 | |
| Miscellaneous: inflammation/oxidati on | Contrapsin-like protease inhibitor 1 | SPA3K | 0.66±0.05 | ND | | N/A | NP-036789 |
| Miscellaneous: inflammation/oxidati on | Contrapsin-like protease inhibitor 3 | SPA3L | 0.62±0.10 | ND | | N/A | N/A |
| Miscellaneous: inflammation/oxidati on | Solute carrier family 2: facilitated glucose transporter member 4 | GTR4 | 2.10±0.02 | ND | | NP-001033 | NP-036883 |

| | | | | | | | |
|---|---|---|---|---|---|---|---|
| 1. Accession is from SwissProt data base. 2. Changes in abundance with p value 0.05 and over 1.5 fold on 4 animals in each group using iTRAQ; a value >1 in corresponding protein is increased with aging while the value <1 increase, ND not detected by this method. 3. Changes in abundance with p value 0.05 and over 1.5 fold on 8 animals in each group using 2DE; a value >1 in corresponding protein is increased with aging while the value <1 increase, ND not detected by this method. 4. Potential PTM based on PNGase F and phospho-staiuing. | | | | | | | |

To identify potential roles of differentially abundant proteins during aging, proteins were grouped into various function classes based on Swiss-Prot protein function and gene ontology (Table 1). The proteins having different abundance play significant roles in a number of cellular pathways including apoptosis/cell cycle/proliferation, cytoskeleton/invasion/migration, extracellular matrix/cell adhesion, metabolism, etc (Table 1).

Ingenuity pathway analysis program (available on the World Wide Web at ingenuity.com) was used to analyze the pathway network of proteins whose abundance change in association with aging. The proteins with their abundance change and corresponding gene names were uploaded as an Excel® spreadsheet file into the Ingenuity software. Ingenuity software uses the data to navigate the Ingenuity pathways database and extract networks (or interactions) between the proteins. A score greater than two is typically considered as a valid network. Ingenuity pathway analysis shows that those proteins are elements of the proinflammatory cellular movement pathway (FIG. 2A). Since cellular movement is a salient feature of invasion/migration, two groups of proteins were focused on: the extracellar matrix-related proteins and cytoskeleton proteins, including MMP-2, biglycan, calponin 1, calponin3, fibronectin, SPARC-like protein 1 and vitronectin etc. The proteins form a network centered on angiotensinogen (AGT), a precursor of Ang II. Interestingly, MFG-E8, a protein that markedly increased between about 2.3-2.5-fold with aging, is shown to be directly linked to protein kinase AKT signaling and extracellular signal-regulated kinase 1/2 in this network(FIG. 2), which is known to play an essential role in cell movement and proinflammation, involving the interplay of the matrix molecules: collagen, FN, vitronectin and its receptor integrins and MMP-2 and the inflammatory molecules: angiotensinogen, MCP-1, TGF-beta, PDGF, and NF-kb. These molecules change with aging and are upregulated in age-associated arterial remodeling. MFG-E8 is a multi-functional glycoprotein originally found in milk and mammary epithelial cells. It is of interest because its integral fragment, known as medin, is a component of amyloid plaque deposition in aged human arteries. Specifically, links between MFG-E8 and these proinflammatory molecules, however, have not been established. MFG-E8 was focused on to determine how it specifically relates to this inflammatory cell invasion signaling axis.

### Example 3

### mRNA analysis, Immunolocalization and Posttranslational Modification Analysis of MFG-E8

This example demonstrates that the increased expression of MFG-E8 protein is conserved across mammalian species.

To confirm the discovery that MFG-E8 increases in older rat aorta by both 2DE and iTRAQ™, RT-PCR, 1 and 2DE Western blotting and immunostaining were performed. Total RNA was purified from rat vascular smooth muscle cells or rat aortae with a Rneasy® Mini Kit (Qiagen®, Valencia, CA). The RNA concentration was determined with ND-1000 spectrophotometer (Nanodrop technologies, Wilmington, DE). From the RNA, cDNA was generated with TaqMan® Reverse Transcription Reagents (Applied Biosystems, CA). Quantitative RT-PCR (qRT-PCR) was carried out with Platinum SYBR® Green qPCR SuperMix-UDG with ROX (Invitrogen®). The sequences of the primers used for qRT-PCR were CTGGACAATCAGGGCAAGAT (SEQ ID NO: 3), which is derived from MFG-E8 exon8, and GTCCACTGCACAACCATCATC (SEQ ID NO: 4), which is derived from MFG-E8 exon9. Each sample has been tested in quadruplicate. The reaction conditions were: 10 min at 95°C (one cycle); 30 sec at 95°C; 30 sec at 60°C; and 30 sec at 72°C (40 cycles). Gene-specific PCR products were continuously measured by an ABI PRISM 7900 HT Sequence Detection System (PE Applied Biosystem Norwalk, CT). The PCR product sizes were verified by agarose gel electrophoresis. Samples were normalized to the expression of the "housekeeping" gene, the 18S rRNA, which did not change with age. Data are expressed using the formula: quantity=10-(Ct-Y intercept)/slope value) where Ct represents the threshold cycle value. For Western Blotting, fifteen µg of whole cell lysates were resolved by SDS-PAGE and transferred onto PVDF membrane (Immobilon). The transferred membranes were incubated in PBS containing primary antibodies: MFG-E8 and MCP-1 at 4°C for 24 hours. HRP-conjugated IgG (Amersham Pharmacia Biotech, Buckinghamshire, GB) were used as secondary antibodies and detected with SuperSignal® West Pico Chemiluminescent Substrate (Pierce Biotechnology, Rockford, IL). The average densitometric analysis of the bands was obtained on the protein extracts from the aortae of four rats of each age group or from VSMC at least three independent experiments. Beta-actin immunoblotting has been used as a protein loading control since its expression does not change with age.

qRT-PCR showed that the MFG-E8 transcriptome increased 2.3-fold in older compared to younger rat aortae (FIG. 3A, p<0.05). 1D Western blot analysis and 2DE (FIG. 3B) confirmed the increased abundance of MFG-E8 in older versus younger aortae.

Immunofluorescence staining demonstrated that the intensity of MFG-E8 increased within the old compared to the young aortic wall, and that MFG-E8 staining colocalizes with that of α-SMA, a marker of VSMC (FIG. 3C). Immunostaining for MFG-8, MCP-1, and α-SMA was performed according to the protocols provided by the manufacturer (Dako Corp).

In addition to changes in the abundance of MFG-E8 with aging, three arterial MFG-E8 spots with similar molecular weight (MW) were observed in the 2DE in the sample from old aortae (FIG. 3B). Treatment of the tissue samples with PNGase F, which specifically removes N-linked carbohydrates, shifts the triplicate spots to a lower MW, as anticipated for PTM by deglycosylation (FIG. 3D). However, PNGase F treatment did not shift to the same pI, indicating the presence of a second unknown PTM (FIG. 3D).

In an attempt to determine whether the age-associated increase in MFG-E8 expression is conserved across various mammalian species, aortic tissues from nonhuman primates and humans were utilized for Western blotting and immunostaining studies. In nonhuman primates, Western blot analysis showed that the abundance of aortic MFG-E8 increased approximately 9 fold with aging (FIG. 4A). Immunolabelling analysis indicated that MFG-E8 staining increased in both the intima and the media aortic wall in older versus younger monkeys (FIG. 4B).

Similarly, in humans, Western blot analysis and dual immunofluorescence staining of MFG-E8 indicated an age-associated increase of approximately 6.5-fold (FIGs. 4C and D), predominantly located in the inner media and intimae, and also colocalized with α-SMA, as in the rat and monkey (FIGs. 3C & 4B).

For Western blotting, fifteen µg of whole cell lysates were resolved by SDS-PAGE and transferred onto PVDF membrane (Immobilon). The transferred membranes were incubated in PBS containing primary antibodies at 4 °C for 24 hours. HRP-conjugated IgG (Amersham Pharmacia Biotech, Buckinghamshire, GB) were used as secondary antibodies and detected with SuperSignal® West Pico Chemiluminescent Substrate (Pierce Biotechnology, Rockford, IL). The average densitometric analysis of the bands was obtained on the protein extracts from the aortae of four rats of each age group in at least three independent trials. Beta-actin immunoblotting was used as a protein loading control since its expression does not change with age.

### Example 4

### Exogenous Administration of Angiotensin II to Young VSMCs Mimics Aging by Elevating MFG-E8 Production

This example demonstrates that MFG-E8 production is activated by Angiotensin.

Bioinformatic functional analysis indicates that MFG-E8 is potentially involved in the biosignaling network of cell movement and proinflammation (FIG. 2), but fails to place it specifically downstream of the Ang II (an AGT cleavage product) signaling cascade, a central feature of arterial aging. Of note, levels of the AGT protein signal determined by immunofluorescence were increased in the aged arterial wall. Dual immunofluorescence studies demonstrated that both Ang II and MFG-E8 expression within VSMC are closely associated and that both markedly increased within the rat aortic wall, particularly in the thickened aged intima (. 5A).

Early passage VSMCs were used to detect a potential functional link between Ang II and MFG-E8. VSMCs were enzymatically isolated. Briefly, F344XBN rat thoracic aortae were rinsed in Hanks balanced salt solution (HBSS) containing 50 µg/mL penicillin, 50 µg/mL streptomycin and 0.25 µg/mL amphotericin B (Gibco). After digestion for 30 minutes in 2 mg/mL collagenase I solution (Worthington Biomedical, Freehold, New Jersey) at 37 °C, the adventitia and intima were removed from the vessel media layer, which was placed overnight in complete medium (DMEM plus 10% FCS). On day 2 the vascular media was further digested with 2mg/mL collagenase II/0.5mg/mL elastase (Sigma) for 1 hour at 37 °C, and the isolated cells were washed and plated in complete medium. In all cases, >95% of cells stained positive for α-smooth muscle actin (α -SMA). Early passage (p3-p5) VSMC were cultured and treated with Ang II, MFG-E8 with and without vCCI for 24 or 48 hours, in 2.5% fetal bovine serum (FBS) Dulbecco's Modified Eagle Medium (DMEM).

Both unglycosylated (lower MFG-E8 bands) and glycosylated forms (upper MFG-E8 bands) of MFG-E8 protein (FIG. 5B, top panel) were detected, and both increased with age in early passage VSMC (FIG. 5B, bottom panel). Treatment of young VSMC with Ang II induced MFG-E8 production, predominantly in the native form (unglycosylated) in a dose-dependent manner, up to the levels of old untreated VSMC (FIG. 5B, bottom panel). Elevated BFG-E8, including glycosylated and unglycosylated forms, in old cells is significantly reduced (approximately 40%) by treatment with [Sar1, Gly8]-Ang II acetate hydrate, an Ang II type 1 receptor blocker. This novel finding indicates that MFG-E8 is downstream of Ang II initiated signaling.

For Western blotting, fifteen µg of whole cell lysates were resolved by SDS-PAGE and transferred onto PVDF membrane (Immobilon). The transferred membranes were incubated in PBS containing primary antibodies at 4 °C for 24 hours. HRP-conjugated IgG (Amersham Pharmacia Biotech, Buckinghamshire, GB) were used as secondary antibodies and detected with SuperSignal® West Pico Chemiluminescent Substrate (Pierce Biotechnology, Rockford, IL). The average densitometric analysis of the bands was obtained on the protein extracts from VSMCs in at least three independent trials. Beta-actin immunoblotting was used as a protein loading control since its expression does not change with age.

### Example 5

### MFG-E8 Stimulates Functional MCP-1 Production in Aging VSMC and Consequently Enhanced Invasion

This example demonstrates that MCP-1 is downstream of MFG-E8.

Prior studies indicate that chronic infusion of Ang II to young rats increases MCP-1 expression; and the acute exposure of early passage VSMC from young rat aortae potentiates their invasive capacity, in part, *via* this enhanced MCP-1 expression.

It was determined whether there was a relationship between MFG-E8 and MCP-1. Dual immunostaining of aortae from old rats demonstrated that MFG-E8 colocalizes with MCP-1, preferentially in the thickened intima (FIG. 6A) and immunostaining of old VSMCs indicates that MFG-E8 also co-localizes with MCP-1, preferentially in the perinuclear region (FIG. 6B). MFG-E8 treatment of early passage VSMC increased the functional dimer of MCP-1 product (FIG. 6C). Conversely, MFG-E8 silencing (>70%) substantially reduces MCP-1 expression and its dimerization within VSMCs but does not affect MCP-1 mRNA levels. Furthermore, the Ang II-induced increase in MCP-1 expression is markedly inhibited by MFG-E8 silencing. The results shown in FIGs. 5 and 6 demonstrate that Ang II is upstream of MFG-E8, and that MCP-1 is downstream of MFG-E8 within the Ang II signaling cascade that potentiates VSMC invasion. Based on the information provided herein, new interactions can be added between Ang II, MCP-1 and MFG-E8 (FIG. 2B).

Next, it was determined whether MFG-E8 is required for enhanced VSMC invasion, and whether it acts in this respect via MCP-1. Modified Boyden chamber analysis in FIG. 7A (left panel) showed that, in the control, VSMC invasive capability significantly increased in old cells compared to young. The VSMC migration assays were performed using modified Boyden chambers were equipped with 8µm pore-size polycarbonate filters (Neuroprobe, Gaithersburg, MD) coated with Matrigel® (BD Bioscience, Palo Alto, CA). VSMCs were untreated or pretreated with MFG-E8 only or together with vCCI for 24 hours. Two hundred and twenty µL of migration medium (MM; DMEM with 0.1% BSA) was added to the lower chamber with or without platelet-derived growth factor (PDGF) for the chemo attraction and random migration assay, respectively. SMCs (10⁶/mL) were placed in the upper chamber in 200 µL of MM. The assay was stopped after 4 hours at 37 °C, and the cells that had crossed the basement membrane and migrated to the lower side of the filter were fixed and stained using the HEMA3 system (Curtin, Matheson Scientific, Inc., Houston, TX). Four random fields were counted at 400X magnification for each filter. MFG-E8 siRNA markedly knocked down MFG-E8 protein expression in both young and old VSMC (FIG. 7A, right panel), and reduced invasive capability of both young and old VSMC, eliminating age differences in invasive capacity that were present in the control (FIG. 7A, left panel). Exposure of VSMCs to Ang II increased the invasive capability of VSMCs in an age-dependent fashion and this increase was substantially reduced by MFG-E8 silencing (FIG. 7B). The addition of exogenous MFG-E8 also increased VSMC invasion capability in an age-dependent fashion (FIG. 7C), and these effects were substantially reduced by an MCP-1 receptor blocker, vCCI (FIG. 7C).

As disclosed herein MFG-E8 is increased within aged VSMC and is a key downstream molecule of Ang II signaling within VSMC. Other and current studies show that the cellular replicative capacity is enhanced in both aged and Ang II treated young VSMC. MFG-E8 treatment increases the proliferative capacity in both young and old VSMC in a dose- and time-dependent manner. MFG-E8 has physical interaction with integrin and other studies have implicated that the integrin receptor is a potential relay for MFG-E8 signaling. MFG-E8 related proliferative effects in both young and old VSMC can be extinguished by treatment with neutralized antibodies against avb3 and avb5.

The age-associated VSMC population composition within the arterial wall was determined by a dynamic balance between cell addition and subtraction. To explore the MFG-E8 role as a homeostatic factor in this process, MFG-E8 treatment of early passage VSMC isolated from young and old rat aorta was performed. VSMC proteomic data indicate that exposure of VSMC to MFG-E8 down-regulates cell cycle inhibitory molecules, i.e., insulin growth factor binding protein-7, while up-regulating cell cycle promoter molecules, including CD 151 antigen, DNA-binding protein A, scaffolding attachment factor B, myotrophin, cyclin-dependent kinase-4 (CDK4), and MAP kinase 1, creating a niche for VSMC proliferation. Furthermore, recombinant human MFG-E8 treatment or over-expression of the full-length MFG-E8 via adenovirus infection elevates levels of VSMC cell cycle accelerators, p-ERK1/2, and CDK4, proliferating cellular nuclear antigen (PCNA), and enhances BrdU incorporation. These effects are substantially reduced by neutralizing antibodies against αvβ3 or αvβ5, the cellular integrin receptors of MFG-E8. Conversely, siRNA knock-down of MFG-E8 reduces levels of PCNA and CDK4 expression and raises levels of cell cycle decelerators ATM, p53, and p21, and enhances senescence-associated β-gal activity indicating cell entry into a growth arrested state. Thus, MFG-E8 signaling not only enhances VSMC invasion capacity but also promotes VSMC proliferation via coordination of diverse effects on the activity of molecules relevant to the cell cycle.

**Table 2. Proteins having different abundance between young and old smooth muscle cells and proteins affected by MFG-E8 treatment in young smooth muscle cells.**

| **Protein name** | **Accession** | **Ratio** | | |
|---|---|---|---|---|
| | | **Old/Young** | **Treated /untreated** | |
| | | | **(10ng/ml)** | **(100ng/ml)** |
| Interleukin-1 receptor antagonist protein | IL1RA_RAT | 3.72 | 1.35 | 1.22 |
| Cell division protein kinase 4 | CDK4_RAT | 3 | 1.56 | 2.57 |
| High mobility group protein B1 | HMGB1_RAT | 2.25 | 0.91 | 0.92 |
| Protein SET | SET_RAT | 2.23 | 1.43 | 1.02 |
| Decorin | PGS2_RAT | 2.22 | 1.22 | 1.42 |
| Microfibril-associated glycoprotein 4 | MFAP4_MOUSE | 2 | 1.2 | 1.1 |
| Mimecan | MIME_MOUSE | 2 | 1.08 | 1.11 |
| Acyl-CoA-binding protein | ACBP_RAT | 1.95 | 0.95 | 0.98 |
| Synaptopodin | SYNPO_RAT | 1.93 | 1.04 | 0.98 |
| Thrombospondin-1 | TSP1_MOUSE | 1.91 | 1.1 | 1.07 |
| Protein S100-A4 | S10A4_RAT | 1.9 | 1.03 | 0.45 |
| Eukaryotic translation initiation factor 1A | IF1A_RAT | 1.82 | 0.98 | 0.97 |
| Collagen alpha-2(I) chain | CO1A2_RAT | 1.77 | 1.12 | 1.04 |
| Collagen alpha-1(I) chain | CO1A1_RAT | 1.75 | 0.96 | 1.1 |
| Serum deprivation-response protein | SDPR_RA | 1.75 | 0.83 | 0.81 |
| Eukaryotic initiation factor 4A-II | IF4A2_RAT | 1.69 | 1.14 | 0.83 |
| Protein S100-A6 | S10A6_RAT | 1.67 | 0.92 | 0.72 |
| Fibronectin | FINC_RAT | 1.67 | 1.08 | 1.05 |
| Hematological and neurological expressed 1-like protein | HN1L_RAT | 1.65 | 1.54 | 1.23 |
| 60S ribosomal protein L29 | RL29_RAT | 1.64 | 1.57 | 1.49 |
| Large neutral amino acids transporter small subunit 1 | LAT1_RAT | 1.62 | 2.1 | 1.49 |
| Protein S100 A11 | S10AB_RAT | 1.61 | 1.64 | 1.63 |
| 60S ribosomal protein L14 | RL14_RAT | 1.6 | 1.1 | 1.03 |
| Collagen alpha-1(III) chain | CO3A1_RAT | 1.58 | 1.1 | 1.14 |
| 60S acidic ribosomal protein P2 | RLA2_RAT | 1.58 | 1.04 | 0.61 |
| Caveolin-1 | CAV1_RAT | 1.55 | 0.85 | 1 |
| Succinyl-CoA ligase [GDP-forming] subunit alpha | SUCA_RAT | 0.74 | 0.7 | 1 |
| Microtubule-associated proteins 1A/1B light chain 3B | MAP1A_RAT | 1.5 | 0.96 | 1.05 |
| Gremlin-1 | GREM1_RAT | 1.5 | 1.06 | 1.41 |
| Myotrophin | MTPN_RAT | 1.46 | 1.85 | 1.31 |
| Polymerase I and transcript release factor | PTRF_MOUSE | 1.45 | 1.1 | 1 |
| Insulin-like growth factor-binding protein 7 | IBP7_MOUSE | 1.4 | 0.5 | 1.2 |
| Double-strand break repair protein MRE11A | MRE11_RAT | 1.4 | 1.17 | 1.14 |
| Scaffold attachment factor B | SAFB1_RAT | 1.38 | 1.83 | 1.31 |
| DNA-binding protein A | DBPA_RAT | 1.36 | 1.45 | 1.46 |
| Protein ADRM1 | ADRM1_RAT | 1.25 | 1.59 | 1.92 |
| Myeloid-associated differentiation marker | MYADM_RAT | 1.17 | 1.06 | 1.32 |
| Myristoylated alanine-rich C-kinase substrate) | MARCS_RAT | 1.01 | 1.1 | 0.36 |
| Serine incorporator 1 | SERC1_MOUSE | 1.04 | 1.59 | 1.2 |
| Serine/threonine-protein kinase WNK1 | WNK1_MOUSE | 1.02 | 2.12 | 1.06 |
| Nuclear factor NF-kappa-B p100 subunit | NFKB2_MOUSE | 0.92 | 0.83 | 0.61 |
| Integrin alpha 5 | ITA5_MOUSE | 0.91 | 0.52 | 0.56 |
| CD151 antigen | CD151_RAT | 0.67 | 1.49 | 1.01 |
| Plasminogen activator inhibitor 1 | PAI1_RAT | 0.65 | 1.01 | 1.01 |
| Protein NDRG 1 | NDRG1_RAT | 0.65 | 1.05 | 1.15 |
| Growth factor receptor-bound protein 14 | | 0.63 | | |
| Serine protease HTRA1 | HTRA1_HUMAN | 0.63 | 0.97 | 1.04 |
| Alcohol dehydrogenase 1 | ADH1_MOUSE | 0.63 | 0.98 | 0.94 |
| Glutathione S-transferase Mu 2 | GSTM2_RAT | 0.61 | 0/87 | 1 |
| Tropomyosin beta chain | TPM2_RAT | 0.6 | 0.82 | 1.07 |
| Selenium-binding protein 1 | SBP1_RAT | 0.6 | 1.1 | 1.02 |
| Superoxide dismutase [Mn | SODM_RAT | 0.59 | 0.98 | 0.98 |
| Osteopontin | OSTP_RAT | 0.54 | 0.76 | 1.02 |
| Ferritin light chain 1 | FRIL1_RAT | 0.5 | 0.92 | 0.93 |
| Midline-1 (Tripartite motif-containing protein 18) | TRI18_RAT | 0.5 | 0.87 | 0.9 |
| Alpha-2-HS-glycoprotein | FETUA_RAT | 0.28 | 0.93 | 1.25 |

It is known that MAP kinase, ERK1/2, is a key signaling molecule orchestrating the cell cycle. Age significantly increases ERK phosphorylation within VSMC. Interestingly, MFG-E8 treatment markedly increased this phosphorylation in both young and old cells in a similar manner. Moreover, age also increased proliferating cell nuclear antigen (PCNA) protein abundance within old compared as young cells. Additionally treatment with MFG-E8 increased PCNA protein expression in both young and old cells.

Provided herein is a proteome profile of arterial aging in FXBN rats, a well-characterized rodent model with similarities to the human anatomic and physiological phenotype. Using a combination of 2DE and iTRAQ, 923 proteins were identified and characterized within the aortic wall, of which 50 significantly change abundance with aging, some of which also have post-translational modifications, such as glycosylation and phosphorylation. The proteins with significant abundance changes play significant roles in a number of cellular pathways including cellular movement, cell proliferation and apoptosis, energy metabolism, etc, each of which are involved in vascular remodeling.

The data provided herein establish molecular links among Ang II, MFG-E8, and MCP-1 in aged VSMCs. Interestingly, MFG-E8 stood out in the proteomic analysis because it has not previously been linked directly with Ang II/MCP-1, VSMCs, or aging. The age-related increase in transcription and translation of MFG-E8 is preserved across species (rat, non-human primates, human), suggesting it may have a central role in arterial aging. Moreover, is was surprisingly shown that Ang II induces MFG-E8 in VSMCs isolated from rat aortae, that MFG-E8 is required for the Ang II to increase MCP-1, and that Ang II and MCP-1 co-localize with MFG-E8 in the arterial wall. In addition, the treatment of isolated VSMCs with MFG-E8 induces the biologically active dimer of MCP-1, which is a potent chemoattractant of smooth muscle cells. Furthermore, MFG-E8 enhanced the Ang II-induced invasion potential of isolated VSMCs. Thus, MFG_E8 is a novel link between Ang II/MCP-1 signaling and VSMCs invasive capacity within the aortic wall.

Thus, provided herein is a comprehensive nonbiased proteomic approach to investigate the biology of age-associated thickening of the aorta which has identified a novel Ang II/MFG-E8/MCP-1 signaling axis that links the cellular movement signaling cascades with the age-associated increase in smooth muscle cell invasive capability.

### Example 6

### Role of MFG-E8 in VSMC Proliferation

This example demonstrates that suppression of MFG-E8 expression reduces the proliferative ability of VSMCs and, conversely, that MFG-E8 enhances proliferation of VSMCs.

The invasion and proliferation of VSMC are salient features of arterial remodeling with aging. MFG-E8 and Ang II, proteins shown to be elevated in the aging aorta, enhance the invasive capacity of arterial VSMC. Given that invasion and proliferation share some common mechanisms, the role of MFG-E8 in increased proliferation of arterial VSMC was investigated.

### Alterations in Arterial VSMC proliferation associated with the Ang II/MFG-E8 signaling axis

8-month (young, n=50) and 30-month (old, n=30) Fisher 344 X Brown Norway (F344XBN) male rats were obtained from the National Institute on Aging contract colonies (Harlan Sprague-Dawley, Indianapolis). Twenty young rats were randomly allocated into 2 groups, and implanted subcutaneously with osmotic minipumps (Model 2004, Alzet Corp) in the midscapular region, according to methods described previously. Briefly, rats were infused with Ang II at a rate of 200ng/kg/min (Ang II infusion, n=10) and rats infused with saline served as untreated young controls (control, n=10). All these rats were also implanted with a second osmotic pump (Model 2004, Alzet Corp) that delivered a continuous infusion of the non-radioactive thymidine analogue BrdU (0.8 mg/kg/day), which is incorporated into DNA during *replication in vivo.* The animal protocol was approved by the Institutional Animal Care and Use Committee and complied with the guide for the care and use of laboratory animals (National Institutes of Health publication No. 3040-2, revised 1999). All of the animals were sacrificed by an overdose of sodium pentobarbital, and thoracic aortae were immediately removed, isolated, and processed as described previously.

VSMC were enzymatically isolated as previously described. Briefly, F344XBN rat thoracic aortae were rinsed in Hanks balanced salt solution (HBSS) containing 50 µg/mL penicillin, 50 µg/mL streptomycin and 0.25µg/mL amphotericin B (Gibco). After digestion for 30 minutes in 2mg/mL collagenase I solution (Worthington Biomedical, Freehold, New Jersey) at 37°C, the adventitia and intima were removed from the vessel media layer, which was placed overnight in complete medium (DMEM plus 10% FCS). On day 2 the vascular media was further digested with 2mg/mL collagenase II/0.5mg/mL elastase (Sigma) for 1 hour at 37°C, and the isolated cells were washed and plated in complete medium. In all cases, >95% of cells stained positive for α-SMA. Early passage (p3-p5) VSMC were cultured with or without MFG-E8, and neutralizing antibodies against αvβ3 and αvβ5, and were lysed for Western blot analysis.

For Western Blotting, fifteen µg of whole cell lysates were resolved by SDS-PAGE and transferred onto PVDF membrane (Immobilon). The transferred membranes were incubated in PBS containing primary antibodies (Table 3) at 4°C for 24 hours. HRP-conjugated IgG (Amersham Pharmacia Biotech, Buckinghamshire, GB) were used as secondary antibodies and detected with SuperSignal® West Pico Chemiluminescent Substrate (Pierce Biotechnology, Rockford, IL). The average densitometric analysis of the bands was obtained on the protein extracts from the aortae of four rats of each age group or from VSMC at least three independent experiments. Beta-actin immunoblotting has been used as a protein loading control since its expression does not change with age.

Immunostaining was performed according to the protocols provided by the manufacturer (Dako Corp, CA). The negative control was stained with serum (no primary antibody). The source and characteristics of primary antibodies used are listed **Table 3.**

**Table 3: Primary Antibodies**

| **Antibody** | **Species** | **Titer Blotting** | **Titer Staining** | **Sources** |
|---|---|---|---|---|
| MFG-E8 | M | 1:1000 | 1:50 | R&D System Inc. MN |
| P21 | M | 1:1000 | 1:50 | Zymed Inc. CA |
| P21 | R | 1:1000 | 1:50 | Santa Cruz, CA |
| PCNA | M | 1:1000 | 1:50 | Dako Corp., Denmark |
| P53 | M | 1:500 | 1:50 | Santa Cruz, CA |
| CDK4 | M | 1:200 | 1:50 | Chemicon Intern. Inc.. CA |
| α-SMA | M | 1:2000 | 1:50 | Dako Corp., Denmark |
| CHK2 | M | 1:100 | 1:100 | Santa Cruz, CA |
| ATM | R | 1:500 | 1:50 | Santa Cruz, CA |
| α v β 3 | M | 1:1000 | 1:50 | COVANCE, CA |
| α v β 5 | M | 1:1000 | 1:1000 | Chemicon Intern. Inc., CA |
| α v | M | 1:600 | 1:100 | Chemicon Intern. Inc., CA |
| β5 | R | 1:100 | 1:1000 | Chemicon Intern. Inc., CA |
| β3 | R | 1:100 | 1:50 | Chemicon Intern. Inc., CA |
| β-actin | M | 1:10000 | 1:50 | Sigma-Aldrich, Inc. MO |

| | | | | |
|---|---|---|---|---|
| R=rabbit; and M=mouse. | | | | |

Western blot analysis shows that the protein abundance of proliferating cell nuclear antigen (PCNA) (FIG. 9A), a DNA polymerase delta and an index of cellular proliferation, increases ∼2-fold in the old versus young rat aorta. Furthermore, immunostaining demonstrates the number of PCNA stained VSMC (brown). This suggests that the number, location and proliferative capability of VSMC within the arterial wall are altered with aging (FIG. 9B). Furthermore, the long and short forms of MFG-E8 (L-MFG-E8, S-MFG-E8), which can exist as either glycated or unglycated forms within the rat aortic wall, have been detected and both significantly increase in old arterial VSMC, the glycated forms in particular (FIG. 9C).

Prior studies show that a chronic administration of Ang II to young rats remodels the structure of arterial walls, resembling those of untreated old animals and also is an ideal *in vivo* model for arterial proliferation. Western blotting indicates that the protein abundance of PCNA is markedly increased in Ang II infused young rats (FIG. 9D), similar to that of old rats (FIG. 9A). Moreover, the BrdU incorporation index, a parameter for the detection of proliferating cells in living tissues or cells, is dramatically increased, predominantly in the thickened intima and the innermost media of aorta from young rats infused with Ang II versus control (FIG. 9E). Interestingly, immunohistostaining shows that the MFG-E8 signal (brown color) is markedly increased within Ang II infused arterial wall, in particular, in the intima and innermost media (FIG. 9F, upper panels). Western blotting analysis further confirms, that both L-MFG-E8 and S-MFG-E8, in particular the glycated forms, significantly increased in arterial walls of young rats infused with Ang II, similar to aging (FIG. 9F, middle and lower panels).

### Expression of MFG-E8 and integrin receptors in VSMC

Since the integrin (αvβ3 and αvβ5) receptor has been shown to have a high affinity for binding MFG-E8 and also is engaged with MFG-E8 during angiogenesis, breast tumor growth metastasis, and wound healing, it was determined whether this is the case in VSMC. Indeed, both immunohistochemistry (FIGs 10A and 10B) and Western blotting (FIG. 10C) demonstrate that both αvβ3 and αvβ5 are also co-expressed and are markedly increased within the old versus young aortic wall. αvβ3 and αvβ5 immunolabelling are colocalized with MFG-E8 (FIGs. 10A and 10B). Higher magnification of the medial aortic wall demonstrates that both αvβ3 and αvβ5 are confined to VSMC (FIG. 10D). In addition, arterial integrin αvβ3 and αvβ5 expression are up-regulated in the arterial wall from rats treated with Ang II.

### MFG-E8 Promotes Proliferation of VSMC via Integrin Signaling

VSMC proliferation was assessed by methylthiazoletetrazolium (MTT) assay: approximately 3000 young and 1000 old VSMCs were plated sparsely in 96-well flat-bottomed microplates in 100 mL of DMEM + 2.5% FBS. After 24 hours at subconfluency, the medium was replaced with MFG-E8 (0, 10, or 50 ng/ml) with or without neutralizing antibodies, and the cells were incubated at 37°C in a humidified, 5% CO₂ atmosphere for 0, 3, or 5 days with routine medium changes. At the end of the incubation, 25 mL of 3-[4,5-dimethylthiozol-2-yl]-2,5-diphenyltetrazolium bromide (MTT) solution (5 mg/mL) were added to each well, followed by a 4-hour incubation at 37°C. Then the medium carefully was removed and 150 mL of dimethylsulfoxide (DMSO) were added to dissolve the produced formazan crystals. The optical density immediately was measured at 570 nm using a microplate reader (Bio-Tek Instrument).

BrdU (bromodeoxyuridine) incorporation assay: young and old VSMCs were cultured as described previously. Cells were grown in 96-well plates for 48 hours and then cultured in serum-free medium overnight before stimulation with MFG-E8, Ang II, and PDGF for 24 hours. BrdU was added during the last 4 hours of the DNA synthesis phase and the binding of a monoclonal anti-BrdU antibody (Roche Diagnostics) was used to quantify the incorporation of BrdU into DNA. Experiments were performed in triplicate and results are shown as mean ± S.D.

MFG-E8 expression in VSMC increases in a broad spectrum of species, including rats, non-human primates, and humans with aging or infusion of Ang II. This study shows quantification of the age difference: old cells contain ∼1.4 fold greater MFG-E8 than young cells (. 11A). The colocalized immunolabelling of MFG-E8, αvβ3 and αvβ5, and α-SMA on VSMC (FIG. 10 and FIG. 11B) suggests that these integrins increase and may relay MFG-E8 signaling with aging. It was determined whether proliferative capacity of VSMC is enhanced by MFG-E8 signaling and whether this is influenced by αvβ3 and αvβ5 integrins. FIG. 11C indicates that exogenous treatment with recombinant human MFG-E8, increases proliferation determined by a methylthiazoletetrazolium (MTT) assay in both young and old VSMC in a dose- and time-dependent manner. Further, FIG. 11D indicates that MFG-E8 treatment, like Ang II, markedly increases BrdU incorporation in old versus young VSMC. FIG. 11B demonstrates that MFG-E8 related proliferative effects in both young and old VSMC is blocked by neutralizing antibodies against αvβ3 and αvβ5.

### Exogenous MFG-E8 triggers expression of proliferation-associated signaling molecules

It was next determined which intracellular mechanisms underlie MFG-E8 proliferation in arterial VSMC from young and old rat aorta. It is known that ERK1/2, an MAP kinase, is a key signaling molecule orchestrating the cell cycle, and the disruption of the cellular basement membrane are crucial events of cell proliferation *in vivo.* Interestingly, MFG-E8 treatment markedly increases ERK1/2 activation, assessed by its phosphorylation status, in both young and old VSMC in a dose-dependent manner (FIG. 12A). Western blotting analysis further confirmed this finding and also indicated that MFG-E8 induces phosphorylation of ERK-1/-2 in a time-dependent fashion (FIG. 12B). Furthermore, treatment of young VSMC with MFG-E8 increases PCNA protein abundance in a dose-dependent manner, up to levels of untreated old cells (FIG. 12C). The present study shows that a chronic exposure of young VSMC to MFG-E8 increased activation of MMP-2, a well-known degrader of the basement membrane, in a dose-dependent manner, reaching levels from old untreated cells (FIG. 12D), favoring VSMC hyperplasia.

### Overexpression of ectopic MFG-E8 drives the progression of the cell cycle, and enhances proinflammation and wound-healing capacity

To further prove effects of endogenous MFG-E8 on VSMC proliferation, an adenovirus harboring a full-length cDNA of the L-or S-MFG-8 forms was constructed. The human calpain-1 full-length cDNA fragment was obtained as described previously. An adenoviral vector expressing green fluorescent protein (GFP) served as a control virus. Standard viral amplification and cesium chloride purification methods were used to amplify and purify these adenoviruses. The titer for each adenovirus in HEK293A cells was determined via a dilution assay. Adenoviral infection of VSMC was performed with a multiplicity of infections (MOI) of 100. After forty-eight hours of adenoviral infection, total cellular proteins were isolated for Western blot or migration assays. Young and old VSMCs infected with adenovirus harboring full length of cDNA of L and S-form MFG-E8 or GFP were seeded on six-well plates. After reaching subconfluence, the cells were wounded with a sterile pipette tip to remove cells by two perpendicular linear scratches. The progress of healing process was photographed under phase contrast light microscopy immediately or 24 h after wounding.

Ectopic MFGE8s are substantially expressed in VSMC infected with the MFG-E8 adenovirus (FIG. 13A). Interestingly, overexpression of MFG-E8 markedly enhanced the phosphorylation of ERK-1/-2 (FIG. 13B), PCNA abundance (adenovirus (FIG. 13C), and dimerization of a powerful proinflammatory molecule, MCP-1, reaching levels of untreated old cells (FIG. 13D).

A scratch-wound repair assay of the VSMC monolayer, a well established *in vitro* model of a combination of cellular proliferation and migration, indicated that the VSMC healing capacity was significantly accelerated in young cells infected with an adenovirus harboring MFG-E8 cDNA, up to levels of untreated old cells (FIG. 13E).

### Silencing MFG-E8 affects withdrawal from the cell cycle

To further establish that endogenous MFG-E8 is a survival factor, MFG-E8 siRNA intervention of early passage VSMC was performed. MFG-E8 siRNA substantially reduced MFG-E8 expression in both younger and old VSMC. MFG-E8 silencing was carried out with the Stealth™ siRNA duplex oligoribonucleotides of rat MFG-E8 (Invitrogen®) using the Amaxa® nuclearfection techniques according to the manufacture's protocol (Lonza, Basel, Switzerland). The program used for the transfection was P-024. The cells transfected with Stealth RNAi Negative Control Duplexes (Invitrogen®) were used as a negative control. After 48 hours of transfection with siRNA, the VSMCs were analyzed for senescence by SA-beta-gal binding. The SA-beta-gal stained and no stained cells were observed and counted under a contrast phase microscope. Notably, MFG-E8 silencing increased the number of SA-beta-gal stained cells 4-fold in young and -4-fold in old cells. In addition, 48 hours of transfection with siRNA VSMCs were lysed with 1x Cell Lysis Buffer (Cell Signaling). Protein concentration was determined with BCA protein assay reagents (Pierce). Proteins were separated with a 4-12% NuPAGE® gel (Invitrogen®) and transferred to a PDVF membrane. After blocked with 5% milk, the membrane was incubated with a specific antibody overnight at 4 °C overnight. The corresponding HRP conjugated secondary antibody was incubated with the membrane the second day. The immunoblot band was visualized with SuperSignal® West Pico Substrate (Pierce, Product # 34080) or SuperSignal® West Dura Extended Duration Substrate (Pierce, Product # 34076). The membrane was stripped with Restore Western Blot Stripping Buffer (Pierce, Product # 21059) followed by re-probed with beta-actin antibodies.

To explore molecular events of roles of MFG-E8 in VSMC proliferation and senescence, the expression of senescence and cell-cycle associated signaling molecules was determined using MFG-E8 siRNA intervention of early passage VSMCs. It has been reported that the complex of PCNA and CDK4 is an accelerator of the transition of G1/0 to S phase of cell cycle. Western blots in FIG. 15A shows that MFG-E8 silencing substantially reduces PCNA expression in both young and old cells, abolishing the pre-silencing age difference. In addition, Western blots (FIG. 15B) prior to MFG-E8 silence shows that CDK4 expression dramatically increases within old compared to young VSMCs, and in particular, the activated CDK4 form is detected only in old cells (FIG. 15B).. Importantly, MFG-E8 silencing abolishes activate form of CDK4 in old cells and also substantially reduces total CDK4 in both young and old cells (FIG. 15B).

Previous findings demonstrate that ATM, CHK2, p53/p21 all relay DNA surveillance signaling messages and consequent cell cycle deceleration. Western blotting shows that ATM, CHK2, p53 and p21 are expressed in both young and old VSMC (FIGs. 16A, 16B, 16C). Interestingly, p21 expression significantly increased with age (FIG. 16B). However, MFG-E8 silencing significantly enhanced ATM, p53, and p21 protein expression (FIGs. 16A, 16B, 16C).

MFG-E8 silencing also increases, by four-fold, the histostaining of SA-β gal, a marker of senescence, in both young and old VSMCs (FIG. 17).

All results presented above are expressed as the mean ± SEM. Statistical analysis was performed via a T test when two groups were analyzed, or via an ANOVA, followed by a Bonferroni post hoc test for multiple comparisons. A p value of <0.05 was taken as statistically significant. The current study shows that exogenous MFG-E8 treatment enhances proliferation capability while MFG-E8 silencing increases SA-beta-gal expression in both young and old rat VSMC. Additionally, the phenotype of rat VSMC resembles that of senesced IMR-90 cells, a human diploid embryonic fibroblast cell line, commonly used as an *in vitro* model of cellular senescence. Therefore, it was hypothesize that MFG-E8 may also be a survival factor for human cells.

IMR-90 cells were selected at population doubling levels (PDL) 42 to 48, the highest levels still responsive to lower levels of serum (2.5%). MFG-E8 treatment increased the proliferative capability of IMR-90 at PDL 42 in a dose-or time-dependent manner. Phase contrast photomicrographs illustrate that untreated cells present a typical senesced morphology *i.e.,* enlarged and flattened compared to treated cells. Importantly, the MFG-E8-associated cellular replication increase was abolished by neutral antibodies against αvβ3 and αvβ5 integrin receptor. In addition, the SA-beta-gal staining showed that the number of SA-beta-gal stained cells after MFG-E8 treatment for 7 days was dramatically reduced compared to untreated control cells. Interestingly, this effect was also abolished by neutralizing antibodies against αvβ3 and αvβ5.

Provided herein are several novel findings: 1) MFG-E8 abundance increases within the aortic wall, predominantly in thickened intima and in the innermost media of Ang II infused young rats, similar to old animals; 2) An age-associated increase in the proliferative capacity of early passage cultured VSMC, which also harbor elevated MFG-E8, is abolished by blockade of integrin signaling; 3) Exogenous administration or over-expression of the VSMC to MFG-E8 enhances proliferation signaling molecules i.e. CDK4; 4) Partial reduction of endogenous MFG-E8 via siRNA drives VSMC toward a classical senescent appearance i.e. SA-β -gal positive staining through activation of senescence signaling molecules i.e. ATM, p21, p53; 5) MFG-E8 accelerates the VSMC wound healing process and also is abundantly present in the proliferative neointima in response to arterial injuries (see Example 7, below). Arterial proliferation may be orchestrated by reciprocal integrin signaling of these arterial age-associated molecules. Indeed, it is demonstrated herein that MFG-E8 signaling is a facilitator of both young and old VSMC proliferation via integrin signaling, indicating that an age-associated increase in engagement of MFG-E8 with integrins may mediate age-associated arterial proliferation. Interactions of exogenous MFG-E8 and integrins (αvβ3 and αvβ5) increase the rate of VSMC proliferation, resembling normal aged cells, through a shortening of the replication time by acceleration of the basic cell cycle. Moreover, it is demonstrated herein that MFG-E8 increases phosphorylation of ERK1/2 and CDK-4 as well as PCNA expression, and that these are substantially inhibited by a blockade of integrin signaling. In addition, the small RNA interference (silencing) studies provided herein further show that the proliferative capacity of VSMC is dependent upon local MFG-E8 abundance.

Thus, it is surprisingly provided herein that MFG-E8 is an essential regulatory element of VSMC proliferation. Increased MFG-E8 is able to compensate for reduced serum levels by facilitating entry into the cell cycle via the intergrin/ERK1/2/ PCNA/CDK4 proliferative signaling cascade accompanying a proinflammtory response. Additionally, MFG-E8 silencing is capable of triggering DNA damage signaling through the ATM/CHK2/p53/p21 signal axis to delay cell cycle and promote entry into the arrested state. Thus, it is surprisingly revealed that MFG-E8 plays a pivotal role in VSMC proliferation. Interventions of MFG-E8 signaling can be used in novel therapeutic approaches to prevent and treat age-associated proliferative vascular diseases, such as atherosclerosis and cancer.

### Example 7

### MFG-E8 Expression is Up-regulated Post Angioplasty within the Neointima

This example describes tests to determine that MFG-E8 expression is increased in response to arterial injury.

Since the underlying cause of post-angioplastic restenosis is the inward neointimal growth due mainly to VSMC proliferation and migration and the current study demonstrates that MFG-E8 is a potent mitogen of VSMC, it was hypothesized that MFG-E8 would be increased in the arterial walls injured arteries. To test this hypothesis, the presence of MFG-E8 was determined in the injured arterial wall. Injured carotid paraffin samples were obtained from a previous study. In brief, rats were anesthetized by intraperitoneal injection with 40 mg/kg of sodium pentobarbital and 8 mg/kg of xylazine. The left carotid artery was surgically separated, and a small incision was made on the external carotid artery with ophthalmologic scissors. A 2F Fogarty arterial embolectomy balloon catheter (Baxter, McGraw Park, IL, U.S.A.) was inserted through the incision in the external carotid artery into the common carotid artery. The catheter was inflated with 30 µl of saline and withdrawn along the common carotid artery. The effective passing distance of the balloon catheter was 2.5-3.0 cm (*i.e.,* the entire length of the common carotid artery). The ballooning procedure was repeated three times to ensure a complete endothelial denudation. The external carotid artery was ligated, and the animals were allowed to recover. Dual immunofluorescence staining showed that the MFG-E8 protein signal was predominantly distributed and colocalized with PCNA staining within the neointima (FIG. 14). This result demonstrates that MFG-E8 is upregulated in response to arterial injury and identifies MFG-E8 as a marker for arterial injury.

*In vitro,* exogenous MFG-E8 treatment or over-expression of MFG-E8 significantly increases transcription factor Ets-1 expression in both young and old VSMC (FIG. 18A). MFG-E8 treatment, as overexpression of Ets-1 does, markedly enhances the activity of TGF-β1, a powerful profibrogenic cytokine (FIG. 18B). Furthermore, both treatments increase phosphorylation of SMA2/3, but did not have an effect on SMAD7 expression (FIG. 18C). Importantly, data indicate that an administration of MFG-E8 to young VSMC increase collagen III production (FIG. 18D). In contrast, MFG-E8 knockdown decreases type III collagen gene transcription (FIG. 18E). In addition, overexpression of MFG-E8 increases AT1 protein expression in both young and old VSMC (FIG. 19A). Data also show that angiotensin II (Ang II) strongly increases MFG-E8 expression. In contrast, angiotensin conversing enzyme, Benazepril Hydrochloride, decreases MFG-E8 expression, particularly the short form, in old VSMC (FIG. 19B). Strikingly, a chronic administration of Benazepril Hydrochloride to 8-month-old FXBN rats for 12 months via a gavage daily markedly decreased MFG-E8 expression compared to placebo animals (FIG. 19C).

### Example 8

### Multiple Reaction Monitoring (MRM)

This example describes the use of MRM to detect, identify and quantitate peptides and the proteins they are derived from in a complex mixture.

MRM is a breakthrough technology for targeted biomarker validation/verification as it completely removes the need for developing antibodies. In other words, sandwich immunoassays are replaced by MRM MS analysis at the validation stages and importantly, it still provides absolute quantification for specific proteins within complex protein mixtures such a serum or plasma. Single protein can be quantified while multiple proteins or multiple forms of the same protein can be assessed simultaneously in a multiplex format. MRM is based on the targeting and selecting of specific peptides that uniquely define their parent (target) protein. This is based on having an MSMS instrument able to determine the precursor *m*/*z* and a fragment *m*/*z* (MRM transition) of a peptide with very high accuracy so that it can only be assigned to a single amino acid sequence. If the amino acid sequence can only be assigned to one protein then quantification of that peptide will correlated directly to the concentration of the protein. The addition of an isotopically labeled peptide (standard) at a known quantity allows for the absolute quantification of the target peptide and hence of the originating protein of interest. The success of this approach is based on knowing the correct peptide(s) that will be observable in MS especially in a complex mixture and a reproducible instrument.

To validate MRM, an optimized Q-Trap LC/MS/MS instrument with improved sensitivity with at least 7 magnitudes in dynamic range was used to detect and identify *E. coli* proteins introduced into a complex mixture. Briefly, a known amount of an *E. coli* protein was added into a digest of cardiac tissue homogenate and one peptide was analyzed by MS for 10 transitions that are specific to the *E. coli* protein present in the sample. As shown in FIG. 8, concentrations of the peptide for the *E. coli* protein are in the range of over 3 orders of magnitude. It can be selected in the Q-Trap LC/MS/MS and detected in linearity response (R²=0.9994). The reproducibility of MRM experiments was excellent with an average % RSD of 6-9%.

In serum and plasma, peptides that are unique to candidate biomarkers of less than 0.3 ng/ml can be directly digested and then targeted, detected and quantified.

### Example 9

### Multiple Reaction Monitoring of Proteins Elevated in Aged Aorta

The MRM method can be extended to the detection, identification and quantitation of the proteins listed in Table 1 in a biological sample obtained from a subject. By way of example, MFG-E8 is elevated with age in the aorta and isolated aortic smooth muscle in a number of mammals, including humans. MFG-E8 is an abundant protein in smooth muscle and has been found in plasma. By selecting peptides to various regions of MFG-E8 the ratio between the mature and the proteolyzed forms (see FIG. 8) is determined.

MFG-E8 is secreted from smooth muscle cells and its biological affect on smooth muscle when exogenous present differs from that exerted endogenously. MFG-E8 is processed into multiple forms: A precursor containing a signal sequence which creates the mature protein when proteolyzed that can be further processed to a shorter version (called lactadherin short) and medin (Table 4). The circulating/secreted form is not known. Therefore, it is of clinical interest to differentiate between these possible circulating forms and investigate a possible correlation between biological age and cardiovascular risk. To do this, multiple reaction monitoring (MRM) mass spectrometry (MS)-based quantitative studies are performed on serum samples from a number of different age groups to determine the presence and amount of circulating forms of MFG-E8 present in serum.

Tissue, isolated subproteomes or protein complexes will be solubilized, concentrated and protein quantified by BCA assay, treated, if needed with 2D Cleanup Kits (GE Health Care), followed by tryptic or chymotryptic digestion (Protein/enzyme ratio 20:1) overnight at 37°C. The dried peptides are suspended in 5% acetonitrile/water with 0.1% TFA.

A series of MRM assays in which peptides combined from different regions of MFG-E8 that will allow differentiation between the intact, short and medin forms of the protein is performed. The correlate the concentration of the various forms (and total) MFG-E8 present in serum with aortic stiffness (a reflection of biological age), plaque burden and long term cardiovascular outcomes. The cohorts that are studied are: i) nonhuman primate aging (3 age groups) with regular versus a calorie restricted diet (as an intervention protocol), ii) Baltimore Longitudinal Study cohort for outcomes and iii) subset of Atherosclerosis Risk in Communities Study (ARIC) cohort of over 18,000 individuals studied over 15 years with available serum samples, aortic plaque size and outcomes.

**Table 4: MRM assay**

| Protein target | Form that will also detected | Region peptides need to be in | Selected peptide 1 | Selected peptide 2 |
|---|---|---|---|---|
| MFG-E8 intact | None other | 24-202 | LASHEYLK | EFVGNWNK |
| | | | (SEQ ID NO: 9) | (SEQ ID NO: 10) |
| Short version | Intact | 202-268 and 317-387 | NNSIPDK | LELLGC |
| | | | (SEQ ID NO: 11) | (SEQ ID NO: 12) |
| Medin | Intact and short version | 268-317 | NFGSVQFVA | EVTGIITQGAR |
| | | | (SEQ ID NO: 13) | (SEQ ID NO: 14) |

The selected peptides are based on observed MS data in rat tissue and smooth muscle cells and human recombinant truncated protein.

In view of the many possible embodiments to which the principles of the disclosed invention may be applied, it should be recognized that the illustrated embodiments are only preferred examples of the invention. The scope of the invention is defined by the following claims.

## Claims

1. A method for diagnosing or prognosing an age-associated vascular disorder in a subject, the method comprising:
detecting an altered expression level of a gene product of Milk Fat Globule Protein Epidermal Growth Factor-8 (MFG-E8), and of a gene product of at least three other genes increasing in expression as a function of age and selected from HTRA1, ITM2B, CSRP2, PLF4, IBP7, RBP1, ARPC4, CNN1, CNN3, TGFB3, CLUS, MMP2, PGS1, FINC, SPRL1, VTNC, KNT1, POSTN, APOE, TRI47, ADCY5, GTR4, and FRIL1 in the biological sample, relative to a reference level, in a
biological sample obtained from the subject,
thereby diagnosing or prognosing an age-associated vascular disorder in the subject.

2. The method of claim 1, wherein:
(I) the reference level is the level of expression of each said gene product:
a) in a biological sample obtained from the same subject at an earlier time point;
b) in a biological sample obtained from another subject that does not have the age-associated vascular disorder;
c) in a biological sample obtained from a younger individual; or
d) a statistical reference value indicative of expression in a subject that does not have the age-associated vascular disorder; and/or
(II) wherein the gene products of MFG-E8 and the at least three other genes increasing in expression as a function of age are each proteins and detection of altered expression of the gene products comprises detecting said proteins; and/or
(III) said method further comprises enrichment of the MFG-E8 protein and the protein gene products of the at least three other genes increasing in expression as a function of age in the biological sample.

3. The method of claim 2 (II) or (III), further comprising:
producing a protein digest from the biological sample; and
detecting a fragment of the MFG-E8 protein and a fragment of a protein gene product of each of the at least three other genes increasing in expression as a function of age in the protein digest, thereby detecting the MFG-E8 protein and the protein gene products of the at least three other genes increasing in expression as a function of age in the biological sample.

4. The method of claim 3, wherein:
(I) the peptide fragment of MFG-E8 comprises or consists of the amino acid sequence of any one of SEQ ID NOs: 1, 2 and 9 to 12; and/or
(II) said method further comprises fractionating the protein digest prior to detecting the fragment of the MFG-E8 protein and the fragment of a protein gene product of each of the at least three other genes increasing in expression as a function of age in the protein digest, optionally wherein fractionating the protein digest comprises separation of the digest by chromatography, preferably wherein the chromatography comprises one or more of gel electrophoresis, capillary electrophoresis, nano-reversed phase liquid chromatography, high performance liquid chromatography, reverse phase high performance liquid chromatography, ion exchange chromatography, phosphorylation enrichment affinity chromatography, glycosylation enrichment affinity chromatography, and protein affinity chromatography; and/or
(III) the protein digest comprises a serine protease digest, optionally a trypsin digest.

5. The method of any one of claims 2 (II) or (III), 3 or 4, wherein detecting protein comprises mass spectrometry.

6. The method of claim 2 (II) or (III), wherein detecting the MFG-E8 protein and the protein gene products of the at least three other genes increasing in expression as a function of age comprises contacting the MFG-E8 protein with an antibody that specifically binds the MFG-E8 protein and contacting each protein gene product of the at least three other genes increasing in expression as a function of age with an antibody specifically binding said protein gene product.

7. The method of any one of claims 2 (II) or (III) and 3 to 6, further comprising quantitating the MFG-E8 protein and the protein gene products of the at least three other genes increasing in expression as a function of age, or a fragment of each thereof.

8. The method of claim 7, wherein quantitating the MFG-E8 protein and the protein gene products of the at least three other genes increasing in expression as a function of age comprises comparing an amount of a fragment of the MFG-E8 protein and of a fragment of each of the protein gene products of the at least three other genes increasing in expression as a function of age to a peptide standard of known amount, optionally wherein the peptide standard for MFG-E8 comprises or consists of a peptide having the amino acid sequence of any one of SEQ ID NOs: 1, 2 or 9 to 12.

9. The method of claim 5 or 8, wherein the peptide standard is an isotopically labeled peptide, optionally wherein the isotopically labeled peptide comprises one or more heavy stable isotopes selected from ¹⁸O, ¹⁷O, ³⁴S, ¹⁵N, ¹³C, ²H or combinations thereof.

10. The method of claim 2 (II), (III) or 3, wherein the MFG-E8 protein and/or one or more of the protein gene products of the at least three other genes increasing in expression as a function of age, or a fragment of any thereof, is post-translationally modified.

11. The method of claim 1 or 2, wherein the gene product of MFG-E8 and the gene products of the at least three other genes increasing in expression as a function of age are each a nucleic acid and detecting altered expression of the gene products comprises detecting each said nucleic acid.

12. The method of claim 11, wherein detecting each said nucleic acid comprises detecting mRNA, optionally using a reverse-transcription-polymerase chain reaction (RT-PCR) or mass spectrometry, preferably wherein the RT-PCR comprises quantitative RT-PCR; and/or
wherein detecting expression comprises using a microarray.

13. The method of any one of the preceding claims, wherein detecting altered expression further comprises detecting an expression level of MFG-E8 and the at least three other genes increasing in expression as a function of age, optionally wherein the altered expression level of MFG-E8 and of the at least three other genes increasing in expression as a function of age comprises an increase in the expression level of MFG-E8 and the at least three other genes increasing in expression as a function of age, preferably an increase in protein or nucleic acid level.

14. The method of any one of the preceding claims, wherein the biological sample comprises a blood sample, a urine sample, a serum sample, an ascites sample, a saliva sample, a cell, or a portion of a tissue sample.

15. The method of claim 14, wherein the biological sample comprises a serum or a plasma sample, optionally wherein the tissue sample is obtained by biopsy, preferably wherein the tissue sample obtained by biopsy comprises vascular tissue.

## Patentansprüche

1. Verfahren zur Diagnose oder Prognose einer altersbedingten Gefäßerkrankung bei einem Subjekt, wobei das Verfahren umfasst:
Detektieren eines veränderten Expressionslevels eines Genproduktes von Milk Fat Globule Protein Epidermal Growth Factor-8 (MFG-E8) und eines Genproduktes von wenigstens drei anderen Genen, deren Expression sich als Funktion des Alters erhöht und die ausgewählt sind aus HTRA1, ITM2B, CSRP2, PLF4, IBP7, RBP1, ARPC4, CNN1, CNN3, TGFB3, CLUS, MMP2, PGS1, FINC, SPRL1, VTNC, KNT1, POSTN, APOE, TRI47, ADCY5, GTR4 und FRIL1 in der biologischen Probe relativ zu einem Referenzlevel in einer
biologischen Probe, die von dem Subjekt erhalten wurde,
wodurch eine Diagnose oder Prognose für eine altersbedingte Gefäßerkrankung bei dem Subjekt erstellt wird.

2. Verfahren gemäß Anspruch 1, wobei:
(I) das Referenzlevel das Expressionslevel jedes Genproduktes ist:
a) in einer biologischen Probe, die von demselben Subjekt zu einem früheren Zeitpunkt erhalten worden ist;
b) in einer biologischen Probe, die von einem anderen Subjekt, das keine altersbedingte Gefäßerkrankung hat, erhalten worden ist;
c) in einer biologischen Probe, die von einem jüngeren Individuum erhalten worden ist, oder
d) ein statistischer Referenzwert, der für eine Expression in einem Subjekt indikativ ist, das keine altersbedingte Gefäßerkrankung hat, und/oder
(II) wobei die Genprodukte von MFG-E8 und den wenigstens drei anderen Genen, deren Expression sich als Funktion des Alters erhöht, jeweils Proteine sind und die Detektion einer veränderten Expression der Genprodukte Detektieren der genannten Proteine umfasst; und/oder
(III) das Verfahren außerdem eine Anreicherung des MFG-E8-Proteins und der Proteingenprodukte der wenigstens drei anderen Gene, deren Expression sich als Funktion des Alters erhöht, in der biologischen Probe umfasst.

3. Verfahren gemäß Anspruch 2 (II) oder (III), außerdem umfassend:
Produzieren eines Proteinverdaus aus der biologischen Probe und
Detektieren eines Fragmentes des MFG-E8-Proteins und eines Fragmentes eines Proteingenproduktes von jedem der wenigstens drei anderen Gene, deren Expression sich als Funktion des Alters erhöht, in dem Proteinverdau, wodurch das MFG-E8-Protein und die Genprodukte der wenigstens drei anderen Gene, deren Expression als Funktion des Alters erhöht ist, in der biologischen Probe detektiert werden.

4. Verfahren gemäß Anspruch 3, wobei:
(I) das Peptidfragment von MFG-E8 die Aminosäuresequenz von einer von SEQ ID NOS:1, 2 und 9 bis 12 umfasst oder aus dieser besteht und/oder
(II) das Verfahren außerdem Fraktionieren des Proteinverdaus vor Detektieren des Fragmentes des MFG-E8-Proteins und des Fragmentes eines Proteingenproduktes von jedem der wenigstens drei anderen Gene, deren Expression sich als Funktion des Alters erhöht, in dem Proteinverdau umfasst, wobei das Fraktionieren des Proteinverdaus gegebenenfalls Trennung des Verdaus durch Chromatographie umfasst, wobei die Chromatographie vorzugsweise eine oder mehrere von Gelelektrophorese, Kapillarelektrophorese, Nano-Umkehrphasen-Flüssigkeitschromatographie, Hochleistungsflüssigkeitschromatographie, Umkehrphasen-Hochleistungsflüssigkeitschromatographie, Ionenaustauschchromatographie, Phosphorylierungsanreicherungs-Affinitätschromatographie, Glykosylierungsanreicherungs-Affinitätschromatographie und Proteinaffinitätschromatographie umfasst, und/oder
(III) der Proteinverdau einen Serinproteaseverdau, gegebenenfalls einen Trypsinverdau, umfasst.

5. Verfahren gemäß einem der Ansprüche 2 (II) oder (III), 3 oder 4, wobei Detektieren von Protein Massenspektrometrie umfasst.

6. Verfahren gemäß Anspruch 2 (II) oder (III), wobei Detektieren des MFG-E8-Proteins und der Proteingenprodukte der wenigstens drei anderen Gene, deren Expression sich als Funktion des Alters erhöht, Inkontaktbringen des MFG-E8-Proteins mit einem Antikörper, der spezifisch das MFG-E8-Protein bindet, und Inkontaktbringen jedes Proteingenproduktes der wenigstens drei anderen Gene, deren Expression sich als Funktion des Alters erhöht, mit einem Antikörper, der das Proteingenprodukt spezifisch bindet, umfasst.

7. Verfahren gemäß einem der Ansprüche 2 (II) oder (III) und 3 bis 6, das außerdem quantitatives Bestimmen des MFG-E8-Proteins und der Proteingenprodukte der wenigstens drei anderen Gene, deren Expression sich als Funktion des Alters erhöht, oder eines Fragments von jedem davon umfasst.

8. Verfahren gemäß Anspruch 7, wobei ein quantitatives Bestimmen des MFG-E8-Proteins und der Proteingenprodukte der wenigstens drei anderen Gene, deren Expression sich als Funktion des Alters erhöht, ein Vergleichen der Menge eines Fragmentes des MFG-E8-Proteins und eines Fragmentes jedes der Proteingenprodukte der wenigstens drei anderen Gene, deren Expression sich als Funktion des Alters erhöht, mit einem Peptidstandard bekannter Menge umfasst, wobei gegebenenfalls der Peptidstandard für MFG-E8 ein Peptid, das die Aminosäuresequenz von einer von SEQ ID NOS:1, 2 oder 9 bis 12 hat, umfasst oder aus diesem besteht.

9. Verfahren gemäß Anspruch 5 oder 8, wobei der Peptidstandard ein isotopisch markiertes Peptid ist, wobei das isotopisch markierte Peptid gegebenenfalls ein schweres stabiles Isotop oder mehrere schwere stabile Isotope, ausgewählt aus ¹⁸O, ¹⁷O, ³⁴S, ¹⁵N, ¹³C, ²H oder Kombinationen davon, umfasst.

10. Verfahren gemäß Anspruch 2 (II), (III) oder 3, wobei das MFG-E8-Protein und/oder eines oder mehrere der Proteingenprodukte der wenigstens drei anderen Gene, deren Expression sich als Funktion des Alters erhöht, oder ein Fragment eines beliebigen davon posttranslational modifiziert ist.

11. Verfahren gemäß Anspruch 1 oder 2, wobei das Genprodukt von MFG-E8 und die Genprodukte der wenigstens drei anderen Gene, deren Expression sich als Funktion des Alters erhöht, jeweils eine Nukleinsäure sind und das Detektieren einer veränderten Expression der Genprodukte Detektieren der jeweiligen Nukleinsäure umfasst.

12. Verfahren gemäß Anspruch 11, wobei ein Detektieren der jeweiligen Nukleinsäure Detektieren von mRNA gegebenenfalls unter Verwendung einer reversen Transkriptions-Polymerasekettenreaktion (RT-PCR) oder von Massenspektrometrie umfasst, wobei die RT-PCR vorzugsweise quantitative RT-PCR umfasst und/oder
wobei Detektieren einer Expression Verwenden eines Mikroarrays umfasst.

13. Verfahren gemäß einem der vorangehenden Ansprüche, wobei ein Detektieren einer veränderten Expression außerdem Detektieren des Expressionslevels von MFG-E8 und den wenigstens drei anderen Genen, deren Expression sich als Funktion des Alters erhöht, umfasst, wobei gegebenenfalls der veränderte Expressionslevel von MFG-E8 und den wenigstens drei anderen Genen, deren Expression sich als Funktion des Alters erhöht, eine Erhöhung beim Expressionslevel von MFG-E8 und den wenigstens drei anderen Genen, deren Expression sich als Funktion des Alters erhöht, vorzugsweise eine Erhöhung beim Protein- oder Nukleinsäurelevel umfasst.

14. Verfahren gemäß einem der vorangehenden Ansprüche, wobei die biologische Probe eine Blutprobe, eine Urinprobe, eine Serumprobe, eine Aszitenprobe, eine Speichelprobe, eine Zelle oder ein Teil einer Gewebeprobe umfasst.

15. Verfahren gemäß Anspruch 14, wobei die biologische Probe eine Serum- oder eine Plasmaprobe umfasst, wobei die Gewebeprobe durch Biopsie erhalten wird, wobei die Gewebeprobe, die durch Biopsie erhalten wird, vorzugsweise Gefäßgewebe umfasst.

## Revendications

1. Procédé de diagnostic ou de pronostic d'un trouble vasculaire lié à l'âge chez un sujet, lequel procédé comporte l'étape suivante :
détecter, dans un échantillon biologique prélevé chez le sujet, une altération du niveau d'expression du produit de gène qu'est la protéine MFG-E8 (Milk Fat Globule Epidermal Growth Factor 8) et des produits de gène d'au moins trois autres gènes dont l'expression augmente avec l'âge, choisis parmi HTRA1, ITM2B, CSRP2, PLF4, IBP7, RBP1, ARPC4, CNN1, CNN3, TGFB3, CLUS, MMP2, PGS1, FINC, SPRL1, VTNC, KNT1, POSTN, APOE, TRI47, ADCY5, GTR4 et FRIL1, dans l'échantillon biologique, par rapport à un niveau de référence,
ce qui permet d'établir chez le sujet un diagnostic ou un pronostic de trouble vasculaire lié à l'âge.

2. Procédé conforme à la revendication 1,
I) pour lequel le niveau de référence, pour chacun desdits produits de gène, est son niveau d'expression
a) dans un échantillon biologique prélevé antérieurement chez le même sujet,
b) dans un échantillon biologique prélevé chez un autre sujet qui n'est pas atteint du trouble vasculaire lié à l'âge,
c) ou dans un échantillon biologique prélevé chez un individu plus jeune,
d) ou est une valeur statistique de référence, indicative de l'expression chez un sujet qui n'est pas atteint du trouble vasculaire lié à l'âge ;
II) et/ou dans lequel les produits du gène de MFG-E8 et des autres gènes, au nombre d'au moins trois, dont l'expression augmente avec l'âge sont chacun des protéines, et la détection de l'altération de l'expression des produits de gène comporte le fait de détecter ces protéines ;
III) et/ou lequel procédé comporte en outre le fait d'enrichir l'échantillon biologique en la protéine MFG-E8 et en les protéines produites par les autres gènes, au nombre d'au moins trois, dont l'expression augmente avec l'âge.

3. Procédé conforme à la revendication 2, item (II) ou (III), qui comporte en outre
- le fait de préparer, à partir de l'échantillon biologique, un produit de digestion de protéines,
- et le fait de détecter, dans ce produit de digestion de protéines, un fragment de la protéine MFG-E8 et un fragment de chacune des protéines produites par les autres gènes, au nombre d'au moins trois, dont l'expression augmente avec l'âge,
ce qui fait que l'on détecte, dans l'échantillon biologique, la protéine MFG-E8 et les protéines produites par les autres gènes, au nombre d'au moins trois, dont l'expression augmente avec l'âge.

4. Procédé conforme à la revendication 3,
I) dans lequel le fragment peptidique de MFG-E8 comporte l'une des séquences d'acides aminés présentées en tant que Séquences N° 1, 2 et 9 à 12, ou est constitué de l'une de ces séquences,
II) et/ou lequel procédé comporte en outre le fait de fractionner le produit de digestion de protéines, avant de détecter, dans ce produit de digestion de protéines, un fragment de la protéine MFG-E8 et un fragment de chacune des protéines produites par les autres gènes, au nombre d'au moins trois, dont l'expression augmente avec l'âge, étant entendu que, en option, fractionner le produit de digestion de protéines comporte le fait de séparer par chromatographie les composants du produit de digestion de protéines, et que, de préférence, la technique de chromatographie est l'une ou plusieurs des suivantes : électrophorèse en gel, électrophorèse en capillaire, nano-chromatographie en phase liquide à polarité de phases inversée, chromatographie en phase liquide haute performance, chromatographie en phase liquide haute performance à polarité de phases inversée, chromatographie d'échange d'ions, chromatographie d'affinité après enrichissement en espèces phosphorylées, chromatographie d'affinité après enrichissement en espèces glycosylées, et chromatographie d'affinité de protéines,
III) et/ou dans lequel procédé le produit de digestion de protéines comprend un produit de digestion par protéase à sérine, en option un produit de digestion par trypsine.

5. Procédé conforme à l'une des revendications 2, item (II) ou (III), 3 et 4, dans lequel la détection de protéines comporte une analyse par spectrométrie de masse.

6. Procédé conforme à la revendication 2, item (II) ou (III), dans lequel la détection de la protéine MFG-E8 et des protéines produites par les autres gènes, au nombre d'au moins trois, dont l'expression augmente avec l'âge comporte le fait de mettre la protéine MFG-E8 en contact avec un anticorps qui se lie spécifiquement à la protéine MGF-E8 et le fait de mettre chacune des protéines produites par les autres gènes, au nombre d'au moins trois, dont l'expression augmente avec l'âge en contact avec un anticorps qui se lie spécifiquement à ladite protéine produit de gène.

7. Procédé conforme à l'une des revendications 2 item (II) ou (III) et 3 à 6, qui comporte en outre le fait de quantifier la protéine MFG-E8 et les protéines produites par les autres gènes, au nombre d'au moins trois, dont l'expression augmente avec l'âge, ou un fragment de chacune d'elles.

8. Procédé conforme à la revendication 7, dans lequel quantifier la protéine MFG-E8 et les protéines produites par les autres gènes, au nombre d'au moins trois, dont l'expression augmente avec l'âge comprend le fait de comparer une quantité d'un fragment de la protéine MFG-E8 et d'un fragment de chacune des protéines produites par les autres gènes, au nombre d'au moins trois, dont l'expression augmente avec l'âge à un étalon peptidique de quantité connue, étant entendu que, en option, l'étalon peptidique utilisé pour MGF-E8 comporte un peptide dont la séquence d'acides aminés est l'une de celles présentées en tant que Séquences N° 1, 2 et 9 à 12, ou est constitué d'un tel peptide.

9. Procédé conforme à la revendication 5 ou 8, dans lequel l'étalon peptidique est un peptide marqué avec un isotope, et en option, ce peptide marqué avec un isotope contient un ou plusieurs isotope(s) lourd(s) stable(s), choisi(s) parmi ¹⁸O, ¹⁷O, ³⁴S, ¹⁵N, ¹³C, ²H et leurs combinaisons.

10. Procédé conforme à la revendication 2 item (II) ou (III) ou 3, dans lequel la protéine MFG-E8 et/ou l'une ou plusieurs des protéines produites par les autres gènes, au nombre d'au moins trois, dont l'expression augmente avec l'âge, ou un fragment de n'importe laquelle ou lesquelles d'entre elles, a ou ont subi une modification post-traductionnelle.

11. Procédé conforme à la revendication 1 ou 2, dans lequel le produit du gène de MFG-E8 et les produits des autres gènes, au nombre d'au moins trois, dont l'expression augmente avec l'âge sont chacun un acide nucléique, et détecter l'altération de l'expression des produits de gène comporte le fait de détecter chacun de ces acides nucléiques.

12. Procédé conforme à la revendication 11, dans lequel détecter chacun desdits acides nucléiques comporte le fait de détecter des ARNm, en option en ayant recours à une réaction RT-PCR (réaction en chaîne de polymérase après transcription inverse) ou à une analyse par spectrométrie de masse, étant entendu que, de préférence, la réaction RT-PCR est une réaction RT-PCR quantitative,
et/ou dans lequel détecter l'expression comporte le fait d'avoir recours à un micro-réseau.

13. Procédé conforme à l'une des revendications précédentes, dans lequel détecter l'altération de l'expression comporte en outre le fait de détecter le niveau d'expression du gène de MFG-E8 et des autres gènes, au nombre d'au moins trois, dont l'expression augmente avec l'âge, étant entendu que, en option, l'altération du niveau d'expression du gène de MFG-E8 et des autres gènes, au nombre d'au moins trois, dont l'expression augmente avec l'âge comprend une augmentation du niveau d'expression du gène de MFG-E8 et des autres gènes, au nombre d'au moins trois, dont l'expression augmente avec l'âge, de préférence une augmentation de la quantité de protéine ou d'acide nucléique.

14. Procédé conforme à l'une des revendications précédentes, dans lequel l'échantillon biologique comprend un échantillon de sang, un échantillon d'urine, un échantillon de sérum, un échantillon de liquide d'ascite, un échantillon de salive, une cellule ou un fragment d'un échantillon de tissu.

15. Procédé conforme à la revendication 14, dans lequel l'échantillon biologique comprend un échantillon de sérum ou de plasma, et dans lequel, en option, l'échantillon de tissu est obtenu par biopsie, étant entendu que, de préférence, l'échantillon de tissu obtenu par biopsie comprend un tissu vasculaire.
